# EUROPÄISCHE PATENTSCHRIFT

(11) **EP 0 218 118 B1**
(45) Veröffentlichungstag und Bekanntmachung des Hinweises auf die Patenterteilung: **18.09.1991**
(21) Anmeldenummer: 86112631.6
(22) Anmeldetag: 12.09.1986
(51) Int. Cl.: C07D 233/60, C07D 249/08, A61K 31/41, A61K 31/415

(54) **Arylmethylazole und deren Salze, Verfahren zu ihrer Herstellung, sie enthaltende Mittel und ihre Verwendung**
Arylmethyl azoles and their salts, method for their preparation, compositions containing them and their use
Arylméthylazoles et leurs sels, leur procédé de préparation, compositions les contenant et leur application

(30) Priorität: 23.09.1985 DE 3533824; 23.11.1985 DE 3541429; 14.08.1986 DE 3627656; 22.08.1986 DE 3628545
(43) Veröffentlichungstag der Anmeldung: 15.04.1987
(73) Patentinhaber: HOECHST AKTIENGESELLSCHAFT, 65926 Frankfurt am Main (DE)
(72) Erfinder: Siegel, Herbert, Dr., D-6238 Hofheim am Taunus (DE); Kampe, Klaus-Dieter, Dr., D-6232 Bad Soden am Taunus (DE); Alpermann, Hans Georg, Dr., D-6240 Königstein/Taunus (DE); Gerhards, Hermann Josef, Dr., D-6238 Hofheim am Taunus (DE); Usinger, Patricia, Dr., D-6239 Eppstein/Taunus (DE); Schacht, Ulrich, Dr., D-6238 Hofheim am Taunus (DE); Leven, Margret, Dr., D-6233 Kelkheim (Taunus) (DE); Raether, Wolfgang, Dr., D-6072 Dreieich (DE); Dittmar, Walter, Dr., D-6238 Hofheim am Taunus (DE); Sachse, Burkhard, Dr., D-6233 Kelkheim (Taunus) (DE)

(56) Entgegenhaltungen:
- EP-A- 0 099 165
- EP-A- 0 129 798
- EP-A- 0 149 976
- EP-A- 0 192 055
- DE-C- 2 041 771
- DE-C- 2 430 039

## Beschreibung

Die Erfindung betrifft substituierte Arylmethylazole einschließlich ihrer Salze, Verfahren zu ihrer Herstellung, diese Verbindungen enthaltende Arzneimittel und ihre Vervendung als Arzneimittel sowie Fungizide.

In den Offenlegungsschriften DE-OS 2 948 206 und EP-OS 61 835 ist beschrieben, daß Benzylazole zur Bekämpfung von Pilzen bei Mensch, Tier und Pflanze geeignet sind. Die Wirksamkeit dieser Verbindungen ist Jedoch insbesondere bei niedrigen Konzentrationen nicht befriedigend. Die EP 0 149 976 beschreibt Benzylimidazole als Verbindungen mit antidepressiven Eigenschaften. Diese Verbindungen sind jedoch Monoamionooxidase-Hemmer (MAO-Hemmer), von denen bekannt ist, daß sie zum Teil schwere Nebenwirkungen hervorrufen. MAO-Hemmer werden deshalb auf dem Idikationsgebiet der Antidepressiva als unvorteilhaft bezeichnet und es bestand das Bedürfnis, Verbindungen zur Verfügung zu stellen, die diesen Effekt der MAO-Hemmung nicht zeigen.

Der Erfindung lag die Aufgabe zu Grunde, Verbindungen zur Verfugung zu stellen, die neben deutlich verbesserter antimykotischer Wirkung auch für andere Indikationen geeignet sind.

Die Erfindung ist daher gerichtet auf Arylalkylazole der Formel I 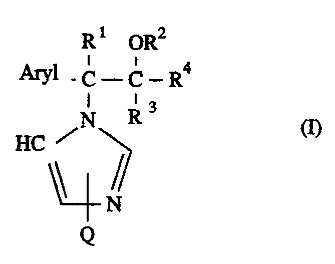 in der bedeuten
Aryl einen Rest 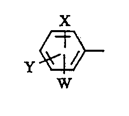 oder einen unsubstituierten
   oder durch einen Substituenten U und/oder einen Substituenten V substituierten 1 - oder 2-Naphthylrest, wobei
X H, (C₁-C₄)-Alkyl, Phenyl, Fluor, Chlor, Brom, (C₁-C₄)-Alkoxy, 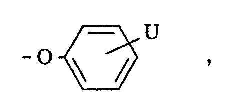 (C₁-C₄)-Alkylthio, -N(R⁵)_{2,} in dem R⁵ -gleich
   oder verschieden - (C₁-C₄)-Alkyl oder zusammen mit dem Stickstoffatom einen Pyrrolidin-, Piperidin- oder Morpholin-Rest bedeutet, CF₃ oder eine unsubstituierte oder eine im Phenylrest 1 oder 2 Substituenten tragende Benzyloxy-Gruppe, wobei die Substituenten gleich oder verschieden sind und Fluor, Chlor, OCH₃, OC₂Hs oder (C₁-C₃)-Alkyl bedeuten,
Y H, (C₁-C₄)-Alkyl, Fluor, Chlor, Brom, (C₁-C₄)-Alkoxy oder (C₁-C₄)-Alkylthio, oder
X und Y zusammen in 2,3- oder 3,4-Stellung eine -(CH₂)_{L}-Kette mit L= 3 oder 4, -OCH₂CH₂- oder -O-CH₂-O-,
W H, CH₃ oder OCH₃,
V (C₁-C₄)-Alkyl, Phenyl, Fluor, Chlor, Brom, Hydroxy, (C₁-C₄)-Alkoxy, (C₁-C₄)-Alkylthio, -N-(R⁵)₂, in dem R⁵ (C,-C₄)-Alkyl oder zusammen mit dem Stickstoffatom einen Pyrrolidin-, Piperidin- oder Morpholin-Rest bedeutet, Benzyloxy oder CF₃ und
U CH₃, F, CI oder OCH₃ bedeuten,
R¹ H
Q H oder (C₁-C₄)-Alkyl,
R² H,
R³ (C₁-C_{1z})-Alkyl, (C₃-C₁₀)-Alkenyl, (C₃-C₁₂)-Cycloalkyl, (C₅-C₁₂)-Cycloalkenyl, (C₇-C₁₂)-Polycycloalkyl, (C₇-C₁₂)-Polycycloalkenyl, (C₅-C₁₂)-Cycloalkyl-(C₁-C₄)-Alkyl, (C_{S}-C₁₂)-Cycloalkyliden-(C₂-C₄)-Alkyl, (C₅-C₁₂)-Cycloalkenyl-(C₁-C₄)-Alkyl, (C₇-C₁₂)-Polycycloalkyl-(C₁-C₄)-Alkyl, (C₇-C₁₂)-Polycycloalkyliden-(C₂-C₄)-Alkyl, (C₇-C₁₂)-Polycycloalkenyl-(C₁-C₄)-Alkyl oder ebendiese Kohlenwasserstoffreste mit bis zu 3 gleichen oder verschiedenen Substituenten, wobei diese F, Cl, Br oder CH₃ bedeuten, oder
   eine unsubstituierte oder eine im Phenylrest bis zu 3 Substituenten tragende Phenyl-(C₂-C₄)-Alkylgruppe, wobei die Substitutenten gleich oder verschieden sind und F, CI, Br, (C₁-C4)-Alkoxy, (C₁-C₄)-Alkyl oder (C₁-C₄)-Alkylthio bedeuten,
   oder eine unsubstituierte oder bis zu 2 Substituenten tragende Naphthyl-(C₁-C₄)-Alkyl- Gruppe, wobei die Substituenten gleich oder verschieden sind und F, Cl, Br, OCH₃ OC_{z}Hs oder (C₁-C₄)-Alkyl bedeuten,
R⁴ H, (C₁-C_{1z})-Alkyl, (C₃-C₁₀)-Alkenyl, (C₃-C₁₂-Cycloalkyl, (C₅-C₁₂)-Cycloalkenyl, (C₇-C₁₂)-Polycycloalkyl, (C₇-C₁₂)-Polycycloalkenyl, (C₅-C₁₂)-Cycloalkyl-(C₁-C₄)-Alkyl, (C_{S}-C₁₂)-Cycloalkyliden-(C2-C4)-Alkyl, (C₅-C₁₂ -Cycloalkenyl-(C₁-C₄)-Alkyl, (C₇-C₁₂- Polycycloalkyl-(C₁-C₄)-Alkyl, (C₇-C₁₂)- Polycycloalkyliden-(C₂-C₄)-Alkyl, (C₇-C₁₂)-Polycycloalkenyl-(C₁-C₄)-Alkyl oder ebendiese Kohlenwasserstoffreste mit bis zu 3 gleichen oder verschiedenen Substituenten, wobei diese F, CI oder Br bedeuten, und wobei die cyclischen Kohlenwasserstoffreste zusätzlich mit (C₁-C₄)-Alkyl substituiert sein können, oder
   eine unsubstituierte oder eine im Phenylrest bis zu 3 Substituenten tragende Phenyl-(C₂-C₄)-Alkylgruppe, wobei die Substitutenten gleich oder verschieden sind und F, Cl, Br, (C₁-C₄)-Alkoxy, (C₁-C₄)-Alkyl oder (C₁-C₄)-Alkylthio bedeuten,
   oder eine unsubstituierte oder bis zu 2 Substituenten tragende Naphthyl-(C₁-C₄)-Alkyl- Gruppe, wobei die Substituenten gleich oder verschieden sind und F, CI, Br, OCH₃ OC₂H₅ oder (C₁-C₄)-Alkyl bedeuten,
R³ und R⁴ gemeinsam
   eine unsubstituierte oder mit (C₁-C₄-Alkyl, OCH₃ oder Phenyl substituierte -(CH₂)ₙ-Kette mit n = 2 - 11 bedeuten, oder eine ebensolche Kohlenwasserstoffkette mit einer Doppelbindung, oder
   eine unsubstituierte oder mit (C₁-C₄)-Alkyl, OCH₃, CH₂OCH₃ oder Phenyl substituierte, einfach oder mehrfach überbrückte -(CHz)m-Kette mit m = 4 oder 5 und 1 bis 5 Brücken- Kohlenstoffatomen, wobei die Brückenkohlenstoffatome ihrerseits wiederum überbrückt sein können und eine Überbrückung keine oder eine C,C-Doppelbindung enthält, sowie deren physiologisch verträgliche Säureadditionssalze und deren Stereoisomere und optisch aktiven Enantiomere.

Bevorzugt sind Verbindungen der Formel I, in denen mindestens einer der Substituenten die folgende Bedeutung hat:
Aryl einen Rest 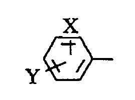 oder einen unsubstituierten oder durch einen Substituent V substituierten 2-Naphthylrest bedeutet, worin
X H, (C₁-C₄)-Alkyl, Phenyl, F, Cl, Br, (C₁-C₄)-Alkoxy, 3-CF₃ oder eine Benzyloxy-Gruppe,
Y H, CH₃, CI oder OCH₃ und
V (C₁-C₄)-Alkyl, Cl, Br, OH oder OCH₃ bedeuten,
Q H, CH₃ oder C₂H₅
R¹ H,
R² H,
R³ (C₁-Cₐ)-Alkyl, (C₃-C₁₀)-Alkenyl, (C₃-C₁₂)-Cycloalkyl, (C₅-C₁₂)-Cycloalkenyl, (C₇-C₁₂)-Poly- cycloalkyl, (C₇-C₁₂)-Polycycloalkenyl, (C₅-C₁₂)-Cycloalkyl-(C₁-C₄)-Alkyl, (C₅-C₁₂)-Cycloalkyliden-(C2-C4)-Alkyl, (C₅-C₁₂)-Cycloalkenyl-(C₁-C₄)-Alkyl, (C₇-C₁₂)-Polycycloalkyl-(C₁-C₄)-Alkyl, (C₇-C₁₂)- Polycycloalkyliden-(C₂-C₄)-Alkyl, (C₇-C₁₂)-Polycycloalkenyl-(C₁-C₄)-Alkyl oder ebendiese cyclischen Kohlenwasserstoffreste mit bis zu 3 gleichen oder verschiedenen Substituenten, wobei diese F, Cl, Br oder CH₃ bedeuten oder
   eine unsubstituierte oder eine im Phenylrest bis Zu 3 Substituenten tragende Phenyl-(C₂-C₄)-Alkyl-Gruppe, wobei die Substitutenten gleich oder verschieden sind und F, Cl, Br, (C₁-C₄)-Alkoxy, (C₁-C₄)-Alkyl oder (C₁-C₄)-Alkylthio bedeuten,
R⁴ H, (C₁-C₁₂)-Alkyl, (C₃-Cₛ)-Alkenyl, Cyclopropyl, (C₅-C₈)-Cycloalkyl, eine unsubstituierte oder eine im Phenylrest bzw. Naphthylrest bis zu 2 Substituenten tragende Phenyl-(C₂-C₄)-Alkyl- oder Naphthyl-(C₁-C₄ )-Alkylgruppe, wobei die Substituenten gleich oder verschieden sind und F, Cl, Br, OCH₃, OC₂Hₛ oder (C₁-C₄ )-Alkyl bedeuten, bedeuten, oder
R³ und R⁴ gemeinsam
   eine unsubstituierte oder mit (C₁-C₄)-Alkyl oder Phenyl substituierte - (CH₂)ₙ-Kette mit n= 4-11,
   oder eine ebensolche Kohlenwasserstoffkette mit einer Doppelbindung,
   oder eine unsubstituierte oder mit CH_{3,} C_{z}Hₛ, OCH₃ oder CH₃OCH₂ substituierte, einfach oder mehrfach überbrückte -(CH_{z})ₘ-Kette mit m = 4 oder 5 und 1 bis 5 Brücken- Kohlenstoffatomen, wobei die Brückenkohlenstoffatome ihrerseits wiederum überbrück: sein können und eine Überbrückung keine oder eine C,C-Doppelbindung enthält, bedeuten.

Besonders bevorzugt sind diejenigen Verbindungen der Formel I, in denen mindestens einer der Substituenten die folgende Bedeutung hat:
Aryl ein Rest 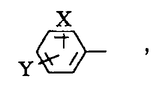 in dem
X (C₁-C₄)-Alkyl, Phenyl, F, CI oder (C₁-C₄)-Alkoxy,
Y H, CH₃, CI oder OCH₃ bedeuten und die 6-Stellung stets unsubstituiert ist, oder
Aryl ein unsubstituierter oder einfach mit Br oder CI substituierter 2-Naphthyl-Rest ist,
Q, R¹ und R² H,
R³ (C₁-C₈)-Alkyl, (C₅-C₇)-Cycloalkyl, (C₇-C₁₂)-Polycycloalkyl, (C₇-C₁₂)-Polycycloalkenyl, (C₇-C₁₂)-Polycycloalkyl-(C₁-C₄)-Alkyl oder ebendiese cyclischen Kohlenwasserstoffreste mit 1 oder 2 gleichen oder verschiedenen Substituenten, wobei diese Cl, Br oder CH₃ bedeuten oder
   eine unsubstituierte oder eine im Phenylrest bis zu 3 Substituenten tragende Phenyl-(C₂-C₄) Alkyl-Gruppe, wobei die Substitutenten gleich oder verschieden sind und F, CI, Br, (C₁-C₄)-Alkoxy oder (C₁-C₄)-Alkylthio bedeuten,
R⁴ H, (C₁-C₄)Alkyl, Cyclopentyl, Cyclohexyl, oder Cyclopropyl oder
R³ und R⁴ gemeinsam
   eine unsubstituierte oder mit CH₃ oder C₆ H₅ substituierte -(CH₂)ₙ-Kette mit n = 4 - 7, oder
   eine unsubstituierte oder mit CH₃ oder CH₃0CH₂ substituierte, einfach oder mehrfach überbrückte -(CH2)m-Kette mit m = 4 oder 5 und bis 5 Brücken- Kohlenstoffatomen, wobei die Brückenkohlenstoffatome ihrerseits wiederum überbrückt sein können und eine Überbrückung keine oder eine C,C-Doppelbindung enthält, bedeuten.
Von den Verbindungen der Formel l sind auch solche bevorzugt, bei denen
Aryl bedeutet 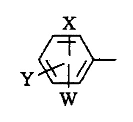
W Wasserstoff
X und Y Wasserstoff, F, CI oder Br
Q und R¹ bedeuten Wasserstoff
_{R}2 Wasserstoff
R³ und R⁴ gemeinsam
   eine (CH₂)ₙ-Kette mit n = 4 bis 6 oder R³ und R⁴ eine überbrückte (CH2)m-Kette mit m = 4 oder 5 und 1 bis 5 Brückenkohlenstoffatomen.

In diesem Zusammenhnng ist unter dem Ausdruck "(C₁-C₃)-,(C₁-C₄)-, (C₂-C₄)-, (C₁-C₈)- oder (C₁-C₁₂)-Alkyl" jeweils ein unverzweigter oder verzweigter Alkyl-Rest, unter dem Ausdruck "(C₃-Cs) oder (C₃-C₁₀)-Alkenyl" ieweils ein unverzweigter oder verzweigter Alkenyl-Rest, und unter dem Ausdruck "(C₁-C₄)-Alkoxy oder (C₁-C₄-)-Alkylthio ein unvezweigter oder verzweigter Alkoxy- oder Alkylthio-Rest zu verstehen.

Unter dem Ausdruck "(C₅-C₁₂)-Cycloalkyliden-(C₁-C₄)-Aikyil"bzw. "(C₇-C₁₂)-Polycycloalkyliden-(C₁-C₄)-Alkyl" sind Ringsysteme zu verstehen, bei denen ein Ringkohlenstoffatom und ein Kohlenstoffatom des geradkettigen oder verzveigten (C₁-C₄)-Alkyl-Restes durch eine Doppelbindung verbunden sind.

Als Beispiele für derartige Cyclo- bzw. Polycycloalkyliden Reste sind die nochstehenden Formelbilder (a) - (d) angeführt.

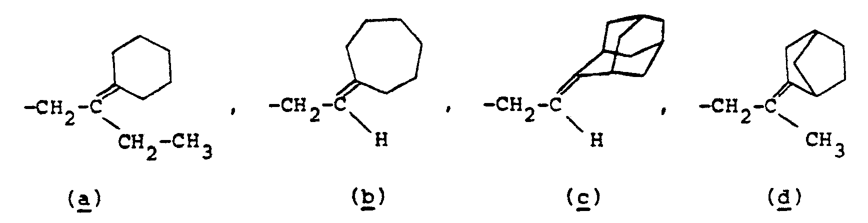

Unter dem Ausdruck "(C₇-C₁₂)-Polycycloalkyl" bzw. "(C₇-C₁₂)-Polycycloalkenyl" ist ein mehrcyclisches, isocyclisches Ringsystem mit 7 - 12 Ring-Kohlenstoffatomen zu verstehen, wobei dieses im Fall von "Polycyclonlkenyl" eine Doppelbindung enthält. Bei solchen mehrcyclischen Ringsystemen handelt es sich um anellierte oder überbrückte oder sowohl anellierte und überbrückte oder spirocyclische Systeme. Genannt seien hierzu beispielsweise: Bicydo[2.2.1]heptan und -hepten, Bicyclo[2.2.2]octan und -octen, Tricyclo[5.2.1.0.^{2.6}]decan und -decen, Adamantan, Oktalin, Dekalin, Bicyclo[4.3.0]nonan, Bicyclo[3.3.1]-nonan, Bicyclo[3.2.1]octan, Deltacyclan (e) oder Spiro[4,5]decan (f) 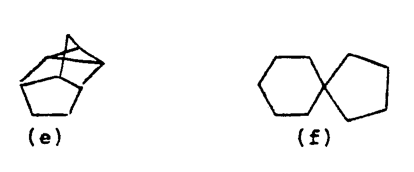 Enthält eines der möglichen Ringsysteme Benzolringe in ankondensierter (anellierter) Form, dann zählen je Benzolring 2 Kohlenstoffatome zur Anzahl der Ring-Kohlenstoffatome. Die mit solchen anellierten Benzolringen zwangsläufig in das jeweilige Ringsystem eingebrachten C,C-Doppelbindungen sind bei der Ausdrucksweise "Cycloalkenyl" bzw. "Polycycloalkenyl" nicht berücksichtigt. Diese Ausdrücke beziehen sich auf C,C-Doppelbindungen, die nicht zu einem Benzolring gehören. Als Beispiele für derartige, anellierte Benzolringe enthaltende Ringsysteme sind die Formelbilder (g) bis (p) zur Erläuterung angeführt.

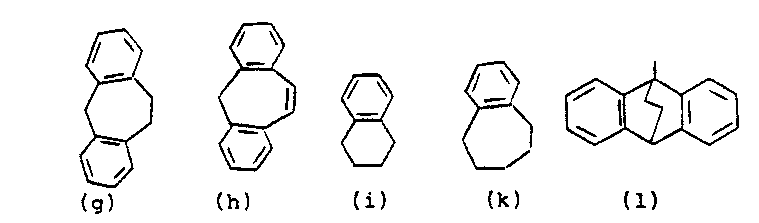

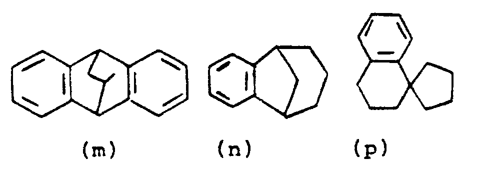

Die für "R³ und R⁴ gemeinsam" genannten Bedeutungen bezeichnen jeweils mono- oder polycyclische, isocyclische Ringsysteme, denen das den Sauerstoff tragende Kohlenstoffatom der Formel I angehört.

Ansonsten handelt es sich bei diesen Ringsystemen um gleiche oder analoge, wie sie voranstehend erläutert worden sind. Als Beispiele sind die Formelbilder (q) bis (y) aufgeführt.

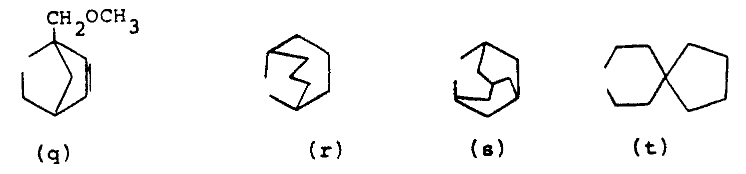

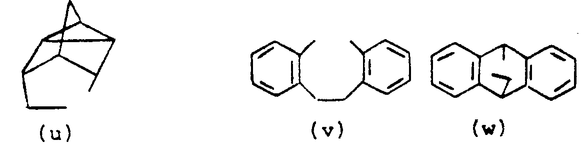

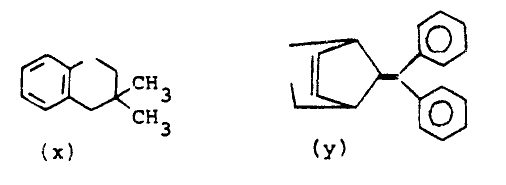

Unter dem Begriff "unsubstituierte oder bis zu 3 Substituenten tragende Kohlenwasserstoff-Reste" sind gesättigte oder ungesättigte, geradkettige oder verzweigte und/oder mono- oder polycyclische und/oder spirocyclische Kohlenwasserstoff-Reste zu verstehen. Diese können auch anellierte Benzolringe enthalten, und im Fall von cyclischen Kohlenwasserstoff-Resten können sie mit einem oder mehreren geradkettigen oder verzweigten Alkyl- oder Alkenyl-Resten oder mit einem oder mehreren Phenyl-Resten substituiert sein. Außerdem dürfen solche Kohlenwasserstoffreste auch Substituenten enthalten, die den Charakter der Kohlenwasserstoffe im wesentlichen unangetastet lassen, wie beispielsweise F, Cl, Br, (C₁-C₄)-Alkoxy- oder Phenoxy-Gruppen.

Mit dem Ausdruck "ungesättigte" sind eine oder mehrere C,C-Doppel- und/oder C,C-Dreifachbindungen enthaltende Kohlenwasserstoff-Reste gemeint.

Die Verbindungen der Formel I enthalten mindestens ein asymmetrisches C-Atom, und zwar dasjenige, woran der Azolrest gebunden ist. Außerdem ist, falls R³ und R⁴ verschieden sind oder gemeinsam ein unsymmetrisches Ringsystem bilden, das den Sauerstoff tragende C-Atom asymmetrisch. Weitere asymmetrische C-Atome können in den Resten R³ und/oder R⁴ enthalten sein. Demzufolge fallen die Verbindungen I bei der Synthese mindestens als Racemate an. Je nach Anzahl der Asymmetriezentren treten darüber hinaus Stereoisomere in Form von Racematen auf. Beispielsweise können bei der Synthese solcher Verbindungen der Formel I, die zusätzlich zu dem stets vorhandenen Asymmetriezentrum ein zweites an dem den Sauerstoff tragenden C-Atom enthalten, zwei Diastereomeren-Racemate in gleichen oder unterschiedlichen Mengen gebildet werden. Häufig fallen solche Diastereomeren-Racemate infolge von Stereoselektivität in unterschiedlichen Mengen an, oder im Extremfall wird nur eines gebildet. Die Erfindung betrifft daher auch die möglichen Stereoisomeren, im einfachsten Fall die Diastereomeren, der Verbindungen l in Form ihrer Racemate oder in Form der optisch aktiven Enantiomeren und die optisch aktiven Enantiomeren von den Verbindungen der Formel I, die nur das eine, stets vorhandene Asymmetriezentrum enthalten, sowie deren pharmazeutisch akzeptablen Salze.

Die Verbindungen der Formel können nach drei Verfahren hergestellt werden. Das Verfahren a) geht von Arylmethylazolen der Formel ll aus 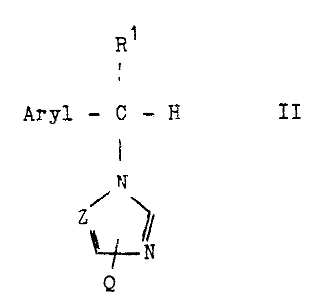 worin Aryl, Z, Q und R¹ die oben angegebenen Bedeutungen haben.

Aus Org. React. 26, S. 128 (1979) ist bekannt, daß bei der Umsetzung von Benzylimidazol mit n-Butyllithium in Diethylether bei Temperaturen zwischen 0 und -70 C und anschließender Zugabe von Carbonylverbindungen nur Hydroxyalkylierung am Imidazolring stattfindet.

Arylmethylazole können mit starken Basen und Carbonylverbindungen an der Methylengrupppe hydroxyalkyliert werden, wenn man mit stärker solvatisierenden Lösungsmitteln oder mit zwei Äquivalenten Base arbeitet.

Das Verfahren wird z.B. so durchgeführt, daß man ein Arylmethylazol der Formel ll in einem aprotischen, zweckmäßig polaren Lösungsmittel bei einer Temperatur zwischen +40° C und -100)° C mit einem oder zwei Äquivalenten einer starken Base versetzt und anschließend zuerst mit einer Carbonylverbindung der Formel III, 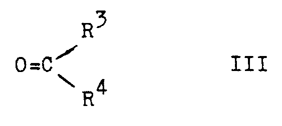 in welcher R³ und R⁴ die obengenannten Bedeutungen haben, und dann mit einer Protonsäure oder einem Alkylhalogenid der Formel IV, 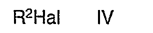 in welcher R² die oben angegebene Bedeutung hat und Hai für Chlor, Brom oder Jod steht, zu einer Verbindung der Formel I umsetzt.

Als starke Basen kommen vor allem Alkalimetallhydride, wie z.B. Natriumhydrid, oder Erdalkali- und Alkalimetallalkyle, wie z.B. n-Butyllithium , tert.-Butyllithium, Methyllithium, Phenyllithium oder Methylmagnesiumchlorid oder -bromid oder metallierte Amine, wie z.B. Lithiumdiisopropylamid, Kalium- oder Natriumamid, in Frage, vorzugsweise werden Lithiumalkyle, wie n-Butyllithium, verwendet.

Die Anwendung von zwei Äquivalenten starker Basen ist bei der Mehrzahl der gemäß der Erfindung in den als Ausgangsstoff zu verwenden den Arylmethylimidazolen vorkommenden Substituenten, wie auch bei im Arylrest unsubstituierten solchen Verbindungen der Formel II angebracht. Eine Ausnahme hiervon machen 1-Benzyl- oder 1-Naphthylimidazole, die im Phenyl- bzw. Naphthylrest mit ausgesprochenen Akzeptor-Substituenten, wie z.B. CF_{3,} oder mit F, CI und/oder Br in ortho- und/oder para-Position bei Benzylimidazolen bzw. in entsprechenden Positionen bei Naphthylimidazolen substituiert sind. Bei diesen und bei (3-Chlorphenyl-methyl)-imidazolen genügt die Anwendung von einem Äquivalent einer starken Base, vorzugsweise von Butyllithium, um ausreichend gute Ausbeuten an Verbindungen der Formel I zu erhalten.

Die Verwendung von zwei Äquivalenten starker Basen ist generell bei Arylmethyltriazolderivaten der Formel II (Z=N) nötig, andernfalls besteht die Gefahr, daß man Produkte erhält, die am Triazolring hydroxyalkyliert sind.

Die Reaktion kann in den für metallorganische Reaktionen gebräuchlichen Lösungsmitteln, wie z.B. Dimethylformamid, Dimethylsulfoxid, Dimethoxyethan, Diethylether oder vorzugsweise Tetrahydrofuran durchgeführt werden. Es können auch Mischungen verschiedener aprotischer Lösungsmittel, darunter auch Mischungen von polaren und unpolaren Lösungsmitteln, angewendet werden. Außerdem können als Lösungsmittel Zusätze wie N,N,N',N'-Tetramethylethylendiamin oder Hexamethylphosphorsäuretriamid Verwendung finden.

Die erfindungsgemäße Umsetzung mit der starken Base und der Carbonylverbindung der Formel III kann bei Temperaturen zwischen + 40 C und -100 C erfolgen, geschieht jedoch vorzugsweise im Bereich zwischen 0°C und -80° C. Die Umsetzung kann auch bei einer Temperatur oberhalb von +40° C ausgeführt werden. Die Verbindungen der Formel I werden in der bei metallorganischen Reaktionen üblichen und bekannten Weise isoliert. Die Reinigung der Verbindungen der Formel I erfolgt in der Regel durch Umkristallisation aus einem organischen Lösungsmittel, wie z.B. Hexan, Cyclohexan, Ethanol, Essigsäureethylester, Diisopropylether oder Acetonitril oder einem Lösungsmittelgemisch oder durch Säulenchromatographie an Kieselgel.

Die als Ausgangsstoff verwendeten, unsubstituierten oder erfindungsgemäß im Phenyl- oder Naphthyl- Rest substituierten 1-Benzylazole bzw. Azol-1-ylmethylnaphthaline der Formel II (R¹ = H) werden nach bekannten Methoden durch Alkylierung von Imidazol bzw. Triazol mit Benzylhalogeniden bzw. mit entsprechenden 1- oder 2-Chlor- oder Brommethylnaphthalinen erhalten.

Ausgangsstoffe der Formel II, worin R¹ (C₁-C₄)-Alkyl bedeutet, werden in analoger Weise nach bekannten Methoden durch Alkylierung von Imidazol bzw. Triazol mit entsprechenden a-Chlor- oder α-Bromalkyl-benzolen oder -naphthalinen der Formel VII, 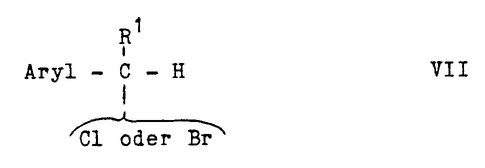 worin Aryl die oben angegebenen Bedeutungen hat und R¹ (C₁-C₄)-Alkyl bedeutet, hergestellt.

Die als Ausgangsstoffe benötigten Benzylhalogenide bzw. Chlor- oder Brommethyl-naphthaline sind großteils bekannt oder können nach bekannten Methoden hergestellt werden, beispielsweise durch Umsetzung entsprechender Hydroxymethyl-Verbindungen mit Thionylchlorid oder durch Bromierung entsprechender Methyl-Verbindungen mit NBS (N-Bromsuccinimid). Die Chlor- oder Bromalkyl-Verbindungen der Formel VII sind zum Teil bekannt oder können nach bekannten Methoden erhalten werden, beispielsweise aus entsprechenden Hydroxy-Verbindungen, die ihrerseits aus den entsprechenden Ketonen durch Reduktion mit NaBH₄ zugänglich sind, durch Einwirkung von Thionylchlorid.

Beim Verfahren b) geht man von Oxiranen der Formel V aus, 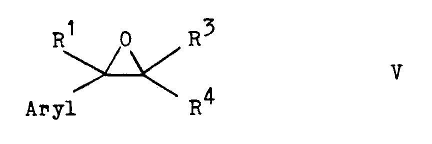 worin Aryl, R¹, R³ und R⁴ die obengenannte Bedeutung besitzen. Die Umsetzung zu Verbindungen der Formel I erfolgt z.B., indem man ein Epoxid der Formel V mit einer Verbindung der Formel VI, 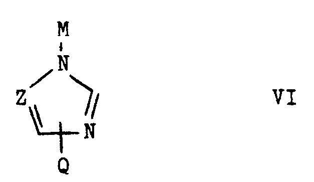 in der M Wasserstoff oder ein Alkali- oder Erdalkalimetall bedeutet und Q und Z die oben angegebene Bedeutung haben, bei einer Temperatur zwischen -20 C, vorzugsweise 0 ° C, und 150° C, vorzugsweise 60 C, bevorzugt in einem polaren Lösungsmittel nucleophil öffnet und dann mit einer Protonensäure oder einem Alkylhalogenid der Formel IV umsetzt.

Oxirane der Formel V erhält man nach literaturbekannten Methoden. Die Herstellung der zugrundeliegenden Olefine erfolgt ebenfalls nach bekannten Methoden. Die Oxidation der Olefine zum Epoxid geschieht mit einer organischen Persäure, wie z.B. Peressigsäure oder m-Chlorperbenzoesäure nach literaturbekannten Methoden.

Bevorzugt werden beim Verfahren b) Verbindungen der Formel VI verwendet, worin M Li, Na oder K, insbesondere Na, bedeutet. Die Verbindungen der Formel VI sind bekannt oder können nach bekannten Methoden hergestellt werden.

Nach dem Verfahren c) wird bei Verbindungen der Formel I, die nach Verfahren a) oder b) hergestellt worden sind, nach bekannten Methoden ein Substituent X oder V in einem Aryl-Rest der Formel I in einen anderen Substituenten X oder V umgewandelt.

Beispielsweise wird nach diesem Verfahren c) ein Benzyloxy-Rest in einer Phenyl- oder Naphthylgruppe durch katalytische Hydrogenolyse in eine Hydroxygruppe und Toluol übergeführt. Als Katalysatoren sind hierzu z.B. verschiedene Arten von feinverteiltem Palladium auf Aktivkohle oder auf Calziumcarbonat oder auf Bariumsulfat geeignet.

Die Reaktionszeiten betragen je nach Verfahrensvariante und je nach Temperaturbereich wenige Minuten bis einige Stunden.

Aus den Azolderivaten der Formel können Säureadditionssalze hergestellt werden. Dazu kommen alle Säuren, die physiologisch verträgliche Salze bilden, in Frage. Dazu gehören sowohl anorganische Säuren als auch mono-, bi- und trifunktionelle organische Säuren, wie beispielsweise Chlor-, Brom- oder Jodwasserstoff-, Schwefel-, Phosphor-, Salpeter-, Benzolsulfon-, Toluolsulfon-, Sulfamin-, Methylschwefel-, Essig-, Propion-, Öl-, Palmitin-, Stearin, Malon-, Malein-, Bernstein-, Glutar-, Äpfel-, Wein-, Zitronen-, Fumar- , Milch-, Glykol-, Brenztrauben-, Benzoe-, Toluyl-, Glutamin-, Furancarbon-, Salicyl- oder Mandelsäure. Bevorzugt werden Salze mit physiologisch verträglichen anorganischen Säuren, stark bis mittelstark sauren Derivaten solcher Säuren oder mit Essigsäure, Bernsteinsßäure, L(+)-Weinsäure, D(-)-Weinsäure, Äpfelsäure, Fumarsäure, (S)-(+)- oder (R)-(-)-Mandelsäure.

Die erfindungsgemäßen Verbindungen der Formel I enthalten ein oder mehrere Asymmetriezentren. Liegen zwei oder mehr Asymmetriezentren vor, erhält man bei der Synthese in der Regel Stereoisomerengemische. Die einzelnen Stereoisomeren können dabei infolge unterschiedlicher Stereoselektivität in unterschiedlichen Mengen anfallen. Bei hoher Stereoselektivität beispielsweise kann im Fall von Diastereomeren überwiegend oder nahezu ausschließlich nur ein Diastereomeres, als Racemat, entstehen. Diastereomere Racemate wie auch Diastereomere, bei denen beispielsweise ein Asymmetriezentrum in einheitlicher (R-oder S-) Konfiguration vorliegt, von Verbindungen der Formel I können in üblicher Weise, z.B. durch selektive, fraktionierte Kristallisation oder Säulenchromatographie getrennt werden.

Die diastereomeren Racemate können ihrerseits in üblicher Weise in ihre optischen Antipoden (Enantiomeren) getrennt werden.

Die Trennung von Racematen und diastereomeren Racematen kann nach im Prinzip bekannten Methoden erfolgen, beispielsweise durch fraktionierte Kristallisation diastereomerer Salze mit optisch aktiven Carbonsäuren oder Sulfonsäuren einheitlicher Konfiguration oder durch Chromatographie an chiralen Trennmedien (Trägerstoffen). Als optisch aktive, chirale Säuren sind beispielsweise geeignet: L(+)- oder D(- )-Weinsäure, D(+)- oder L(-)-Äpfelsäure, R-(-)- oder (S)-(+)-Mandelsäure, L(+)-Milchsäure, (+)-Campher-10-sulfonsäure oder (+)-3-Bromcampher-8-sulfonsäure. Diastereomere Salze derartiger chiraler Säuren mit Verbindungen der Formel I werden aus geeigneten Lösungsmitteln oder Lösungsmittelgemischen fraktioniert umkristallisiert, bis ein konstanter Drehwert erreicht ist.

Durch Einwirkung einer äquivalenten Menge oder eines Überschusses Base können die optisch reinen diastereomeren Salze in die reinen Enantiomeren der Verbindungen I übergeführt und als solche isoliert werden.

Werden bei der Synthese von Verbindungen nach Verfahren a) Carbonylverbindungen der Formel 111 in Form optisch reiner Enantiomerer verwendet, dann können bei ausreichender Kristallisationstendenz in der Regel die gebildeten Diastereomeren ohne chiralen Hilfsstoff, z.B. einer chiralen Säure, in bekannter Weise durch fraktionierte Kristallisation und/oder mittels chromatographischer Methoden in die optischen Antipoden getrennt werden.

Die chromatographische Trennung von Diastereomeren oder Racematen an chiralen Trägerstoffen ist mittels konventioneller Säulenchromatographie möglich, eine effektivere Trennung wird in der Regel mit der Mitteldruck- oder Hochleistungsflüssigkeitschromatographie erreicht.

Die Verbindungen der Formel und ihre Säureadditionssalze sind wertvolle Arzneimittel. Sie wirken insbesondere antimikrobiell und eignen sich zur Vorbeugung und Behandlung von Pilzinfektionen beim Menschen und bei verschiedenen Säugetierarten. Außerdem zeichnen sich erfindungsgemäße Verbindungen der Formel und ihre Säureadditionssalze durch eine starke psychotrope, insbesondere antidepressive Wirkung aus, sie können daher zur Behandlung von depressiven Zuständen verwendet werden. Sie sind innerhalb eines breiten Dosisbereichs wirksam. Die Höhe der verabreichten Dosis ist abhängig von der Art der gewünschten Behandlung, von der Verabreichungsweise, vom Zustand, vom Typ und von der Größe des behandelten Säugers. Bei oraler Dosierung werden befriedigende Ergebnisse mit Dosen von ab 0,1 mg, vorzugsweise ab 0,4 mg, bis zu 100 mg, vorzugsweise bis zu 30 mg, einer Verbindung der Formel I pro kg Körpergewicht erreicht.

Die Verbindungen der Formel (I) und ihre Säureadditionssalze stellen wertvolle Psychopharmaka dar. Sie sind in den für Antidepressiva spezifischen Testmodellen biochemischer und pharmakologischer Art sehr gut wirksam.

Die erfindungsgemäßen Verbindungen antagonisieren mit einer ED₅₀ von 0,5 - 100 mg/kg oral appliziert, die Tetrabenazin-Ptosis an der Maus und an der Ratte. Sechs männl. Tieren wird die entsprechende Menge eines Homogenats der jeweiligen Substanz in wäßriger Carboxymethylzellulose eine Stunde vor der Behandlung mit Tetrabenazin (40 mg/kg intraperitoneal) mit Schlundsonde gefüttert. Die Tetrabenazin-Ptosis wird durch diese Vorbehandlung antagonisiert.

Die Verbindungen I und ihre Säureadditionssalze potenzieren⁻die Toxizität von Yohimbin an Mäusen. Antidepressiva des Typs mit 0,5 bis 100 mg/kg per os verursachen die Erhöhung der Todesrate einer sonst nicht tödlichen Dosis von Yohimbinhydrochlorid (20 mg/kg subkutan gegeben).

Ein wesentlicher Befund ist, daß die Verbindungen und ihre Salze an Ratten und Mäusen auch nach Dosierungen bis zu 300 mg/kg p.o. keine Stereotypien verursachen.

Die antidepressive Wirkung und die Brauchbarkeit zur Behandlung von depressiven Zuständen wurde weiterhin anhand der Hemmung der Wiederaufnahme von Noradrenalin an Mäusehirn-Synaptosomen-Präparationen demonstriert.

Die Verbindungen sowie ihre Säureadditionssalze sind besonders wertvoll durch eine von den bisher bekannten Antidepressiva abweichende Struktur. Bekannten Handelsprodukten sind sie in ihrer Wirkung bei geringerer Toxizität gleichwertig oder überlegen.

Die Verbindungen der Formel I sind außerdem in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen gram-positive und gramnegative Bakterien.

Auch in vivo, z.B. bei der experimentellen Nierencandidose der Maus, besitzen die Verbindungen nach oraler oder parenteraler Anwendung einen guten systemischen Effekt, z.B. gegen Candida albicans. Ebenso besteht ein sehr guter Effekt gegen verschiedene Erreger der Hautmykosen (z.B. Trichophyton mentagrophytes) am Meerschweinchen nach oraler, parenteraler oder insbesondere lokaler Anwendung.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutischen geeigneten Trägerstoffen sind feste, halbfeste oder flüssige Verdünnungsmittel, Füllstoffe und Formulierungshilfsmittel jeder Art zu verstehen.

Als geeignete Anwendungsformen der erfindungsgemäßen Verbindungen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen zweckmäßig in einer Konzentration von etwa 0,01, vorzugsweise von 0,10 bis 99,0 vorzugsweise bis 50,0, Gewichtsprozenten der Gesamtmischung vorhanden sein.

Die Anwendungskonzentrationen für Lösungen, Gelees, Cremes oder Salben sowie Aerosole in Form von Spray betragen im allgemeinen zwischen 0,1 - 20, vorzugsweise 0,5 - 5 Gewichtsprozenten.

Für die lokale Anwendung können z.B. Suspensionen, Lösungen, Gelees, Cremes, Salben oder Suppositorien verwendet werden.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und im Fall von antimykotisch wirksamen Verbindungen in der Human- und Veterinärmedizin zur Verhütung, Besserung und/oder Heilung der oben angeführten Erkrankungen.

Bei antimykotisch wirkenden erfindungsgemäßen Verbindungen hat es sich sowohl in der Human- als auch in der Veterinärmedizin als vorteilhaft erwiesen, den oder die erfindungsgemäßen Wirkstoffe in Gesamtmengen von etwa 0.05 bis etwa 200, vorzugsweise 0,1 bis 100, insbesondere 0,5 bis 30 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen verabreicht.

Die Wirkstoffe oder die pharmazeutischen Zubereitungen können lokal, parenteral, intraperitoneal und/oder rectal appliziert werden.

Die als Antidepressiva verwendbaren Verbindungen der vorliegenden Erfindung und ihre Salze können zur Herstellung von pharmazeutischen Präparaten verwendet werden, welche eine wirksame Menge der Aktivsubstanz zusammen mit Trägerstoffen enthalten und die sich zur enteralen und parenteralen Verabreichung eignen. Vorzugsweise verwendet werden Tabletten oder Gelatinekapseln, welche den Wirkstoff zusammen mit Verdünnungsmitteln, z.B. Lactose, Dextrose, Rohrzucker, Mannitol, Sorbitol, Cellulose und/oder Glycin und Gleitmitteln wie Kieselerde, Talk, Stearinsäure oder deren Salze, wie Magnesium- oder Calciumstearat, und/oder Polyethylenglykol enthalten. Tabletten enthalten ebenfalls Bindemittel wie Magnesiumaluminiumsilicat, Stärke, Gelatine, Traganath, Methylcellulose, Natriumcarboxymethylcellulose und/oder Polyvinylpyrrolidon und, falls benötigt, Farbstoffe, Geschmacksstoffe und Süßmittel. Injizierbare Lösungen sind vorzugsweise isotonische wäßrige Lösungen oder Suspensionen, die sterilisiert sein können und Hilfsstoffe wie Konservier-, Stabilisierungs-, Netz- und/oder Emulgiermittel, Löslichkeitsvermittler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z.B. mittels konventioneller Misch-Granulier- und Dragierverfahren, hergestellt und enthalten 0,1 % bis etwa 75 %, bevorzugt etwa 1 % bis etwa 50 % des Wirkstoffs.

Die orale Anwendung erfolgt in pharmazeutisch üblichen Zubereitungen, beispielsweise in Form von Tabletten, Dragees oder Kapseln, die z.B. pro Tagesdosis 5, vorzugsweise 50, bis 600 mg, vorzugsweise bis 300 mg, des Wirkstoffes in Mischung mit einem gebräuchlichen Trägerstoff und/oder Konstituents enthalten, wobei Einzeldosen von 5 bis 200 mg, vorzugsweise ein- bis dreimal täglich, gegeben werden können.

Für die parenterale Anwendung kommen geeignete Suspensionen oder Lösungen in einer Anwendungskonzentration zwischen 0,1 - 10 Gewichtsprozenten in Frage.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen der Formel I zeichnen sich durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener phytopathogener Pilze, wie z. B. Piricularia oryzae, Pellicularia sasakii, verschiedene Rostarten, vor allem aber Venturia inaequalis, Cercospora-Arten und Echte Mehltaupilze im obst-, Gemüse-, Getreide- und Zierpflanzenbau.

Die Verbindungen der Formel eignen sich auch für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z. B. durch Auflösen des Wirkstoffes in einem inerten organischen Lösungsmittel, z.B. Butanol, clohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden:
Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylen-sorbitanfettsäureester oder Polyoxethylensorbitester.

Stäubemittel kann man durch Vermahlen des Wirkstoffes mit fein verteilten, festen Stoffen, z.B. Talkum, natürlichen Tonen wie Kaolin, Bentonit, Pyrophyllit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaoliniten oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.- % besteht aus üblichen Formulierungsbestandteilen. Bei emulgierbaren Konzentraten kann die Wirkstoffkonzentration etwa 10 bis 80 Gew.-% betragen.

Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die Jeweils üblichen Haft-, Netz-, Dispergier-, Emulgier-, Penetrations-, Lösungsmittel-, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich. Insbesondere bei Mischungen mit Fungiziden werden teilweise auch synergistische Wirkungssteigerungen erzielt.

Die folgenden Beispiele erläutern die Herstellung der Verbindungen. Die in den Beispielen 1 bis 167 beschriebenen Umsetzungen wurden unter einer Stickstoffatmosphäre durchgeführt.

### Beispiel 1 (Verbindung 1)

### 4-Chlorphenyl-1-(1-hydroxycyclohexyl)-1-imidazolylmethan

Zu einer Lösung von 9,65 g (50 mmol), N-(4-Chlorbenzyl)imidazol in 100 ml Tetrahydrofuran wurden bei -70 C 33 ml (50 mmol) einer Lösung von n-Butyllithium in Hexan getropft. Man rührte eine halbe Stunde nach und gab dann 4,9 g (50 mmol) Cyclohexanon in 20 ml Tetrahydrofuran so zu, daß die Temperatur bei -70 C gehalten werden konnte. Anschließend rührte man noch eine Stunde bei -10°C, ließ das Reaktionsgemisch in einer Stunde auf Zimmertemperatur aufwärmen, versetzte mit Wasser und extrahierte mit Methylenchlorid. Die Methylenchloridphase wurde getrocknet und eingeengt. Man erhielt 14,5 g Rohprodukt, das aus Cyclohexan/Essigsäureethylester (5:1) umkristallisiert wurde, und 9,3 g Reinprodukt vom Fp. 145 C ergab.

^{C}₁₆^{H}₁₉C1N₂0 (290, 78)

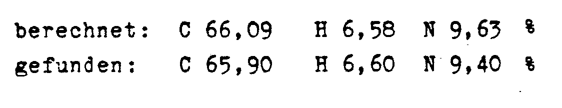

### Beispiel 2 (Verbindung 2)

### (2-Chlor-6-fluorphenyl)-1-(1-hydroxycyclopentyl)-1-imidazolylmethan

105 g (0,5 Mol) N-(2-Chlor,6-fluorbenzyl)imidazol wurden in 1 I Tetrahydrofuran gelöst, auf -78° C abgekühlt und bei -70° C mit 320 ml (0,5 Mol) n-Butyllithium in Hexan metalliert. Nach einer halben Stunde gab man dann, ebenfalls bei -70^{°} C, 42 g (0,5 Mol) Cyclopentanon zu und ließ das Reaktionsgemisch innerhalb von drei Stunden auf Zimmertemperatur aufwärmen. Anschließend versetzte man mit Wasser und extrahierte mit Methylenchlorid. Einengen der organischen Phase ergab 142 g Öl, aus dem mit Cyclohexan/ Essigsäureethylester (1:1) 87 g Kristalle vom Fp. = 142^{°} C erhalten wurden.

C₁₅H₁₆C1FN₂0 (294,76)

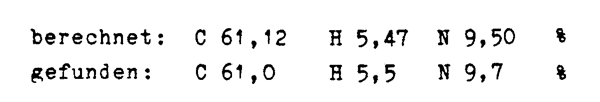

### Beispiel 3 (Verbindungen 3a und 3b)

### 2,4-Dichlorphenyl-9-(9-hydroxy-tricyclo[5.2.1^{1 7.}0^{2 6}]-dec-3-enyl)-1-imidazolylmethan

Zu einer analog Beispiel 1 hergestellten Lösung von 11,6 g (50 mmol 1-Imidazolyl-2.4-dichlorphenylme- thyllithium in 100 ml Tetrahydrofuran gab man bei -75 ^{°} C langsam 7,5 g (50 mmol) 9-Keto-tricyclo-[5.2.1^{1 7.}0^{2 6}]dec-3-en, entfernte die Kühlung und rührte, bis das Reaktionsgemisch Zimmertemperatur erreicht hatte. Nach Aufarbeitung mit Wasser und Methylenchlorid blieben 18 g Rohprodukt. Aus diesem isolierte man durch Umkristallisation mit Hexan/ Ethanol (10:1) 4,7 g Kristalle vom Schmelzpunkt 212 C.

^{C}₂₀^{H}₂₀^{C1}₂^{N}₂⁰ (375,2₉)

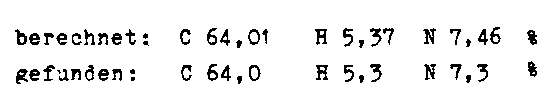

Einengen der Mutterlauge und Aufkochen des Rückstandes mit Isopropylether lieferte 7,3 g eines zweiten Isomeren vom Fp. = 150 C.

^{C}₂₀^{H}₂₀^{C1}₂N₂0 (375,29)

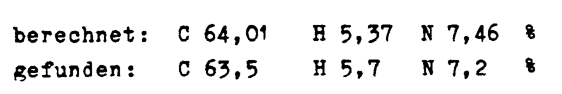

### Beispiel 4 (Verbindung 4)

### 1 -(4-Biphenyl)-3,3-dimethyl-1-(1-imidazolyl)-butan-2-ol

Zu einer analog Beispiel 1 hergestellten Lösung von 7,2 g (30 mmol) 1-lmidazolyl-4-biphenylmethyllithi- um in 100 ml Tetrahydrofuran tropfte man bei -70^{°} C langsam 2,6 g (30 mmol) Pivalaldehyd in 20 ml Tetrahydrofuran und ließ auf Zimmertemperatur aufwärmen. Die Aufarbeitung mit Wasser/Methylenchlorid ergab 9,4 g eines Öls, aus dem nach Umkristallisation mit Cyclohexan/Ethylacetat (1:1) 4,8 g Produkt vom Schmp. 166 ^{°} C erhalten wurden.

^{C}₂₁^{H}₂₄^{N}₂⁰ (320,42)

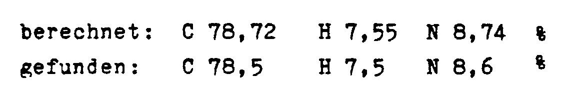

### Beispiel 5 (Verbindung 5)

### 2-Allyloxy-2-cyclohexyl-1-(2,4-dichlorphenyl)-1-(1-imidazolyl)-ethan

Zu 11,4 g (50 mmol) N-(2.4-Dichlorbenzyl)-imidazol in 100 ml Tetrahydrofuran wurden bei -78^{°} C 33 ml (50 mmol) n-Butyllithium in Hexan gegeben, eine halbe Stunde nachgerührt und dann 6,1 g (55 mmol) Hexahydrobenzaldehyd zugetropft. Man hielt noch zwei Stunden bei -70 ^{°} C, ließ dann auf 0^{°} C aufwärmen und versetzte mit 6,7 g (55 mmol) Allylbromid in 50 ml Tetrahydrofuran. Das Reaktionsgemisch wurde anschließend 15 Stunden bei Zimmertemperatur gerührt und mit Wasser/Methylenchlorid aufgearbeitet. Man erhielt 19,8 g Rohprodukt als Öl, aus dem säulenchromatographisch an Kieselgel mit Cyclohexan/Essigsäureethylester (4:1) 5,5 g des Imidazolderivats isoliert wurden.

^{C}₂₀^{H}₂₄^{C1}₂^{N}₂0 (379,32)

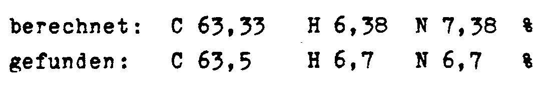

### Beispiel 6 (Verbindung 6)

### 1-Cyclohexyl-2-(2,4-dichlorphenyl)-2-(1-imidazolyl)-propan-1-ol

Man stellte analog Beispiel 1 eine Lösung von 12,4 g (50 mmol) 1-(1-Imidazolyl)-1-(2,4-dichlorphenyl)-ethyllithium her und tropfte bei -70 ^{°}C 5,6 g (50 mmol) Cyclohexanal zu. Man rührte noch 2 Stunden bei -70 C, ließ das Gemisch auf Zimmertemperatur kommen und arbeitete mit Wasser/ Methylenchlorid auf. Nach Einengen der organischen Phase blieben 18 g eines Öle, aus dem mittels Säulenchromatographie an Silicagel mit Cyclohexan/Essigsäureethylester (2 : 1) 4,1 g Produkt als Öl isoliert wurden.

^{C}₁₈^{H}₂₂^{Cl}₂^{N}₂⁰ (353,28)

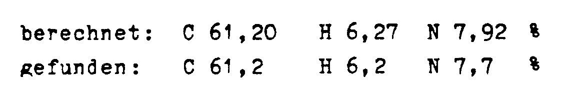

### Beispiel 7 (Verbindung 7)

### 2-Bromphenyl-1-(1-hydroxycycloheptyl)-1-imidasolylmethan

In 100 ml Tetrahydrofuran wurde bei -30^{°} C aus 5,1 g (50 mmol) Diisopropylamin und 33 ml (50 mmol) n-Butyllithium in Hexan eine Lösung von Lithiumdiisopropylamid hergestellt. Hierzu tropfte man bei -78° C 11,85 g (50 mmol) N-(2-Brombenzyl)-imidazol, rührte eine halbe Stunde nach und gab dann bei dieser Temperatur 5,6 g (50 mmol) Cycloheptanon zu. Man erwärmte das Reaktionsgemisch innerhalb von 3 Stunden auf Zimmertemperatur und arbeitete mit Wasser und Methylenchlorid auf. Man erhielt 12 g Rohprodukt, das nach Umkristallisation mit Cyclohexan/Essigsäureethylester 5,5 g Imidazolderivat vom Schmelzpunkt 108^{°} C ergab.

### Beispiel 8 (Verbindung 8)

### 1-(1-Hydroxycycloheptyl)-1-imidazolyl-phenylmethan

7,9 g (50 mmol) N-Benzylimidazol wurden in 150 ml Tetrahydrofuran gelöst, 6,0 g (52 mmol) N,N,N',N'-Tetramethylethylendiamin zugegeben und auf -78° C abgekühlt. Anschließend gab man 66 ml (100 mmol) n-Butyllithium in Hexan so zu, daß die Temperatur des Reaktionsgemisches -50^{°} C nicht überstieg. Man rührte noch 20 Minuten bei -70 ^{°} C, tropfte 5,6 g Cycloheptanon zu und ließ auf Zimmertemperatur aufwärmen. Nach Aufarbeitung mit Wasser/Methylenchlorid und Umkristallisation mit Essigsäureethylester erhielt man 5,8 g Kristalle vom Schmp. 175^{°} C.

C₁₇H₂₂N₂0 (270,36)

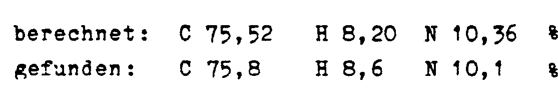

### Beispiel 9 (Verbindung 9)

### 2-Chlorphenyl-2-(2-hydroxyadamantyl)-1-(1,2,4-triazolyl)-methan

Zu einer Lösung von 9,7 g (50 mmol) 1-(2-Chlorbenzyl)-1,2,4-triazol und 6 g (52 mmol) N,N,N',N'-Tetramethylethylendiamin in 100 ml Tetrahydrofuran tropfte man bei -78^{°} C 66 ml (100 mmol) n-Butyllithium in Hexan, rührte eine halbe Stunde bei dieser Temperatur nach und gab dann 7,5 g (50 mmol) Adamantanon in Tetrahydrofuran so zu, daß -70 C nicht überschritten wurden. Das Reaktionsgemisch wurde noch 2 Stunden bei -78 C gehalten und dann auf Zimmertemperatur aufwärmen gelassen. Nach Aufarbeitung mit Wasser/Methylenchlorid erhielt man 17,4 g Rohprodukt, das nach Umkristallisation aus Essigsäureethylester 10,0 g reines Triazolderivat vom Schmp. 178° C ergab.

C₁₉H₂₂C1N₃0 (343,86)

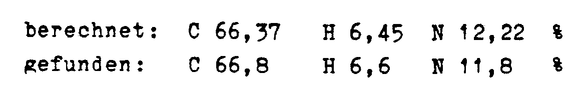

### Beispiel 10 (Verbindung 10)

### 2,4-Dichlorphenyl-1-(1-hydroxycyclopentyl)-1-(1.2.4-triazolyl)-methan

11,4 g (50 mmol) 1-(2,4-Dichlorbenzyl)-1,2,4-triazol wurden in 100 ml Tetrahydrofuran gelöst und bei -50 C mit 66 ml (100 mmol) n-Butyllithium in Hexan metalliert. Nach 30 Minuten wurden ebenfalls bei -50^{°} C 4,2 g (50 mmol) Cyclopentanon zugegeben, eine Stunde nachgerührt und das Gemisch anschließend langsam auf Zimmertemperatur erwärmt. Die Aufarbeitung mit Wasser/Methylenchlorid ergab 14 g eines Öls, aus dem durch Aufkochen mit Essigsäureethylester 7,4 g Kristalle vom Schmp. 110 ^{°} C erhalten wurden.

C ₁₄H₁₅ Cl₂ N₃0 (312,21)

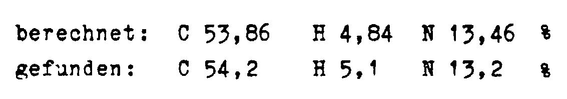

Analog zu den Beispielen 1 - 10 wurden folgende Verbindungen der Formel I mit Z=CH und Q=H erhalten:

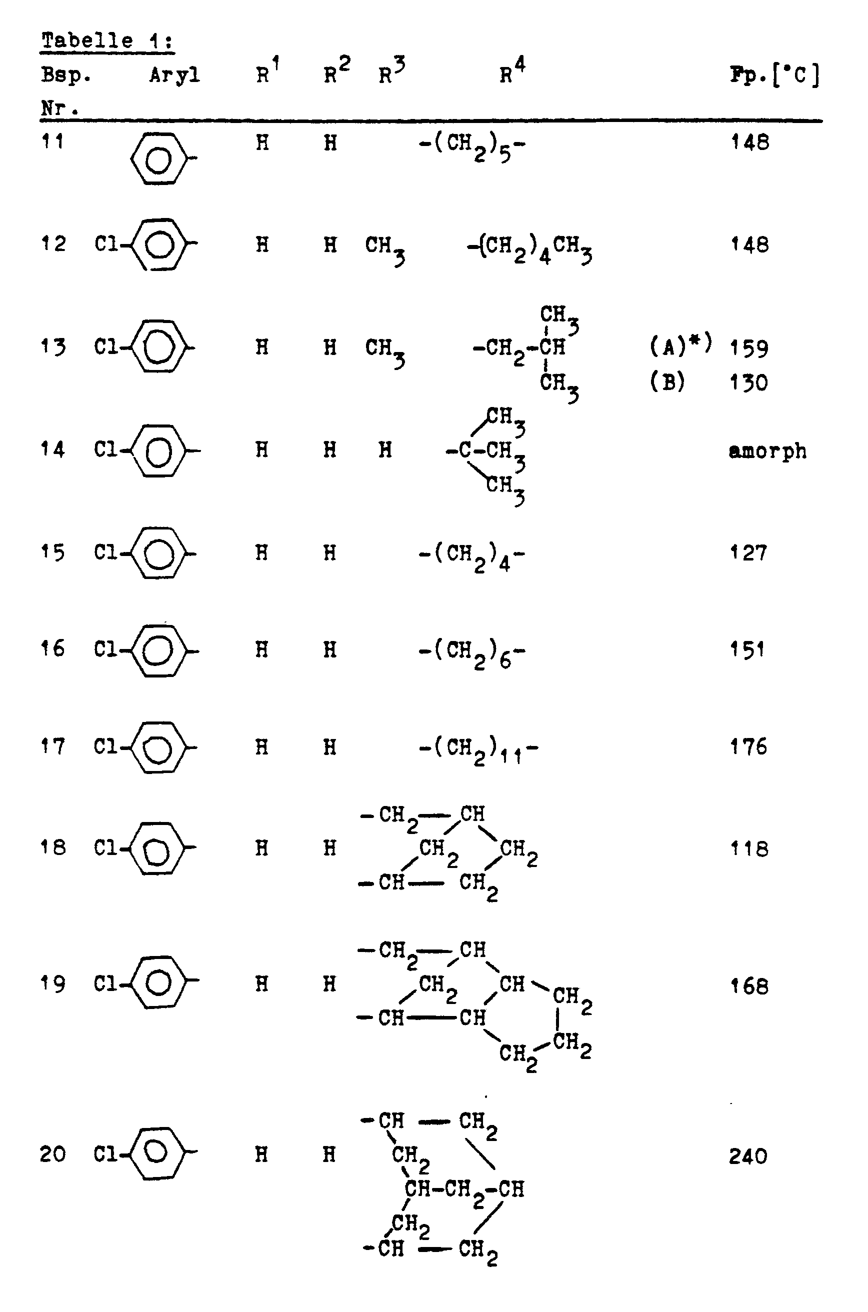

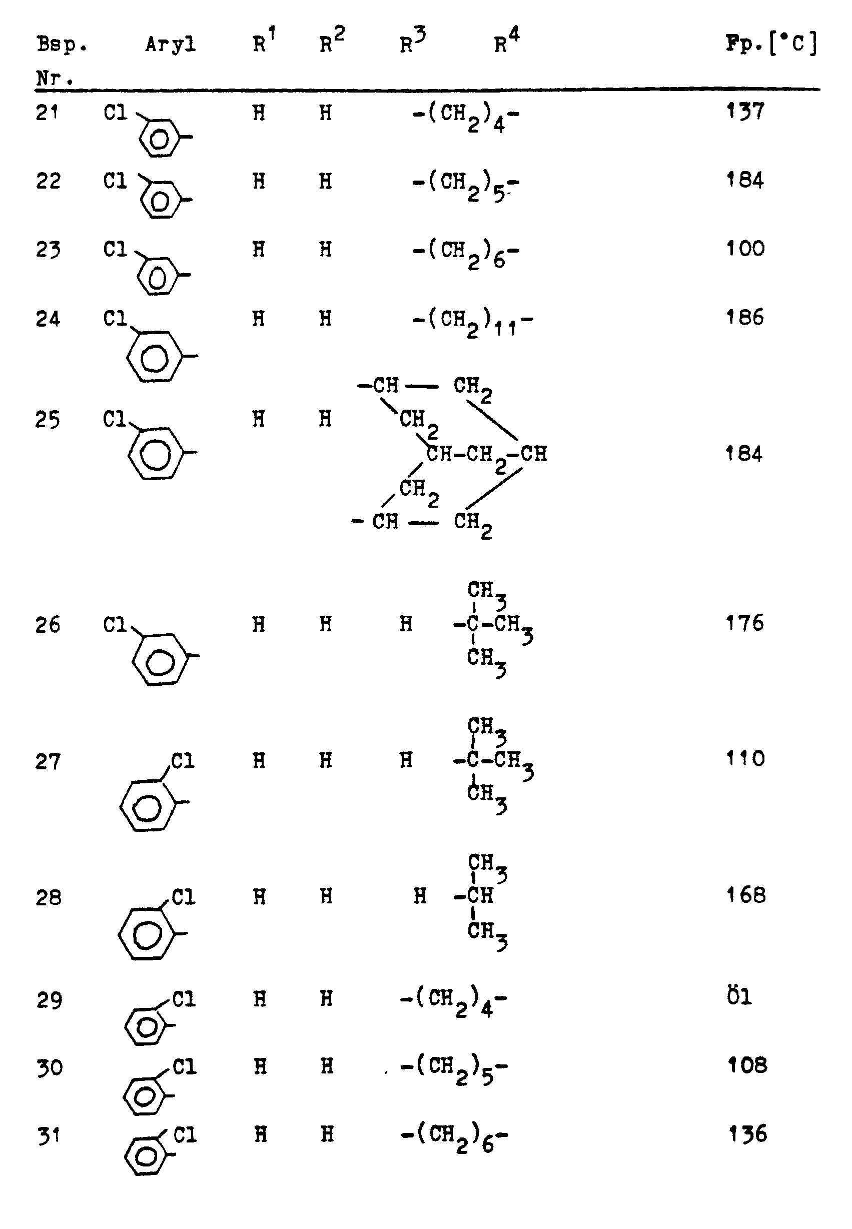

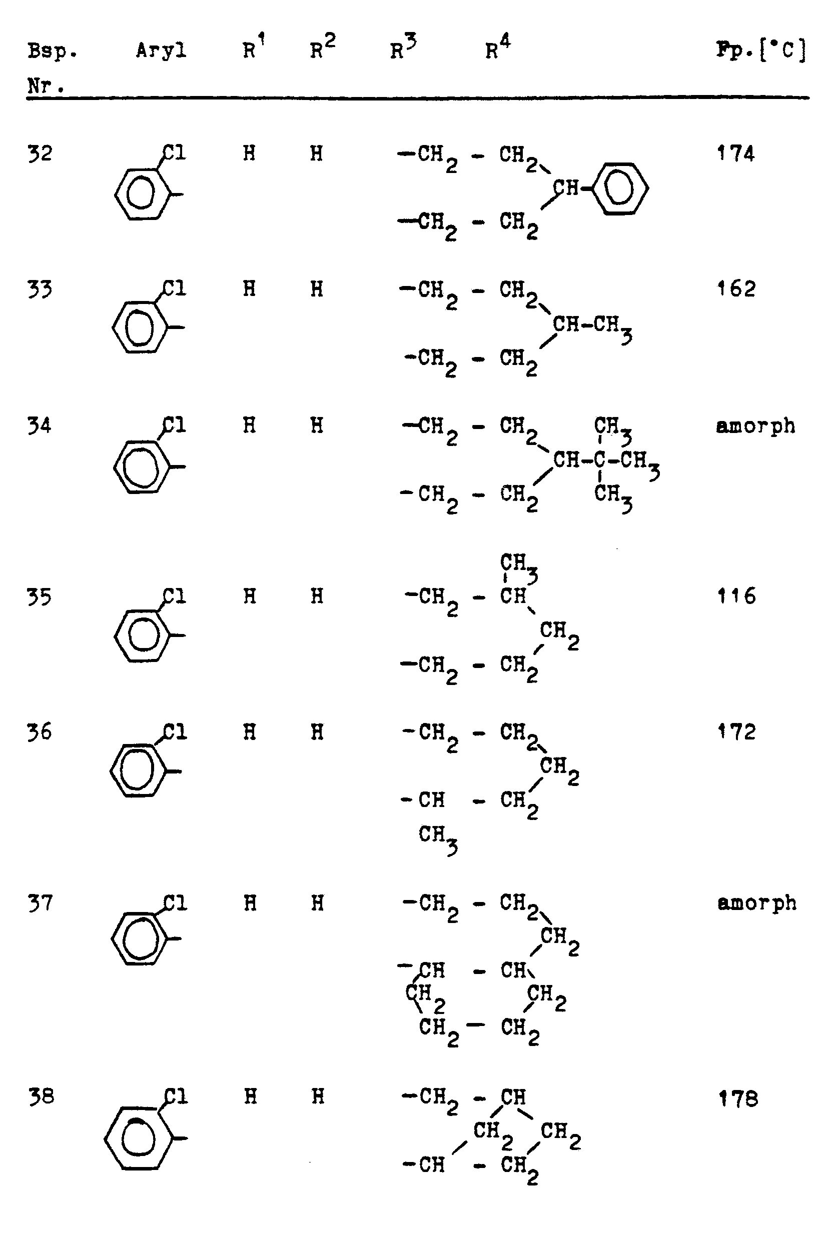

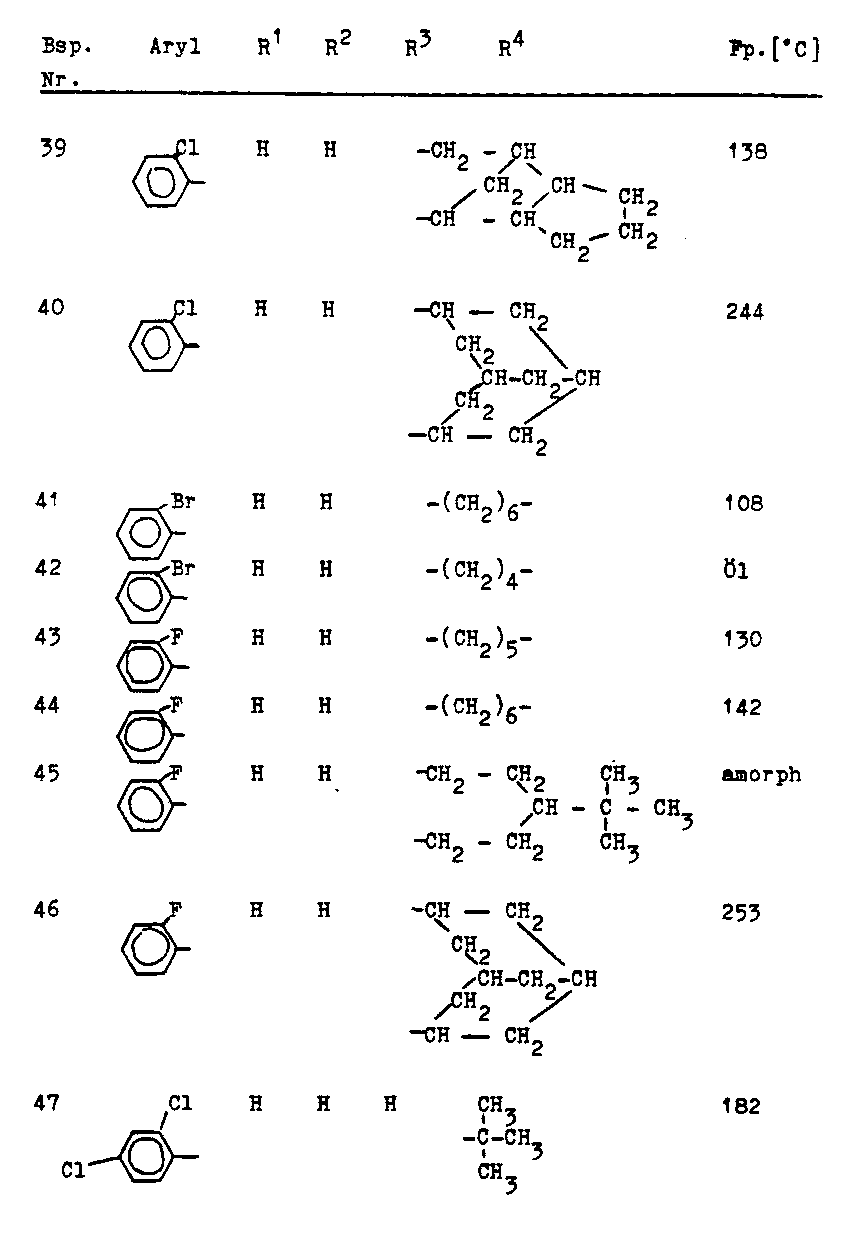

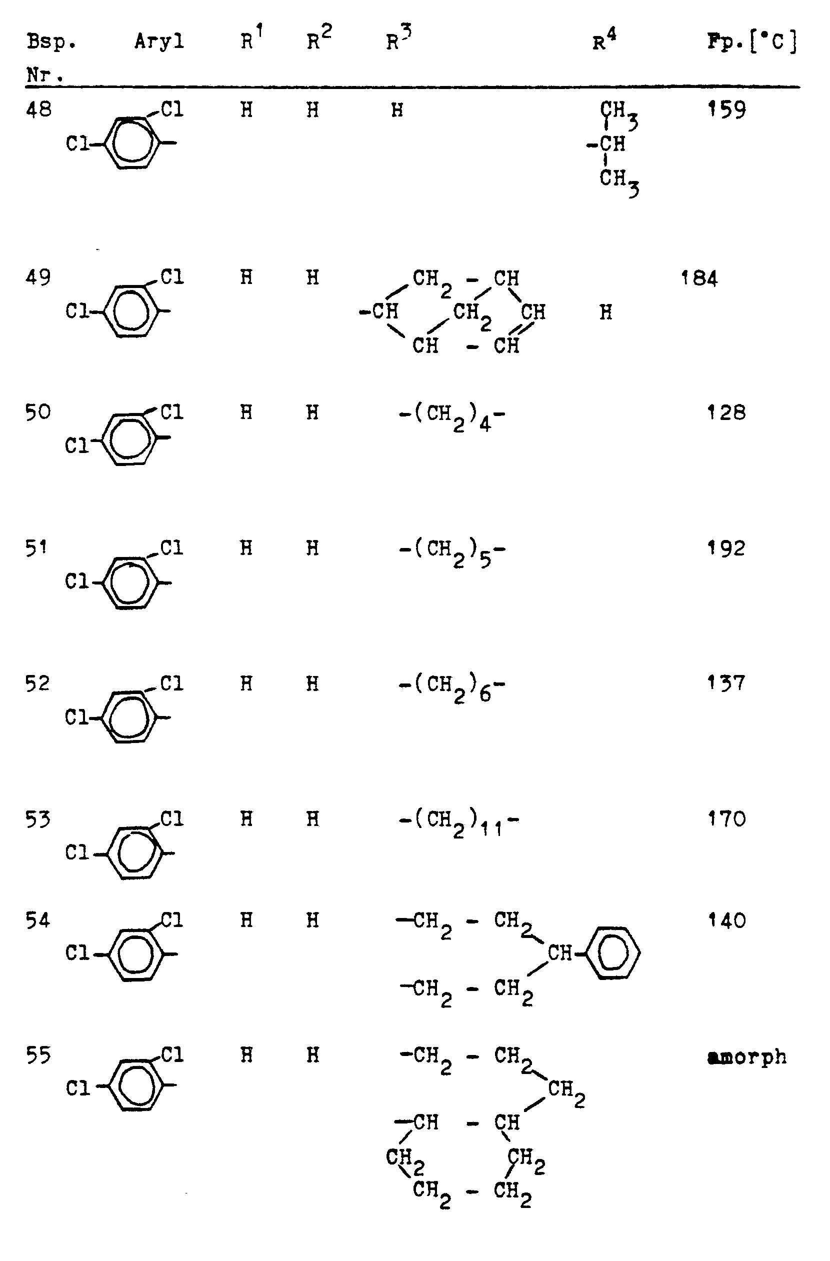

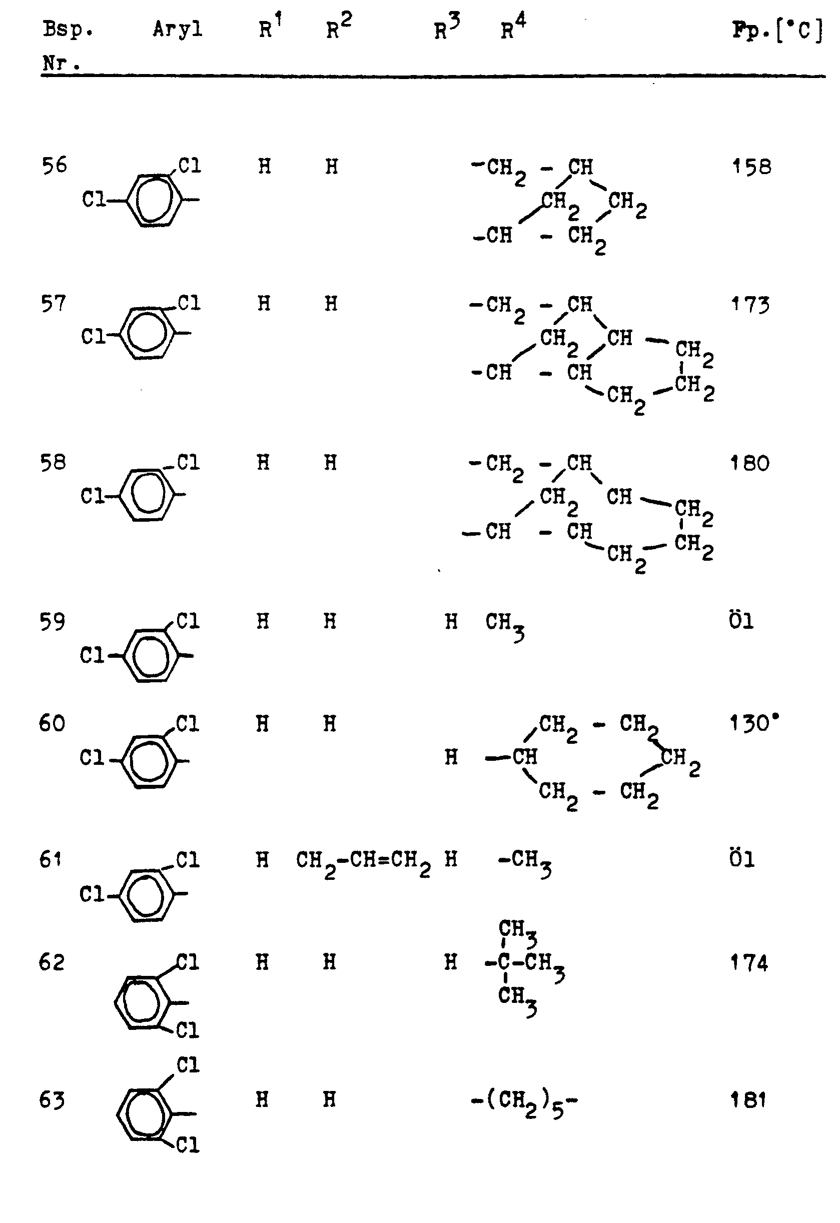

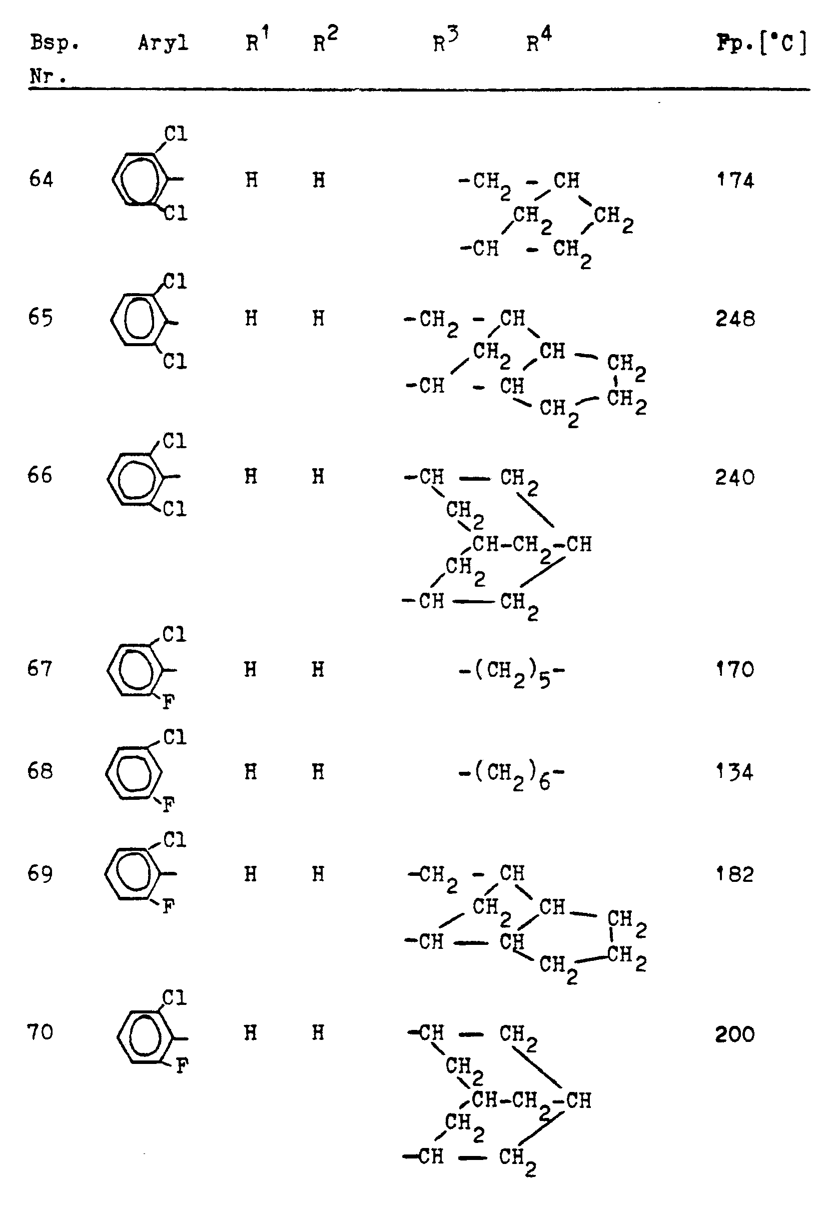

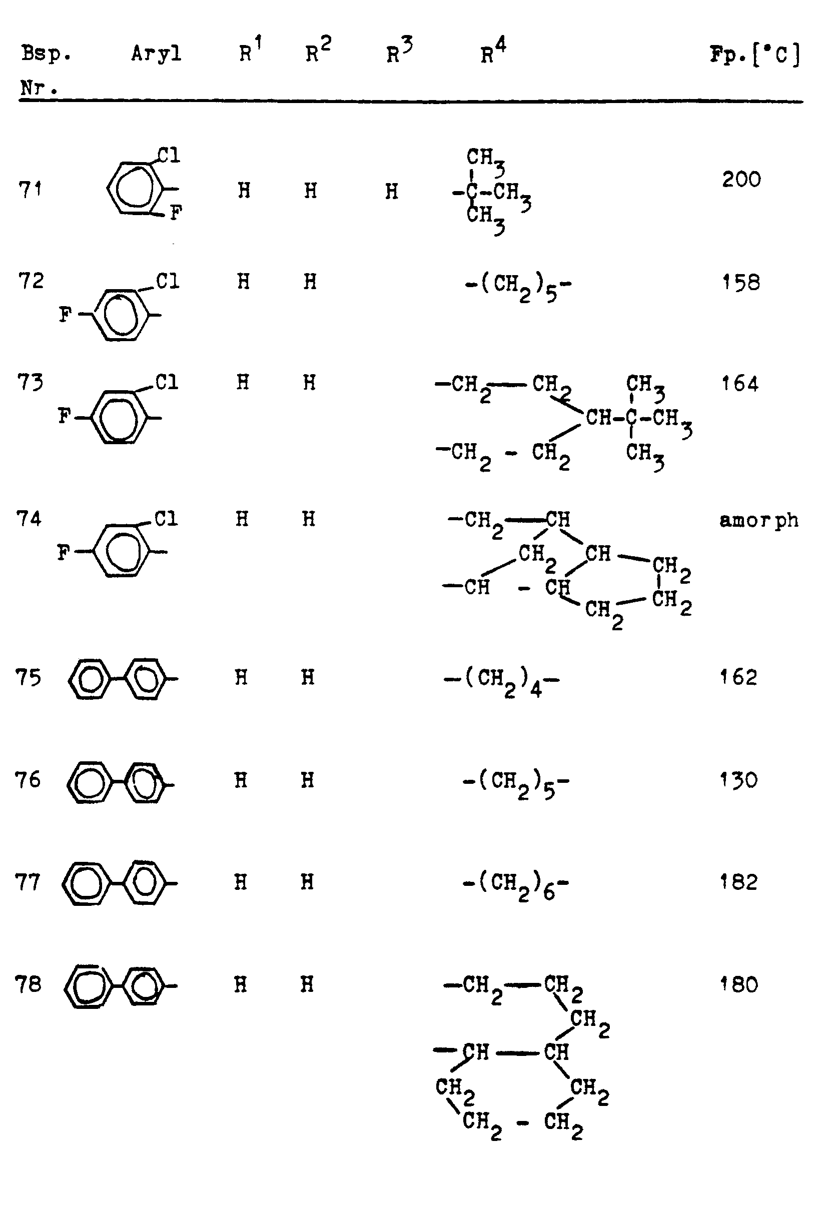

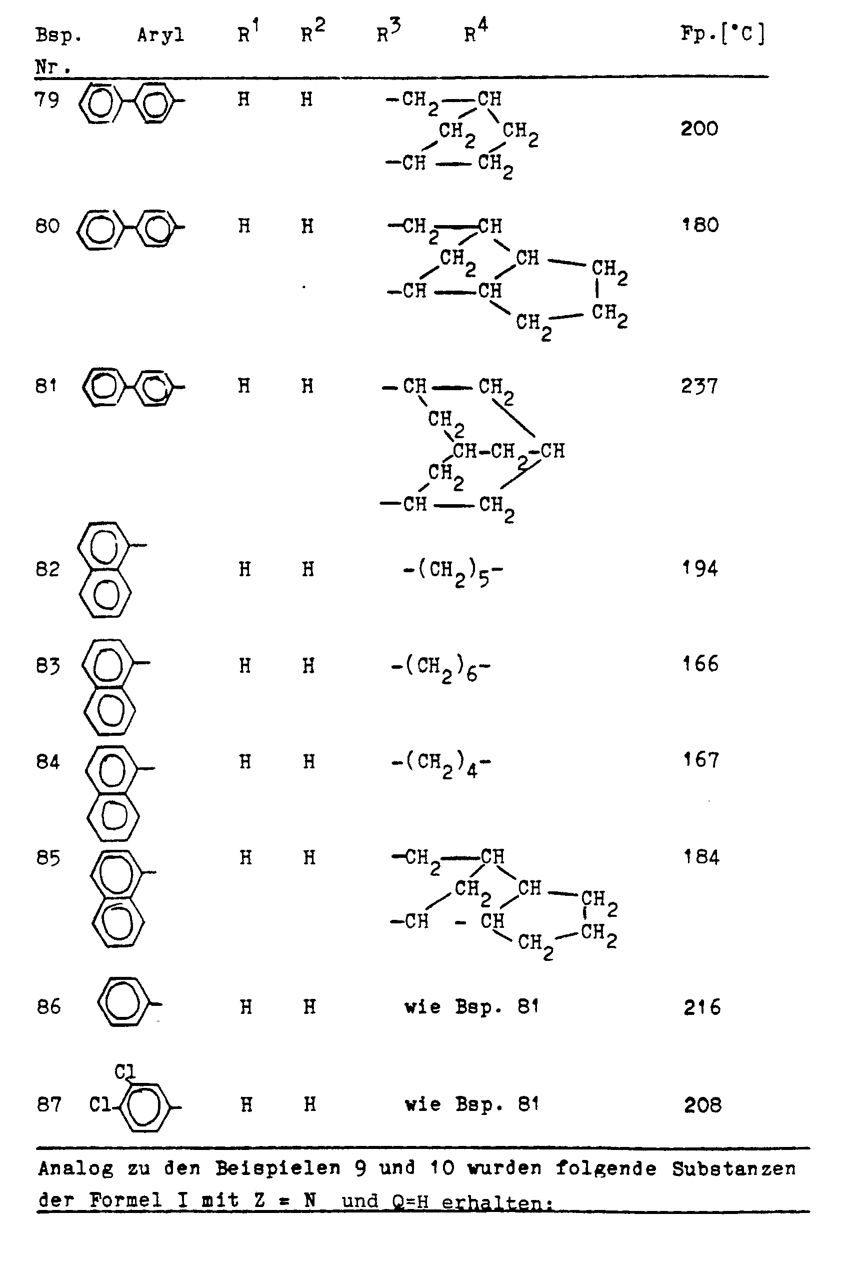

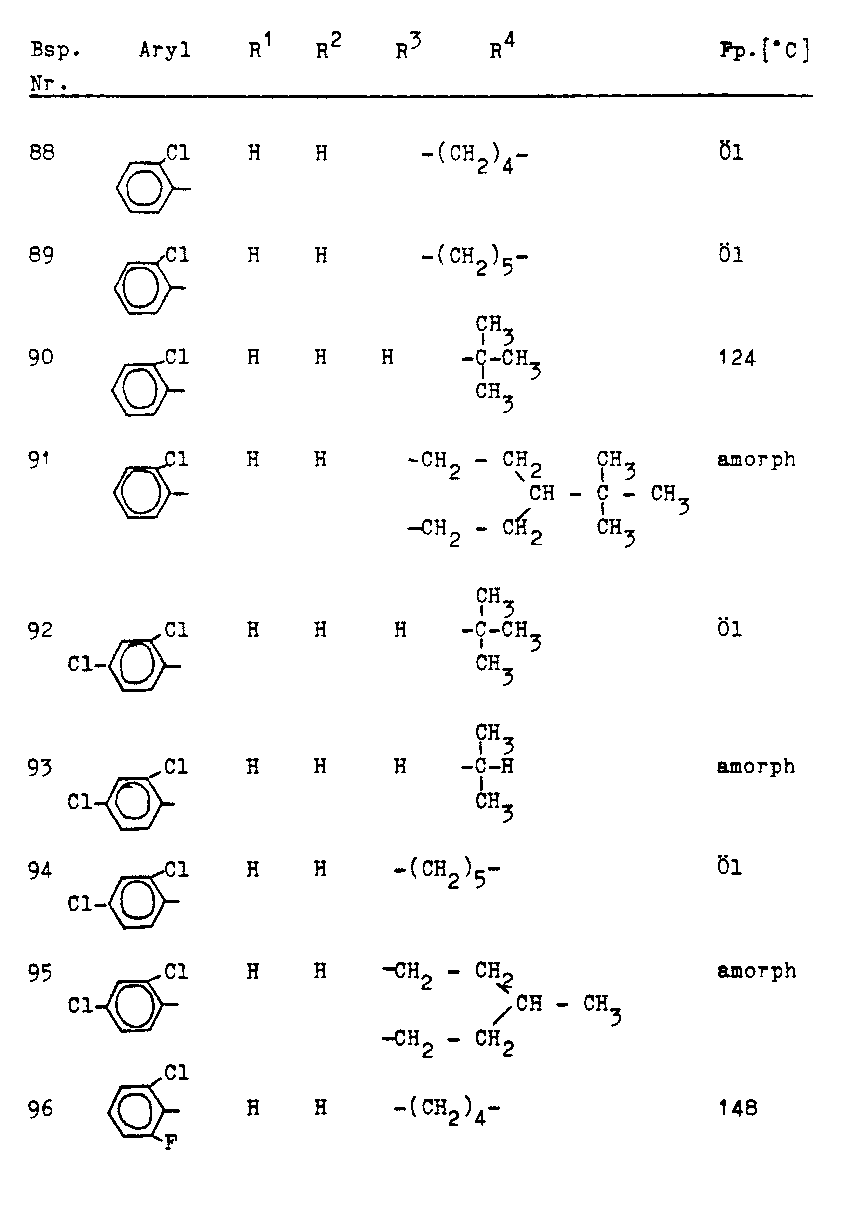

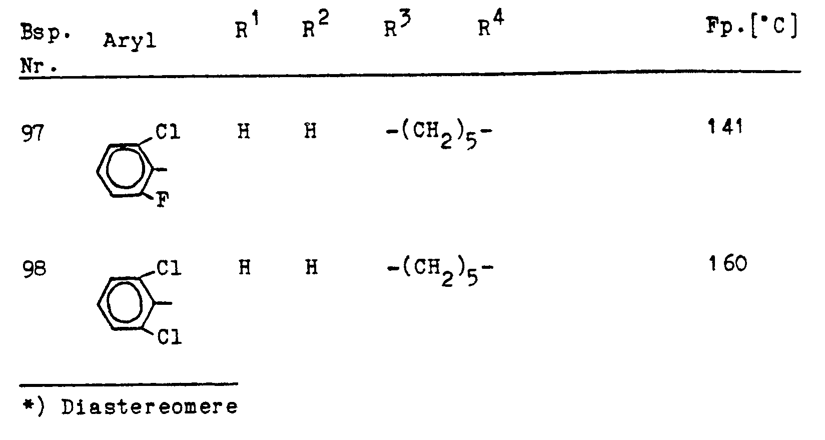

### Beispiel 99 (Herstellung des Ausgangsmaterials 1-(2,4-Dichlorbenzyl)-1,2,4-triazol)

34,5 g (0,5 Mol) Triazol wurden in 400 ml Dimethylformamid vorgelegt, und dann wurdenlangsam 15,0 g (0,5 Mol) 80 %iges Natriumhydrid zugegeben. Nachdem die Lösung klar war, tropfte man bei 35 - 45⁰ C 98 g (0,5 Mol) 2,4-Dichlorbenzylchlorid zu, rührte 4 Stunden bei 80 C, goß auf Eiswasser und extrahierte mit Methylenchlorid. Nach Einengen der Methylenchloridphase blieben 125 g Rückstand, der aus Hexan/Essigsäureethylester umkristallisiert wurde. Man erhielt 98 g des Triazolderivats vom Fp. 67° C.

### Beispiel 100 (Verbindung 100)

### 3-Fluorphenyl-(2-hydroxy-2-adamantyl)-1-imidazolyl-methan

Zu einer Lösung von 7,04 g (40 mmol) N-(3-Fluorbenzyl)-imidazol und 4,65 g (40 mmol) N,N,N',N'-Tetramethylethylendiamin (TMEDA) in 90 ml abs. Tetrahydrofuran (THF) wurden bei -70^{°} C 55 ml (85 mmol) einer 1,55-molaren Lösung von n-Butyllithium in Hexan getropft. Man rührte 20 Minuten bei -70 C nach und tropfte dann bei -70 ^{°} C in ca. 15 Minuten eine Lösung von 6,01 g (40 mmol) Adamantanon in 30 ml abs. Tetrahydrofuran (THF) zu. Anschließend rührte man 15 Minuten bei ca. -70 ^{°} C nach, ließ die Reaktionsmischung in ca. 2 Stunden auf Zimmertemperatur aufwärmen, versetzte unter Kühlung bei ca. 5 - 18° C mit 200 ml Wasser und extrahierte die Mischung mit CH₂Cl₂. Die vereinigten CH₂Cl₂-Extrakte wurden getrocknet, filtriert und im Vakuum eingedampft. Den Extraktrückstand löste man in Acetonitril, wonach kristallines 3-Fluorphenyl-(2-hydroxy-2-adamantyl)-1-imidazolyl-methan in reiner Form ausfiel. Man erhielt nach dem Absaugen und Auswaschen des kristallinen Produkts mit Acetonitril 8,70 g reines Produkt vom Fp. 191 ^{°} C.

^{C}₂₀^{H}₂₃^{FN}₂0 (326,42)

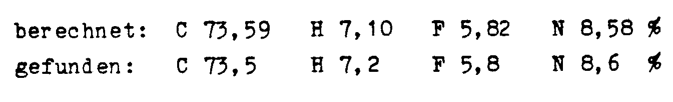

### Beispiel 101 (Verbindung 101)

### 3-Methoxyphenyl-(2-hydroxy-2-adamantyl)-1-imidazolyl-methan

Zu einer Lösung von 7,52 g (40 mmol) N-(3-Methoxybenzyl)-imidazol und 4,65 g (40 mmol) TMEDA in 90 ml abs. THF wurden bei -70° C 55 ml (85 mmol) einer 1,55-molaren Lösung von n-Butyllithium in Hexan getropft. Man rührte 20 Minuten bei -70 ^{°} C nach und tropfte dann bei -70^{°} C in ca. 10 Minuten eine Lösung von 6,01 g (40 mmol) Adamantanon in 30 ml abs. THF zu. Anschließend wurde wie im Beispiel 100 beschrieben weitergearbeitet. Den CH₂Cl₂-Extrakt-Rückstand (13 g) kristallisierte man aus Acetonitril. Man erhielt 7,5 g reines 3-Methoxyphenyl-(2-hydroxy-2-adamantyl)-1-imidazolyl-methan vom Fp. 182^{°} C.

^{C}₂₁^{H}₂₆^{N}₂⁰₂ (338,46)

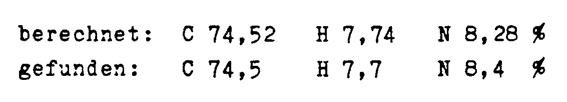

### Beispiel 102 (Verbindung 102)

### 1-(3-Chlorphenyl)-1-(2-hydroxy-2-adamantyl)-1-(1-imidazolyl)-ethan

Zu einer Lösung von 5,37 g (26 mmol) 1-(3-Chlorphenyl)-1-(1-imidazolyl)-ethan und 3,14 g (27 mmol) TMEDA in 60 ml abs. THF wurden bei -70^{°} C 36 ml (54 mmol) einer 1,5-molaren Lösung von n-Butyllithium in Hexan getropft. Nach 20 Minuten Rühren bei -70 C tropfte man bei -70 C eine Lösung von 3,91 g Adamantanon in 20 ml abs. THF zu. Man rührte 1 Stunde bei -70 C und 1,5 Stunden bei -70 C bis Zimmertemperatur nach, versetzte unter Kühlung bei ca. 10°C mit 100 ml Wasser und extrahierte die Mischung mit CH₂Cl₂. Die vereinigten CH₂Cl₂-Extrakte wurden nach dem Trocknen und Filtrieren im Vakuum eingedampft. Den Rückstand (10,1 g) chromatographierte man unter Elution mit CH₂Cl₂/C₂H₅OH-Mischungen mit steigendem C₂HsOH-Gehalt (bis max. 2 Vol.-%) an einer Kieselgel S/ CH₂Cl₂-Säule (0 2,0 cm, Höhe 41 cm). Nach Elution von 3,3 g unverändertem Adamantanon (≙ 84 % des Einsatzes) wurden bei der Elution mit CH₂CI₂ eine Fraktion (ca. 1,0 g) erhalten, in der die gesuchte Verbindung angereichert war. Diese Fraktion wurde erneut an einer Kieselgel S/Petrolether:CH₂CI₂/4:1-Säule (0 2,0 cm, H 34 cm), unter Elution mit Petrolether/CH₂CI₂-Mischungen mit steigendem CH₂CI₂-Gehalt und anschließend mit CH₂CI_{2,} chromatographiert. Nach Elution von Vorzonen (Gehalt 0,25 g) wurden DC-einheitliche Fraktionen vereinigt und im Vakuum eingedampft. Man erhielt 0,58 g (≙ 6,3 % Ausbeute) reines 1-(3-Chlorphenyl)-1-(2-hydroxy-2-adamantyl)-1-(1-imidazolyl)-ethan vom Fp. 121 C (aus CH₃CN).

C₂₁H₂₅C1N₂0 (356,90)

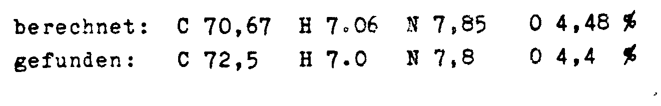

### Beispiel 103 (Verbindung 103)

### (3-Trifluormethyl-phenyl)-(2-hydroxy-2-adamantyl)-1-imidazolyl-methan

Zu einer Lösung von 4,80 g (20 mmol) 95 %igem N-(3-Trifluormethyl-benzyl)-imidazol in 40 ml abs. THF wurden bei -70 C 13,5 ml (20 mmol) einer 1,5-molaren Lösung von n-Butyllithium in Hexan getropft. Nach 45 Minuten bei -70 ^{°} C tropfte man bei -40 ^{°} C eine Lösung von 3,03 g Adamantanon in 16 ml abs. THF zu. Man rührte 1 Stunde bei ca. -40^{°} C, 45 Minuten bei -40^{°} C bis 0^{°} C, 20 Minuten bei 0^{°} C bis Zimmertemperatur und 2 Stunden bei ca. 20^{°} C nach, versetzte unter Kühlung bei ca. 10° C mit 300 ml Wasser und extrahierte die Mischung mit CH₂CI_{2.} Die vereinigten CH₂CI₂-Extrakte wurden nach dem Trocknen und Filtrieren im Vakuum eingedampft. Der Rückstand (7,3 g) kristallisierte beim Lösen in Acetonitril. Nach dem Absaugen und Auswaschen mit CH₃CN und Trocknen erhielt man 2,10 g kristallisiertes Reinprodukt vom Fp. 233 C. Der Mutterlaugenrückstand (5,2 g) wurde an einer Kieselgel S/CH₂CI₂-Säule (⌀ 2,1 cm, H 34 cm) unter Elution mit CH₂CI₂ und CH₂CI₂/C₂H₅OH-Mischungen (bis max. 2 Vol.% C₂H₅OH) chromatographiert. Dabei erhielt man aus vereinigten einheitlichen Fraktionen nach Kristallisation aus CH₃CN weitere 0,50 g (3-Trifluormethyl-phenyl)-(2-hydroxy-2-adamantyl)-1-imidazolyl-methan vom Fp. 232 C.

C₂₁H₂₃F₃N₂0 (376,43)

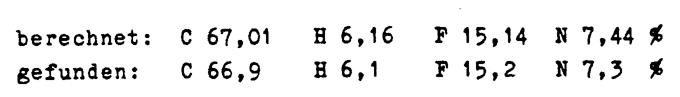

### Beispiel 104 (Verbindung 104)

### (3-Dimethylamino-phenyl)-(2-hydroxy-2-adamantyl)-1-imidazolyl-methan

Zu einer Lösung von 3,03 g (15 mmol) (3-Dimethylamino-benzyl)-imidazol und 1,75 g (15 mmol) TMEDA in 30 ml abs. THF wurden bei -70 C 20 ml (31 mmol) einer 1,55-molaren Lösung von n-Butyllithium in Hexan getropft. Nach 1 Stunde Rühren bei -70 C tropfte man bei -30° bis -15^{°} C eine Lösung von 2,26 g Adamantanon in 40 ml abs. THF zu. Man ließ in ca. 45 Minuten auf Zimmertemperatur anwärmen, rührte 2 Stunden bei Zimmertemperatur nach, versetzte unter Kühlung bei 10' C mit 400 ml Wasser und rührte 15 Minuten bei 0 - 5° C nach, wobei kristalliner Niederschlag gebildet wurde. Dieser wurde abgesaugt, mit Hexan und Diisopropylether nachgewaschen und im Vakuum getrocknet. Man erhielt 2,20 g (= 41,7 % Ausbeute) reines (3-Dimethylamino-phenyl)-(2-hydroxy-2-adamantyl)-1-imidazolyl-methan vom Fp. 185 C.

C₂₂H₂₉N₃0 (351,50)

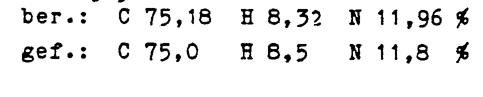

### Beispiel 105 (Verbindung 105)

### 1-(1-Adamantyl)-2-(3-chlorphenyl)-2-(1-imidazolyl)-ethanol

Zu einer Lösung von 3,85 g (20 mMol) N-(3-Chlorbenzyl)-imidazol in 46 ml abs. THF wurden bei -70^{°} C 15,5 ml (23 mmol) einer 1,5 molaren Lösung von n-Butyllithium in Hexan getropft. Nach 30 Minuten Rühren bei -70^{°} C tropfte man bei -70^{°} C eine Lösung von 3,29 g (20 mmol) 1-Formyladamantan in 20 ml abs. THF zu. Man rührte 1 Stunde bei ca. -70 C und 2 Stunden bei -70^{°} C bis Zimmertemperatur nach, versetzte unter Kühlung bei ca 10°C mit 120 ml Wasser und rührte die entstandene Suspension 1,5 Stunden bei 5 - 8° C. Danach saugte man die kristalline Substanz (Rohprodukt) ab, wusch mit Wasser und Hexan nach und filtrierte das kristalline Produkt gelöst in CH₂CI₂ über eine Kieselgel/CH₂CI₂-Säule (⌀ 2,0 cm, H 20 cm). Die filtrierte Lösung wurde im Vakuum eingedampft und der Rückstand (2,36 g) aus Aceton kristallisiert. Mal. erhielt 2,15 g (= 30,1 % Ausbeute) reines 1-(1-Adamantyl)-2-(3-chlorphenyl)-2-(1-imidazolyl)-ethanol vom Fp. 115^{°}C.

C₂₁H₂₅ ClN ₂0 (356,90)

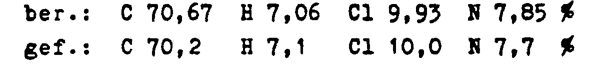

### Beispiel 106 (Verbindung 106)

### (2-Hydroxy-2-adamantyl)-1-imidazolyl-(3-tolyl)-methan

Zu einer Lösung von 5,17 g (30 mmol) N-(3-Methyl-benzyl)-imidazol und 3,49 g (30 mmol) TMEDA in 60 ml. abs. THF wurden bei -70 C 39 ml (60,5 mmol) 1,55 molare n-Butyllithium/Hexan-Lösung getropft. Nach 30 Minuten Rühren bei -70 ^{°} C tropfte man bei ca. -70 ^{°} C eine Lösung von 4' 51 g Adamantanon in 35 ml abs. THF zu, rührte 20 Minuten bei -7 ^{°} C nach, Ließ auf Zimmertemperatur aufwärmen, versetzte unter Kühlung mit 300 ml Wasser und rührte die Mischung dann 15 Minuten bei ca 5 ^{°} C. Danach wurde die abgeschiedene kristalline Substanz abgesaugt, mit Wasser und Hexan nachgewaschen, getrocknet und mit ca. 25 ml CH₃CN 10 Minuten aufgekocht. Danach wurde nach Abkühlung der Feststoff wiederum abgesaugt und getrocknet. Man erhielt 3,87 g (= 40 % Ausbeute) reines (2-Hydroxy-2-adamantyl)-1-imidazolyl-(3-tolyl)-methan vom Fp. 186 ^{°} C.

c₂₁ H₂₆ N₂0 (32₂,₄6)

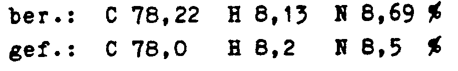

### Beispiel 107 (Verbindungen 107a, 107b, 107c und 107d)

### 1-(3-Chlorphenyl)-1-(1-imidazolyl)-nona-3,7-dien-2-ol

Zu einer Lösung von 7,70 g (40 mmol) N-(3-Chlorbenzyl)-imidazol in 100 ml abs. THF wurden bei -70 ^{°} C in 15 Minuten 26 ml (40 mmol) 1,55 molare n-Butyllithium/Hexan-Lösung getropft. Nach 30 Minuten Rühren bei -70 C tropfte man bei ca. -70 C eine Lösung von 6,10 g (40 mmol) Citral (cis/trans-Mischung) in 50 ml abs. THF zu, rührte 45 Minuten bei ca. -70° C nach, ließ in ca. 2 Stunden auf Zimmertemperatur aufwärmen, versetzte unter Kühlung mit 300 ml Wasser und extrahierte die Mischung mit CH₂CI_{2.} Die vereinigten CH₂CI₂-Extrakte wurden getrocknet, filtriert und im Vakuum eingedampft. Der Extraktrückstand (13,2 g) wurde an einer Kieselgel S/n-Hexan-Säule (0 2,5 cm, H 56 cm) unter Elution mit Hexan/Ethanol-Mischungen, mit steigendem Ethanol-Gehalt (bis max. 20 % Ethanol) chromatographiert. Der Verlauf der Elution wurde mittels DC überprüft. Daraus ergab sich, daß die gesuchte Verbindung in 4 Stereoisomeren in untereinander ähnlichen Mengen vorlag. DC-einheitliche Fraktionen wurden vereinigt und Im Vakuum zur Gewichtskonstanz eingedampft. Auf diese Weise wurden 1,20 g nahezu reines Stereoisomeres (a), mit größtem R_{F}-Wert, erhalten. Daneben fielen 8,2 g Mischung aus den Stereoisomeren (b), (c) > (a), (d) an. Zuletzt wurden 2,6 g eluiert, worin laut DC das Stereoisomere (d) (kleinster R_{F}-Wert) stark angereichert war. Dieses Produkt wurde einer zweiten Chromatographie nach gleicher Arbeitsweise an einer Kieselgel S/Hexan-Säule (0 2 cm, H 22 cm) unterworfen. Dabei wurden 1,2 g DC-nahezu reines Stereoisomeres (d) erhalten.

C ₂₀ H ₂₅ C1N ₂0 (344,89)

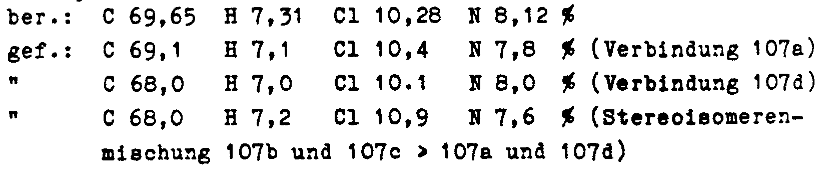

### Beispiel 108 (Verbindung 108)

### 3-Chlorphenyl-1- imidazolyl-(2-hydroxy-1-methoxymethyl-5-norbornen-2-yl)-methan

3,85 g (20 mmol) N-(3-Chlorbenzyl)-imidazol wurden in 45 ml. abs. THF gelöst und bei -70^{°} C mit 13,4 ml (20 mmol) 1,5 m n-Butyllithium/Hexan-Lösung metalliert. Nach 30 Minuten gab man ebenfalls bei -70 C eine Lösung von 3,05 g (20 mmol) 1-Methoxymethyl-5-norbornen-2-on in 20 ml abs. THF zu, rührte 20 Minuten bei -70^{°} C nach und Ließ innerhalb von 2 Stunden auf Zimmertemperatur anwärmen. Anschließend versetzte man mit Wasser und extrahierte mit Ether. Der Ether-Extrakt-Rückstand (7,6 g) wurde aus Ethylacetat/Diisopropylether kristallisiert. Nach Absaugen und Trocknen der kristallinen Substanz erhielt man 3,75 g (= 54,5 % Ausbeute) reines 3-Chlorphenyl-1-imidazolyl-(2-hydroxy-1-methoxymethyl-5-norbornen-2-yl)-methan vom Fp. 161 C.

C₁₉H₂₁C1N₂0₂ (344,85)

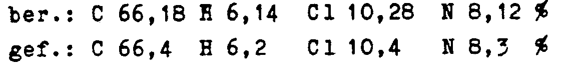

### Beispiel 109 (Verbindung 109)

### 1-(1-imidazolyl)-1 -(3-tolyl)-3,3-dimethyl-butanol-(2)

Zu einer Lösung von 5,17 g (30 mmol) N-(3-Methyl-benzyl)-imidazol und 3,49 g (30 mmol) TMEDA in 60 ml abs. THF wurden bei -70^{°}C 39 ml (60 mmol) 1,55 molare n-Butyllithium/Hexan-Lösung getropft. Nach 30 Minuten Rühren bei ca. -70 ^{°}C tropfte man bei ca. -70 ^{°} C in 20 Minuten eine Lösung von 3,80 g (33 mMol) 75 %igem Pivalinaldehyd in 30 ml abs. THF zu, rührte 20 Minuten bei ca. -70^{°} C nach und Ließ innerhalb von 2 Stunden die Mischung auf Zimmertemperatur anwärmen. Anschließend wurde unter Kühlung mit Wasser versetzt und die entstandene Mischung mit Ether extrahiert. Der Etherextrakt-Rückstand (9,3 g) wurde aus einer Diisopropylether/Hexan-Mischung kristallisiert. Dabei erhielt man 2,2 g Kristallisat, das zu ca. 90 % aus einem Nebenprodukt dem 1-(3-Methyl-benzyl)-2-(1-hydroxy-2,2-dimethyl- prop-1-yl)-imidazol bestand. Der Mutterlaugenrückstand wurde aus einer Diisopropylether/Acetonitril-Lösung umkristallisiert und das dabei isolierte kristalline Produkt anschließend zweimal aus Acetonitril umkristallisiert. Man erhielt auf diese Weise 0,64 g (= 8,3 % d.Th.) reines 1-(1-lmidazolyl)-1-(3-tolyl)-3,3-dimethyl- butanol-(2) vom Fp. 126" C.

C₁₆H₂₂N₂0 (258,37)

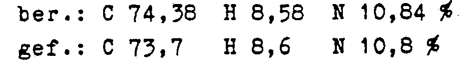

### Beispiel 110 (Verbindung 110)

### 2-(4-Benzyloxy-phenyl)-2-(1-imidazolyl)-1,1-diphenyl-ethanol

Zu einer Lösung von 6,87 g(26 mmol) N-(4-Benzyloxy-benzyl)-imidazol und 3,03 g (26 mmol) TMEDA in100 ml absl THF tropfte man bei ca. -70 C 34 ml (53 mmol) 1,55 molare n-Butyllithium/Hexan-Lösung, rührte 20 Minuten bei -70^{°} C nach, tropfte dann bei -70^{°} C eine Lösung von 4,74 g Benzophenon in 40 ml abs. THF zu, rührte 30 min ca. -70 C und 1,5 Stunden von -70 C bis Zimmertemperatur nach und versetzte unter Kühlung mit 350 ml Wasser. Die Mischung wurde mit CH₂CI₂ extrahiert. Der CH₂CI₂-Extrakt- Rückstand (12 g) wurde wie im Beispiel 102 beschrieben an einer Kieselgel S/CH₂CI₂-Säule (⌀ 2,1 cm, H 55 cm) chromatographiert. Fraktionen, in denen laut DC die gesuchte Substanz angereichert vorlag, wurden vereinigt und aus Ethylacetat/Diisopropylether kristallisiert. Man erhielt auf diese Weise 1,09 g (= 16,4 % Ausbeute) reines 2-(4-Benzyloxy-phenyl)-2-(1-imidazolyl)-1,1-diphenyl-ethanol vom Fp. 204° C.

C₃₀H₂₆N₂0₂ (446,55)

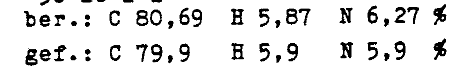

### Beispiel 111 (Verbindung 111)

### 2-(3-Chlorphenyl)-2-(1-imidazolyl)-1-(3-methyl-adamant-1-ylmethyl)-ethanol

Zu einer analog Beispiel 105 hergestellten Lösung von 20 mmol Lithium-N-(3-chlorbenzyl)-imidazol in THF/Hexan gab man bei ca. -60° C eine Lösung von 4.05 g (20 mmol) 2-(3-Methyl-1-adamantyl)-acetaldehyd (95 %ig) in 40 ml abs. THF und rührte 45 Minuten bei ca. -60^{°} C und .1,75 Stunden von -60^{°} C bis Zimmertemperatur nach. Nachdem mit ca. 200 ml Wasser versetzt worden war, wurde die Mischung mit Ether extrahiert. Der Etherextrakt-Rückstand (8,8 g) wurde aus Diisopropylether kristallisiert und das erhaltene Produkt anschließend aus Acetonitril umkristallisiert. Nach Isolierung erhielt man 1,90 g (= 24,7 % d.Th.) 2-(3-Chlorphenyl)-2-(1-imidazolyl)-1-(3-methyl-adamant-1-ylmethyl)ethanol vom Fp. 126^{°} C als DCreines Diastereomeres. In der Mutterlauge waren laut DC noch Anteile des gleichen Diastereomeren und als Hauptanteil das andere Diastereomere enthalten.

^{C}₂₃^{H}₂₉^{C}1N₂0 (384,96)

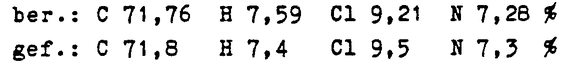

### Beispiel 112 (Verbindung 112)

### 2-(3-Chlorphenyl)-2-(1-imidazolyl)-1-cyclopropyl-1-phenyl-ethanol

Zu einer analog Beispiel 105 hergestellten Lösung von 25 mmol Lithium-N-(3-chlorbenzyl)-imidazol in THF/Hexan gab man bei ca. -70 C eine Lösung von 3,77 g Cyclopropyl-phenyl-keton (97 %ig) in 37 ml abs. THF und rührte 20 Minuten bei ca. -70^{°}C und 1,75 Stunden von -70^{°} C bis Zimmertemperatur nach. Nachdem mit ca. 200 ml Wasser versetzt worden war, wurde die Mischung mit Ether extrahiert. Den Etherextrakt-Rückstand (6,1 g) chromatographierte man unter Elution mit CH₂CI₂ und CH₂CI₂/C₂H₅OH-Mischungen mit steigendem C₂H₅OH-Gehalt (bis max. 1 Vol.%) an einer Kieselgel S/CH₂CI₂-Säule (0 2cm, Höhe 32 cm). Laut DC ähnlich zusammengesetzte Fraktionen wurden vereinigt. Das zuerst eluierte Diastereomere (A) (2,56 g) war dabei ungenügend angereichert worden und wurde deshalb nochmals chromatographiert (s.u.). Anschließend wurden Gemische der beiden Diastereomeren (A) und (B) eluiert, woraus das Diastereomere (B) (mit kleinerem R_{F} in DC) aus Diisopropylether kristallisierte. Nach Isolierung und Trocknung erhielt man 0,83 g (= 9,8 % d.Th.) 2-(2-Chlorphenyl)-2-(1-imidazolyl)-1-cyclopropyl-1-phenyl-ethanol in Form des reinen Diastereomeren (B) (DC, kleiner R_{F}) vom Fp. 146 ^{°} C. In der Mutterlauge waren 0,90 g Gemisch der Diastereomeren (A) und (B) enthalten. Das chromatographisch angereicherte Diastereomere (A) (DC, großer R_{F}) (2,56 g) wurde nochmals an einer Kieselgel S/ CH₂CI₂-Säule (0 2 cm, Höhe 30 cm) wie vorstehend beschrieben chromatographiert. Dabei sind 2,04 g stark angereichertes Diastereomeres (A) erhalten worden, das aus Ether/Diisopropylether-Lösung kristallin erhalten wurde. Nach Isolierung und Trocknung erhielt man 1,60 g (= 18,9 % d.Th.) 2-(3-Chlorphenyl)-2-(1-imidazolyl)-1-cyclopropyl-1-phenyl-ethanol in Form des reinen Diastereomeren (A) (DC, großer R_{F}) vom Fp. 140°C. In der Mutterlauge waren 0,70 g Gemisch beider Diastereomeren ((A)>(B)) enthalten.

C₂₀H₁₉C1N₂0 (338,85)

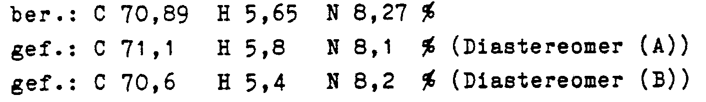

Analog zu den Beispielen 1 bis 10 und 100 bis 112 wurden folgende Verbindungen der Formel I mit Z= CH und R¹ und R²= H erhalten:

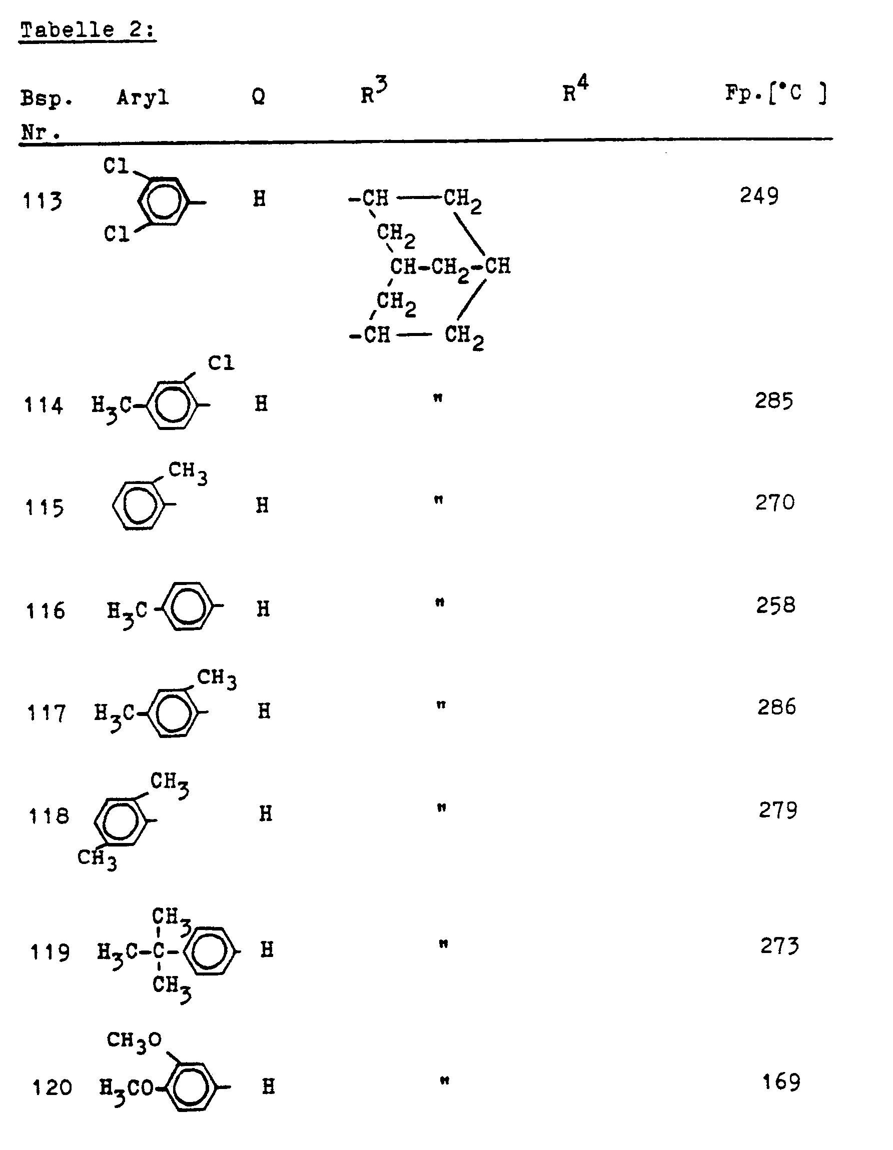

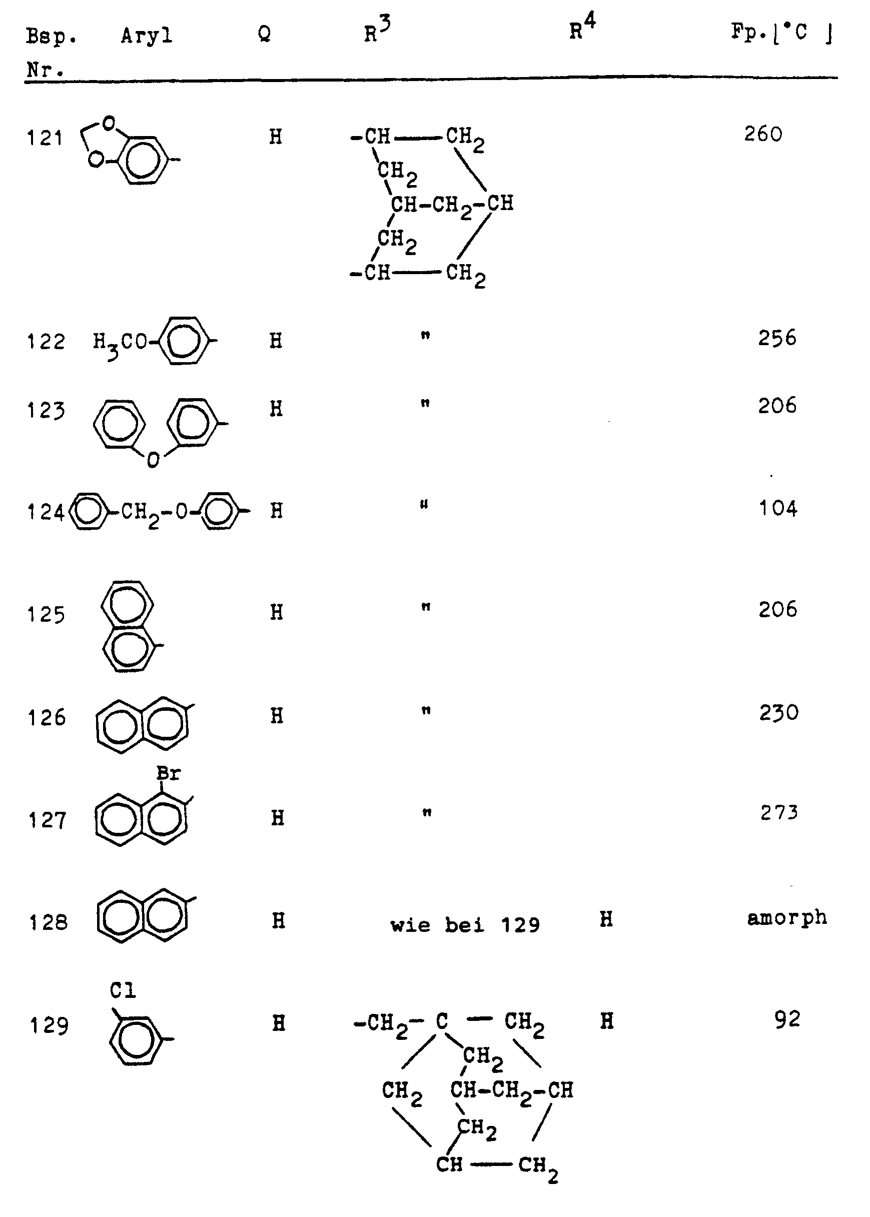

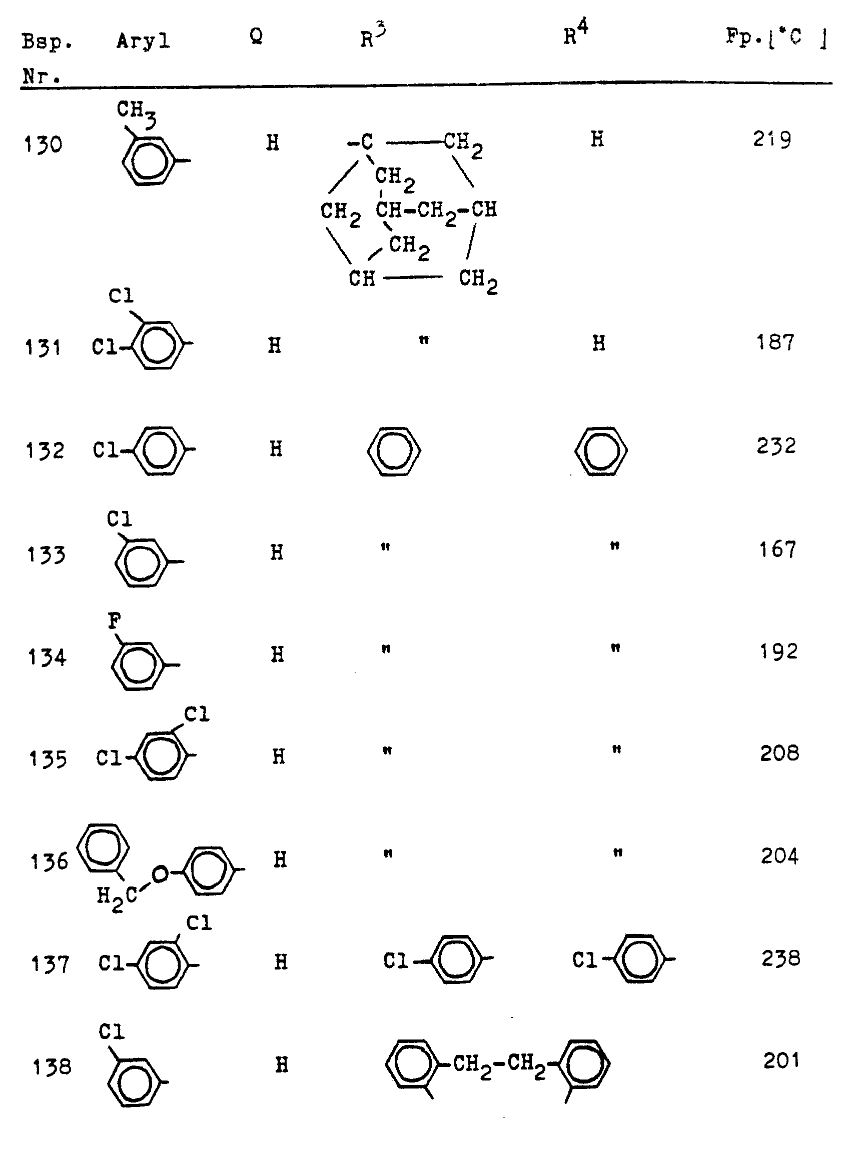

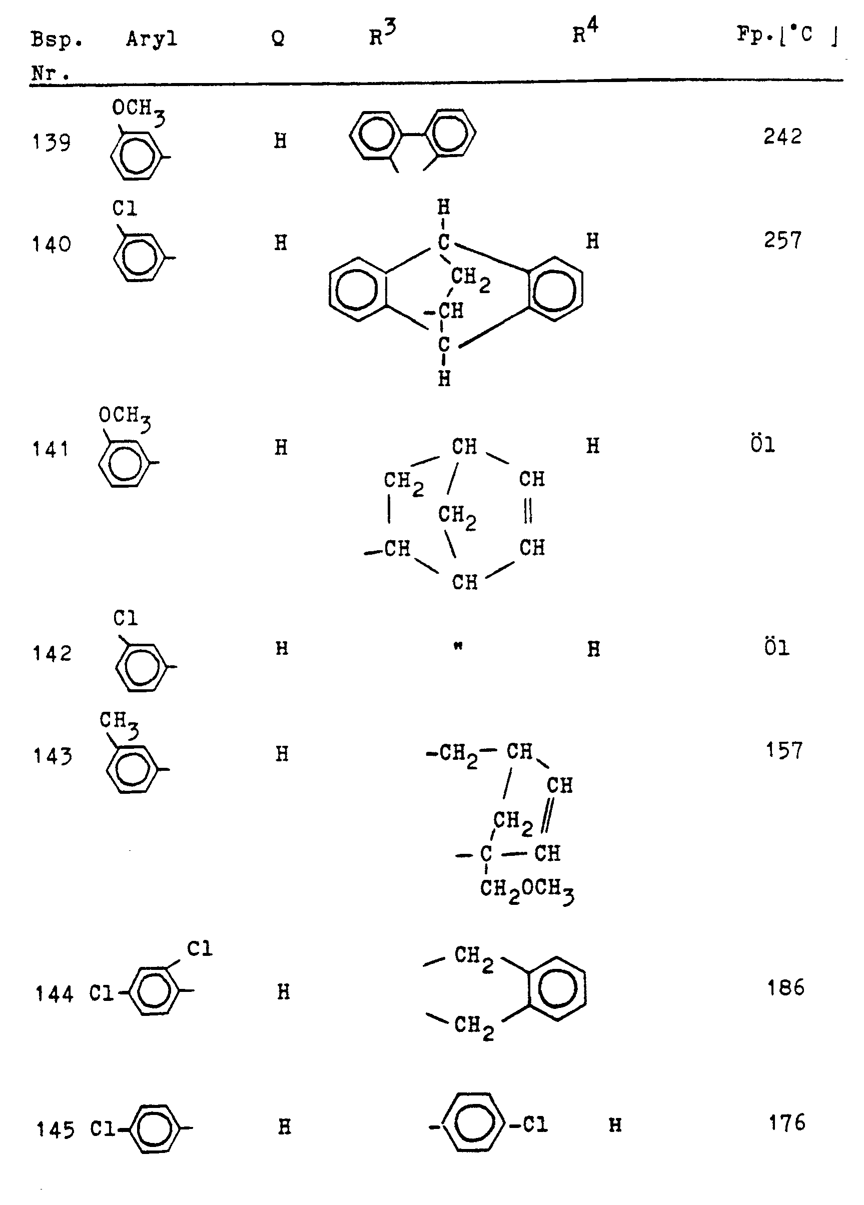

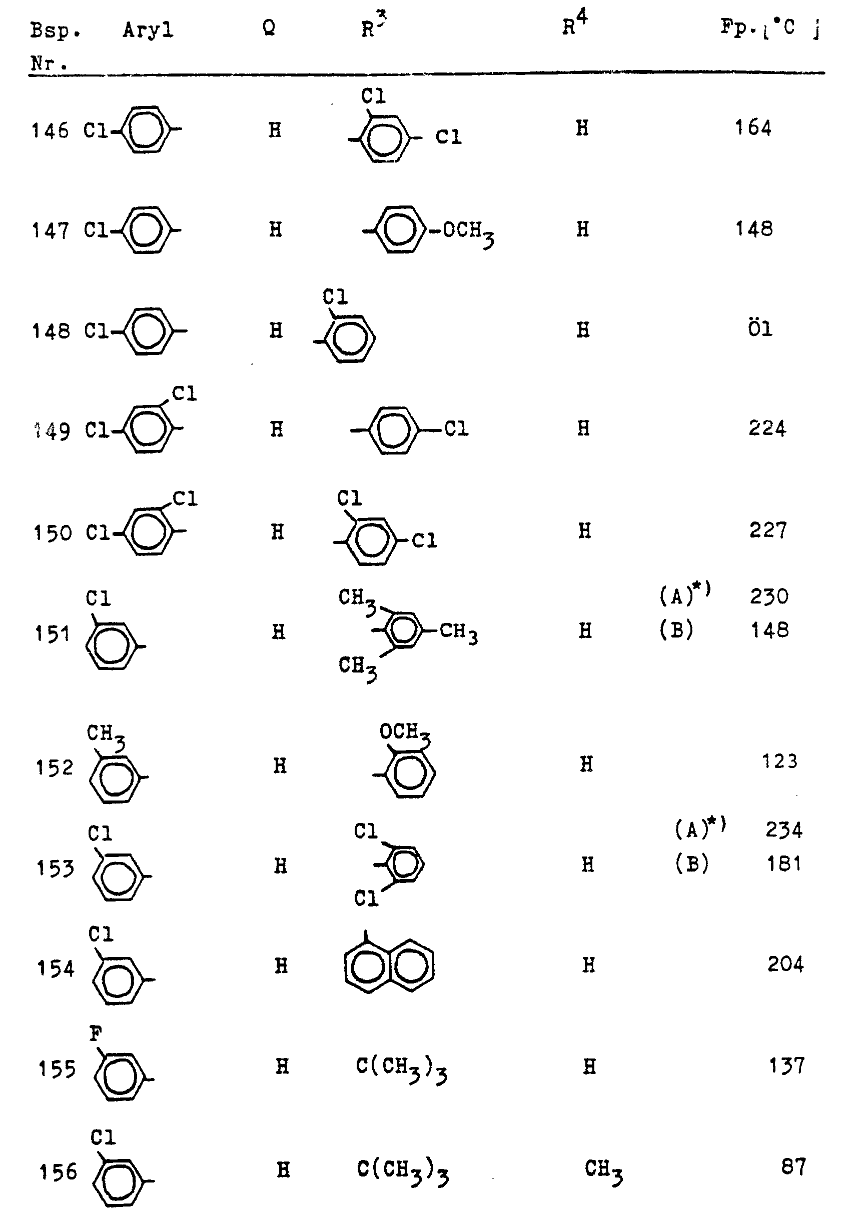

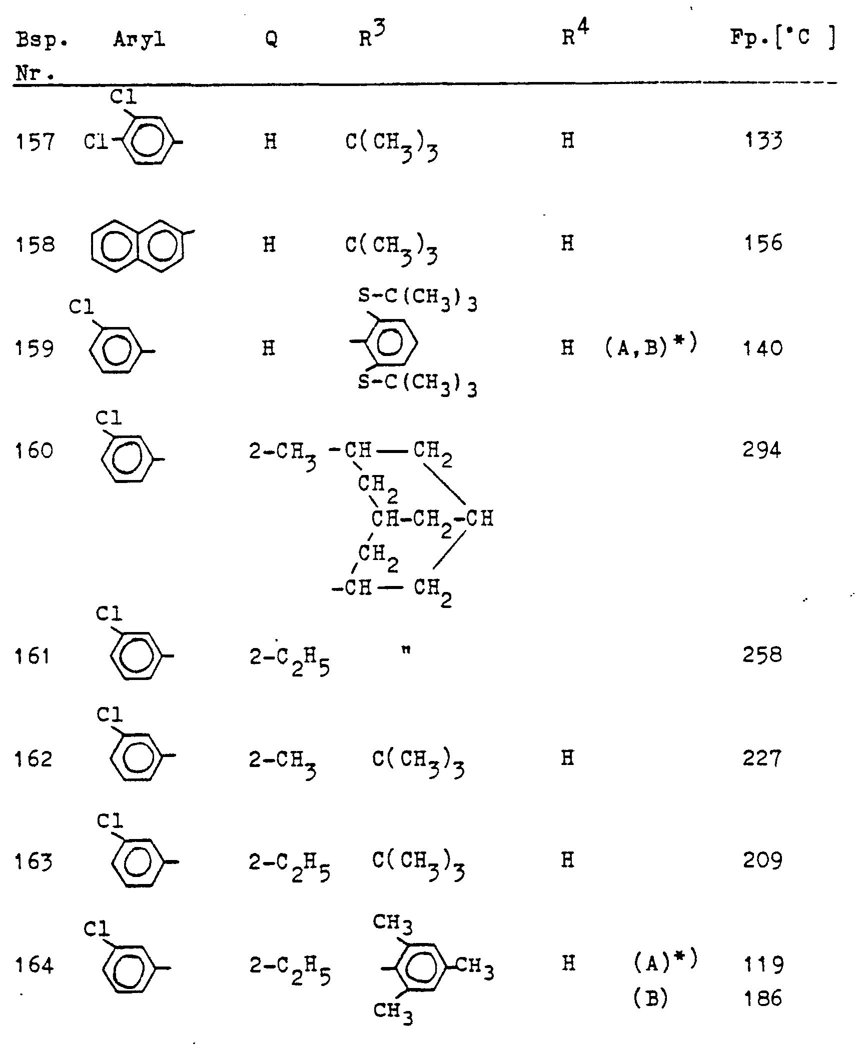

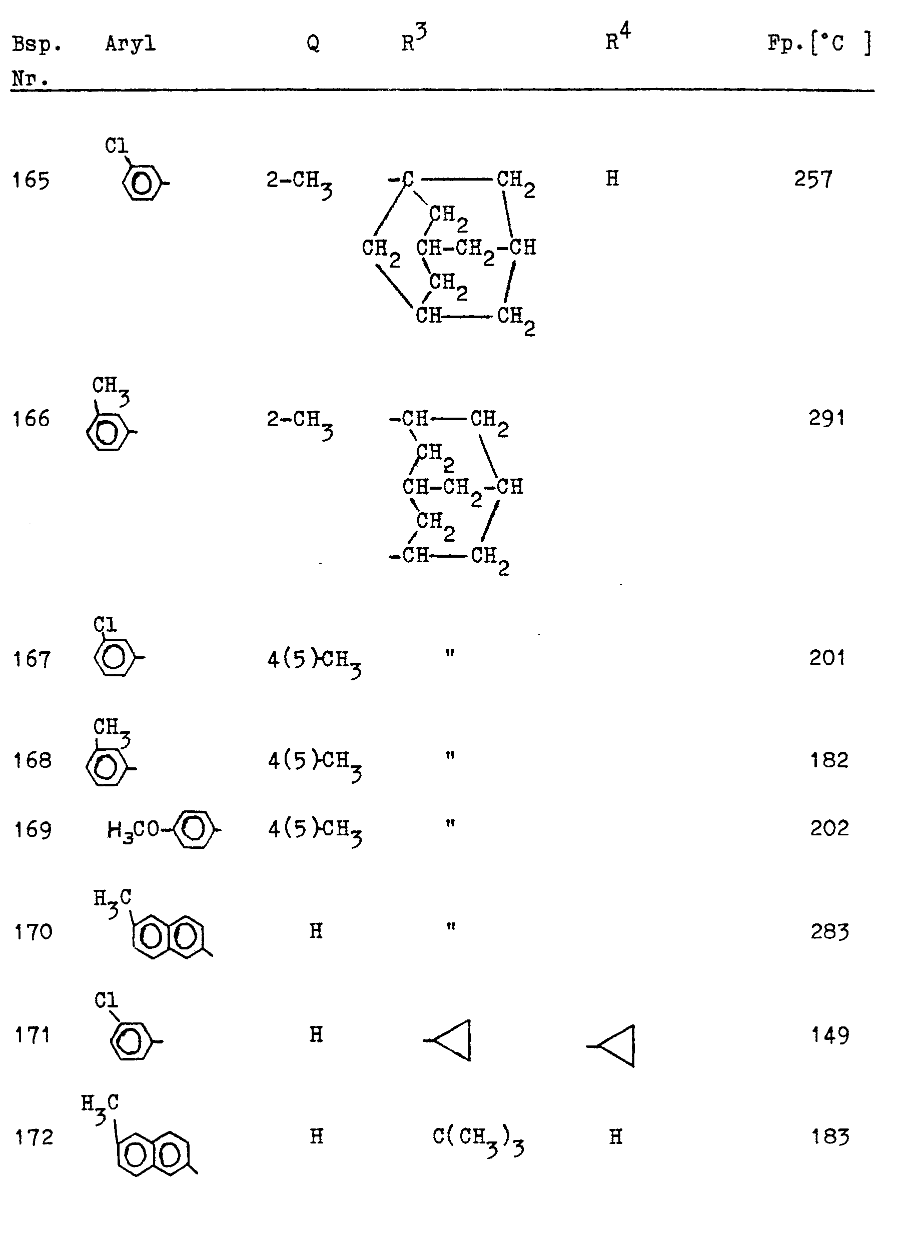

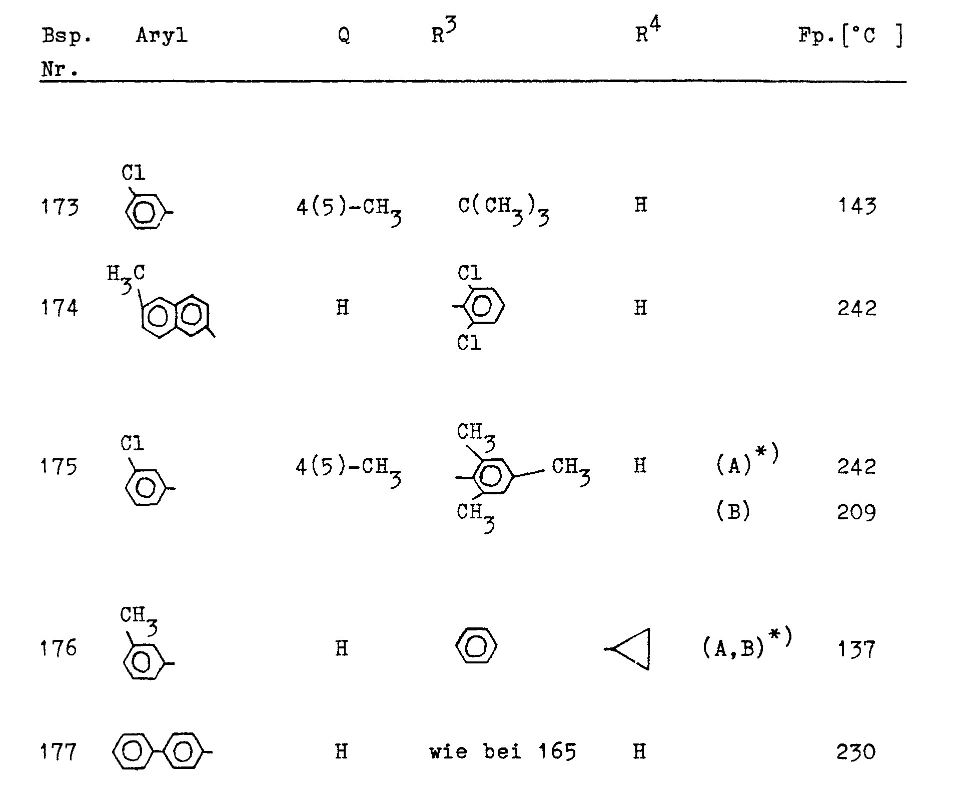

Analog zu den Beispielen 9 und 10 wurden folgende Verbindungen der Formel I mit Z = H und R¹ und R² = H erhalten:

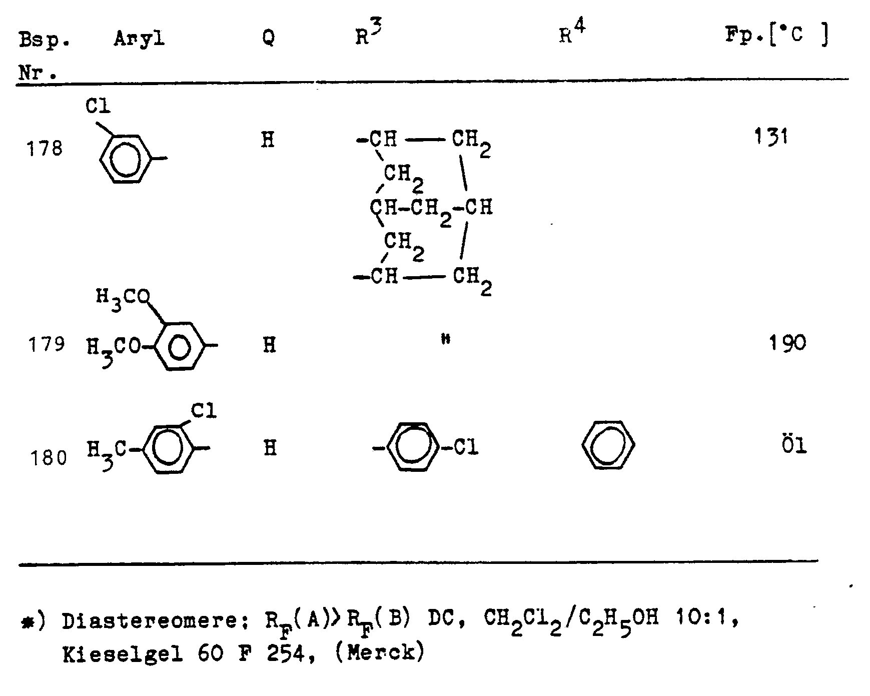

### Beispiel 181

### (Racemattrennung von (3-Chlorphenyl)-(2-hydroxy-2-adamantyl)-(1-imidazolyl)-methan, Verbindung gemäß Beispiel 25)

a) 43,0 g (125,4 mmol) (3-Chlorphenyl)-(2-hydroxy-2-adamantyl)-(1-imidazolyl)-methan und 18,83 g (125,4 mmol) D(-)-Weinsäure wurden in einer Mischung von 67 ml Methanol und 440 ml Acetonitril bei Siedetemperatur gelöst. Beim langsamen Abkühlen der Lösung auf Zimmertemperatur erfolgte Kristallisation. Nach 2 Tagen wurde das Kristallisat abgesaugt und mit Acetonitril und Ether gewaschen und 3 Stunden bei 98^{°} C/3-6 mbar getrocknet. Man erhielt 23,50 g (3-Chlorphenyl)-(2-hydroxy-2-adamantyl)-(1-imidazolyl)-methan-hydrogentartrat
   ([α]_{D}22 : (+) 4,26° (c= 1,0, CH₃OH)). Dieses Produkt löste man in einer siedenden Mischung Von 33 ml Methanol und 193 ml Acetonitril und ließ bei Zimmertemperatur auskristallisieren. Nach Isolierung wie vorstehend beschrieben wurden 18,00 g Produkt([α]²²_{D} : (+) 5,30° (c= 1,0, CH₃OH)) erhalten. Diese 18,00 g wurden in analoger Weise aus 26 ml Methanol und 151 ml Acetonitril umkristallisiert. Man erhielt 14,2 g Produkt ([α]_{D}²²:(+) 5,53^{°} (c= 1,0, CH₃OH)). Eine weitere Umkristallisation aus 20 ml Methanol und 116 ml Acetonitril lieferte 11,7 g Produkt ([α]²²_{D} : (+) ₅,90⁰ (c= 1,0, CH₃OH)). Nochmalige Umkristallisation dieser Substanz aus 16 ml Methanol und 95 ml Acetonitril ergab 9,60 g (3-Chlorphenyl)-(2-hydroxy-2-adamantyl)- (1-imidazolyl)-methan-hydrogentartrat ([α]_{D}²² : (+) 6,18^{°} (c= 1.0, CH₃OH)) als reines diastereomeres Salz, Fp. 112-113^{°} C (Zers.).

   Die Mutterlaugen Vom 3., 4. und 5. Umkristallisieren wurden zusammengefaßt, im Vakuum eingedampft und der verbliebene Rückstand (8,20 g) wurde in analoger Weise wie vorstehend beschrieben aus 11,5 ml Methanol und 66,5 ml Acetonitril umkristallisiert. Man erhielt 5,4 g Produkt ([α]_{D}²² : (+) 5,81 (c= 1,0, CHaOH)), das in analoger Weise noch zweimal aus entsprechenden Methanol/Acetonitril-Mischungen umkristallisiert wurde. Danach fielen an dieser Stelle weitere 3,60 g reines diastereomeres Hydrogentartrat ([α]D²² : (+) 6,20° (c= 1,0, CH₃OH)) an. Insgesamt erhielt man auf diese Weise 13,20 (= 42,7 % d.Th.) reines (+)-(3-Chlorphenyl)-(2-hydroxy-2-adamantyl)-(1-imidazolyl)-methanhydrogentartrat (ausgehend von D(-)-Weinsäure).
   C₂₄H₂₉C1N₂0₇ (492,97) 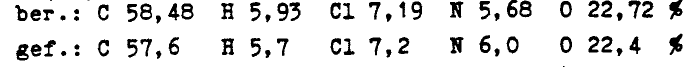
b) Nachdem gemäß a) ca. 43 % des (+)-Enantiomeren in Form des reinen diastereomeren (+)-Hydrogentartrats abgetrennt worden waren, wurden die Mutterlaugen zusammengefaßt im Vakuum eingedampft. Den verbliebenen Rückstand (47,5 g) löste man in einer Mischung von 450 ml Wasser und 300 ml CH₂CI_{2,} versetzte bis pH 9,5 mit NaOH, durchmischte und trennte anschließend die Phasen und dampfte nach dem Trocknen und Filtrieren die CH₂CI₂-Phase im Vakuum ein. Den Rückstand kochte man unter Rühren kurz mit ca. 230 ml Hexan auf, ließ erkalten und saugte die kristalline Substanz, in der das (-)Enantiomere im Überschuß vorlag, ab. Man erhielt 30 g (3-Chlorphenyl-(2-Hydroxy-2-adamantyl)-(1-imidazolyl)-methan ([α]_{D}²² : (-) 8,6 (c= 1.05, CH₃0H))(ca. 70 % d.Th.).
   Diese 30 g Produkt (87,5 mmol) und 13,13 g (87,5 mmol) L(+)-Weinsäure wurden in einer siedenden Mischung von 44 ml Methanol und 308 ml Acetonitril gelöst und unter langsamem Abkühlen auf Zimmertemperatur auskristallisieren gelassen. Die weitere Bearbeitung erfolgte in analoger Weise wie unter a) beschrieben. Man kristallisierte das isolierte Kristallisat anschließend viermal aus entsprechenden Methanol/Acetonitril-Mischungen um, wonach 14,2 g (≙ 45,9 % d.Th.) quasi reines (-)-(3-Chlorphenyl)-(2-hydroxy-2-adamantyl)-(1-imidazolyl)-methan-hydrogentartrat (ausgehend vonL(+)-Weinsäure) ([α]²²D : (-) 6,08 ^{°} (c = 1,0, CH3OH)), Fp. 112-113° C (Zers.), erhalten wurden.
   C₂₄ H₂₉C1N₂0₇ (492,97) 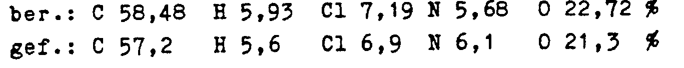
c) Eine Mischung aus 9,86 g (20 mmol) (+)-(3-Chlorphenyl)-(2-hydroxy-2-adamantyl)-(1-imidazolyl)-methan-hydrogentartrat, 400 ml CH₂CI_{2,} 22 ml 2n Natronlauge und 100 ml Wasser wurde solang bei Zimmertemperatur geschüttelt bis zwei klare Phasen entstanden waren. Nach Trennung der Phasen wurde die wäßrige zweimal mit CH₂CI₂ extrahiert. Die vereinigten CH₂CI₂-Phasen wurden getrocknet, filtriert und im Vakuum eingedampft. Der kristalline Rückstand (6,86 g) wurde aus einer Mischung von 86 ml Acetonitril und 16 ml Methanol umkristallisiert. Man erhielt noch Isolierung und Trocknung der kristallinen Substanz 5,34 g (≙ 78 % d.Th.) reines (+)-(3-Chlorphenyl)-(2-hydroxy-2-adamantyl)-(1-imidazolyl)-methan ([α] ²²D : (+)19,61 ^{°} (c= 1.05, CH₃0H)), Fp. 194,5^{°} C. Aus dem MutterlaugenRückstand wurde in analoger Weise eine weitere Menge (1,26 g, ≙ 18,4 % d.Th.) dieser Substanz erhalten ([α]²²D : (+) 19,09 ^{°} (c = 1,0, CH₃OH)), Fp. 193,5-194,5⁰ C.
   C₂₀H₂₃ClN₂0 (342,88)

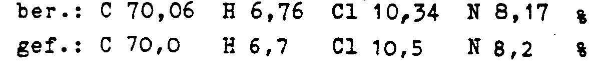

### Pharmakologische Prüfung

### 1. Prüfung auf Beeinflussung der Tetrabenazin-Ptosis (TBZ) Methode

Versuch an Mäusen (NMRI) männlich, Gewicht 19-21 g bzw. an Ratten (Wistar) männlich, ca. 120 g Körpergewicht. Präparat per os in 1 %iger Tylose homogenisiert, 1 Stunde vor Tetrabenazin, 40 mg/kg s.c. (N = 6/dosi). Während des Versuchs sitzen 6 Tiere zusammen in einem Gefäß. Zur Prüfung auf Oberlid-Ptosis werden die Tiere einzeln auf die Tischplatte gesetzt. Das Ausmaß der Ptosis (0 - 4 Punkte) wird beurteilt und als Prozentsatz des maximal erreichbaren Scores angegeben. Die Bewertung erfolgt 30 Minuten nach Tetrabenazingabe. Als Vergleich dienen Kontrollen, die nur Tetrabenazin erhalten haben. Als ED₅₀ wird die Dosis ermittelt, die den Grad der Ptosis nur mit Vehikel behandelter Kontrolltiere um 50% vermindert (lineare Regressionsanalyse).

### 2. Potenzierung der Yohimbin-Toxizität an Mäusen Methode

Die Toxizität von Yohimbin, einem Blocker präsynaptischer alpha-Rezeptoren, wird durch Antidepressiva potenziert (Literatur: R.M. Quinton, Brit. J. Pharmac. 21, 55-56 (1963)). Männliche Mäuse, NMRI-Stamm, 20-22 g, wurden in Versuchsgruppen zu n = 10 zugeordnet. Das Prüfpräparat wurde in 1 %igem wäßrigem Tylose^{(R)}-Schleim suspendiert und im Volumen 10 mi/kg oral gegeben. Kontrollen erhielten nur das Vehikel.

Nach einer Vorbehandlungszeit von 1 Stunde erhielten alle Tiere eine allein nicht toxische Dosis von Yohimbin-Hydrochlorid, 20 mg/kg subkutan. Die verstorbenen Tiere wurden 18 Stunden nach dieser Behandlung ermittelt. (Berechnung der LD₅₀ mit Probit-Analyse).

### 3. Prüfung der Auslösung von Stereotypien an Ratten

Die Versuche wurden an Wistar-Ratten, männlich, 130-140 g durchgeführt. Die Stereotypien wurden in den ersten beiden Stunden nach Substanzgabe in 15-Minuten-Intervallen beurteilt, danach bis zur 6. Stunde in halbstündigen Intervallen. Die folgende Skala wurde benutzt.

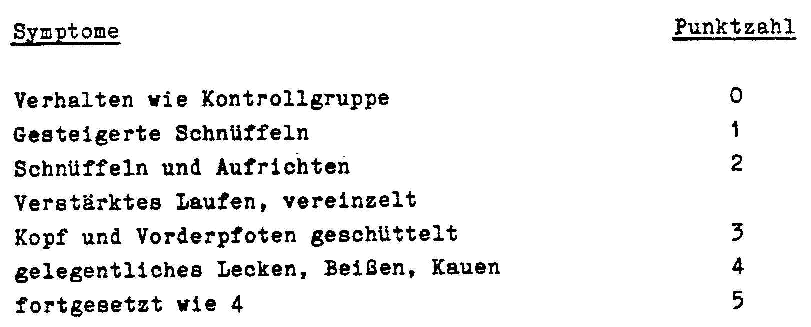

Die Punktzahlen wurden errechnet als Gesamtzahl pro 6 Stunden, dividiert durch die Zahl der Beobachtungen.

Die EDₛo-Dosis ist die Dosis, bei der die Hälfte der maximal möglichen Punktzahl (5 Punkte multipliziert mit der Zahl der Beobachtungen) erreicht wird.

### 4. Wiederaufnahmehemmung von Noradrenalin in Synaptosomen Methode

Synaptosomen aus Rattenhirn werden nach der Methode von Whittaker (Handbook of Neurochemistry 2, 327-364, Editor A. Lajtha; London and New York, 1969) isoliert und die Monoamin-Aufnahme nach der Methode von Schacht und Heptner gemessen (Biochemical Pharmac. 23, 3413-3422). Die ¹⁴C-Noradrenalin-Aufnahme wurde in einem Krebs-Henseleit-Bikarbonatpuffer pH 7,4, der 11 Millimol Glucose enthielt, gemessen. 2,5 ml der Synaptosomen-Suspension wurden mit markiertem Noradrenalin bei 37 C inkubiert in Gegenwart von oder ohne Testsubstanz. Die Inkubationszeit betrug 4 Minuten. Die weitere Aufnahme wurde dann durch Abkühlen mit Eis gestoppt. Um nichtspezifische Adsorptionen auszuschließen, wurden Kontrollproben bei 0 C unter sonst gleichen Bedingungen inkubiert.

Die aufgenommenen Noradrenalinmengen wurden mit Hilfe der Membranfiltrationstechnik mit einem Millipore Sampling Manifold mit Cellulosenitratfiltern von 25 mm Durchmesser und 0,6 Mikrometer Porengröße gemessen. Die Synaptosomen wurden unter vermindertem Druck gesammelt und die Radioaktivität in einem Packard-Tricarb-Scintillationszähler bestimmt. Die Menge an angesammeltem Noradrenalin wurde angegeben als Prozent Radioaktivität, die zur Inkubationsmischung zugesetzt wurde.

Die ICso-Werte (inhibition concentration) der nachstehenden Tabelle geben die Konzentrationen der Testsubstanzen an, welche die Aufnahme von ¹⁴C-Noradrenalin zu 50% hemmen.

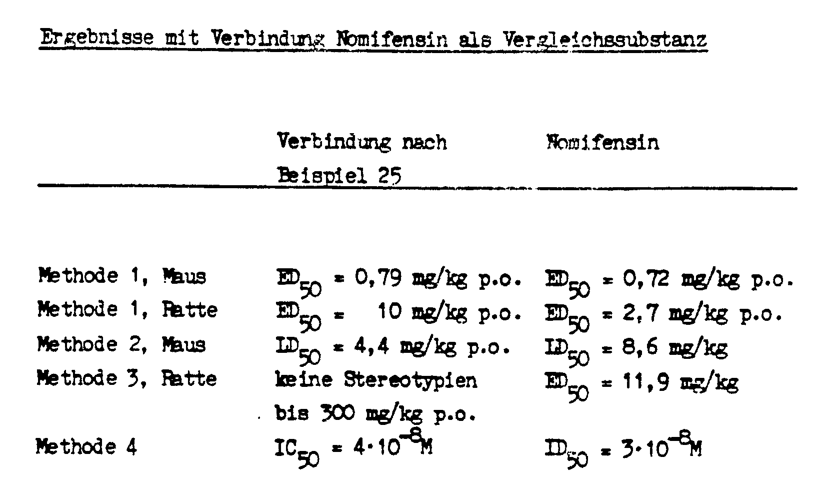

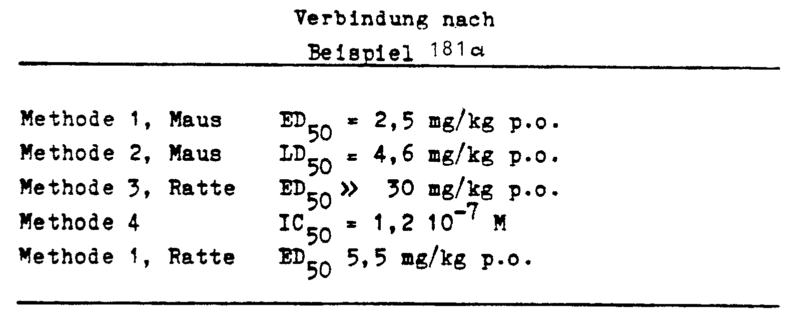

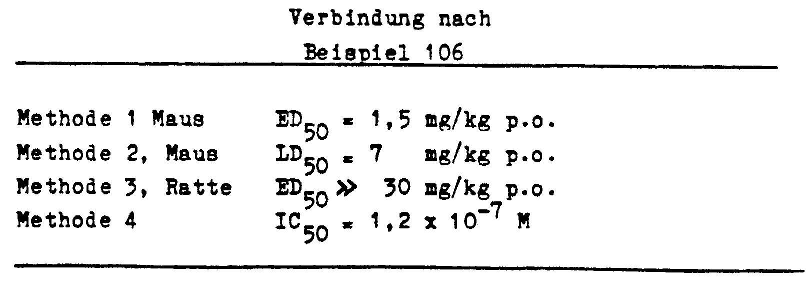

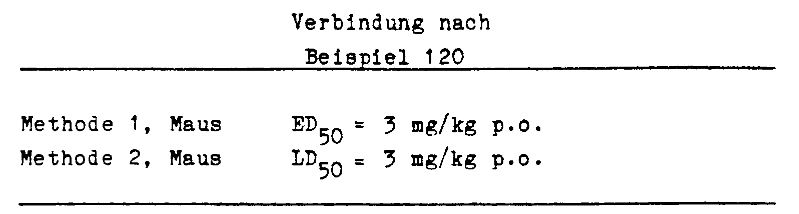

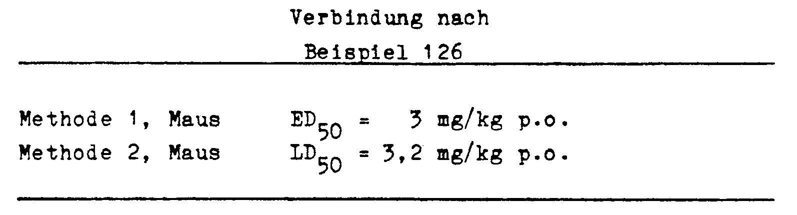

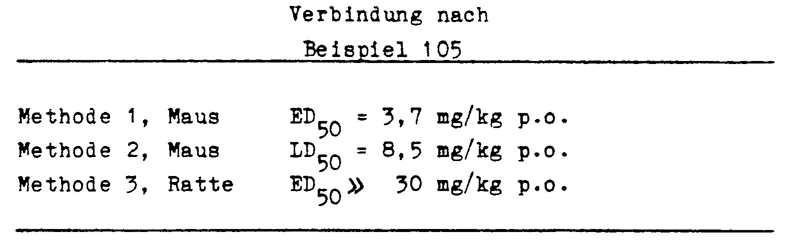

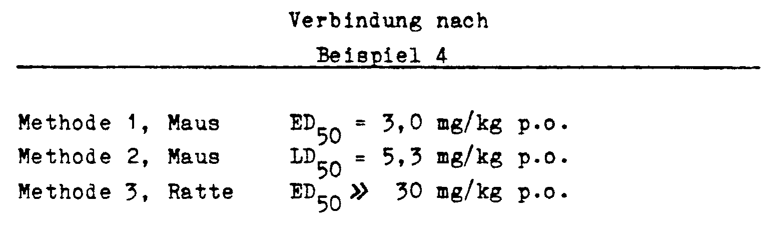

## Patentansprüche (Patentansprüche für folgende(n) Vertragsstaat(en) : BE, CH, DE, FR, GB, IT, LI, LU, NL, SE)

1. Verbindung der Formel I 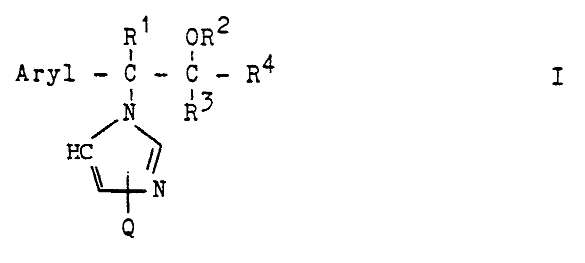 in der bedeuten
Aryl einen Rest 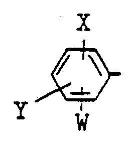 oder einen unsubstituierten oder durch einen Substituenten U und/oder einen Substituenten V substituierten 1- oder 2-Naphthylrest, wobei
X H, (C₁-C₄)-Alkyl, Phenyl, Fluor, Chlor, Brom, (C₁-C₄)-Alkoxy, 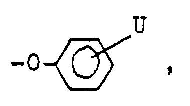 (C₁-C₄)-Alkylthio, -N(R⁵)₂,in dem R^{S -} gleich oder verschieden - (C₁-C₄)-Alkyl oder zusammen mit dem Stickstoffatom einen Pyrrolidin-, Piperidin- oder Morpholin-Rest bedeutet, CF₃ oder eine unsubstituierte oder eine im Phenylrest 1 oder 2 Substituenten tragende Benzyloxy-Gruppe, wobei die Substituenten gleich oder verschieden sind und Fluor, Chlor, OCH₃, OC₂H₅ oder (C₁-C₃)-Alkyl bedeuten,
Y H, (C₁-C₄)-Alkyl, Fluor, Chlor, Brom, (C₁-C₄)-Alkoxy oder (C₁-C₄)-Alkylthio, oder
X und Y zusammen in 2,3- oder 3,4-Stellung eine -(CH₂)_{L}-Kette mit L= 3 oder 4, -OCH₂CH₂-oder -O-CH₂-O-,
W H, CH₃ oder OCH₃,
V (C₁-C₄)-Alkyl, Phenyl, Fluor, Chlor, Brom, Hydroxy, (C₁-C₄)-Alkoxy, (C₁-C₄)-Alkylthio, -N(R⁵)₂, in dem R⁵ (C₁-C₄)-Alkyl oder Zusammen mit dem Stickstoffatom einen Pyrrolidin-, Piperidin- oder Morpholin-Rest bedeutet, Benzyloxy oder CF₃ und
U CH₃, F, CI oder OCH₃ bedeuten,
R¹ H
Q H oder (C₁-C₄)-Alkyl,
_{R}² H,
R³ (C₁-C₁₂)-Alkyl, (C₃-C₁₀)-Alkenyl, (C3-C1z)-Cycloalkyl, (C₅-C₁₂)-Cycloalkenyl, (C₇-C₁₂)-Polycycloalkyl, (C₇-C₁₂)-Polycycloalkenyl, (C₅-C₁₂)-Cycloalkyl-(C₁-C₄)-Alkyl, (C₅-C₁₂)-Cycloalkyliden-(C₂-C₄)-Alkyl, (C₅-C₁₂)-Cycloalkenyl-(C₁-C₄)-Alkyl, (C₇-C₁₂)-Polycycloalkyl-(C₁-C₄)-Alkyl, (C₇-C₁₂ )-Polycycloalkyliden-(C₂-C₄)-Alkyl, (C₇-C₁₂)-Polycycloalkenyl-(C₁-C₄)-Alkyl oder ebendiese Kohlenwasserstoffreste mit bis zu 3 gleichen oder verschiedenen Substituenten, wobei diese F, CI, Br oder CH₃ bedeuten, oder
eine unsubstituierte oder eine im Phenylrest bis zu 3 Substituenten tragende Phenyl-(C₂-C₄)-Alkyl-Gruppe, wobei die Substitutenten gleich oder verschienen sind und F, Cl, Br, (C₁-C₄)-Alkoxy, (C₁-C₄)-Alkyl oder (C₁-C₄)-Alkylthio bedeuten,
oder eine unsubstituierte oder bis zu 2 Substituenten tragende Naphthyl-(C₁-C₄)-Alkyl- Gruppe, wobei die Substituenten gleich oder verschieden sind und F, Cl, Br, OCH₃, OC₂Hs oder (C₁-C₄)-Alkyl bedeuten,
R⁴ H,(C₁-C₁₂)-Alkyl, (C₃-Cio)-Alkenyl, (C₃-C₁₂)-Cycloalkyl, - (Cs-C₁₂)-Cycloalkenyl, (C₇-C₁₂)-Polycycloalkyl, (C₇-C₁₂)-Polycycloalkenyl, (C₅-C₁₂)-Cycloalkyl-(C₁-C₄)-Alkyl, (Cs-C₁₂)-Cycloalkyliden-(C₂-C₄)-Alkyl, (C₅-_{C12} )-Cycloalkenyl-(C₁-C₄)-Alkyl, (C₇-C₁₂)-Polycycloalkyl-(C₁-C₄)-Alkyl, (C₇-C₁₂)-Polycycloalkyliden-(C₂-C₄)-Alkyl, (C₇-C₁₂)-Polycycloalkenyl-(C₁-C₄)-Alkyl oder ebendiese Kohlenwasserstoffreste mit bis zu 3 gleichen oder verschiedenen Substituenten, wobei diese F, CI oder Br bedeuten und wobei die cyclischen Kohlenwasserstoffreste zusätzlich mit (C₁-C₄)-Alkyl substituiert sein können,
oder
eine unsubstituierte oder eine im Phenylrest bis zu 3 Substituenten tragende Phenyl-(C₂-C₄)-Alkyl-Gruppe, wobei die Substitutenten gleich oder Verschieden sind und F, Cl, Br, (C₁-C₄.)-Alkoxy, (C₁-C₄)-Alkyl oder (C₁-C₄)-Alkylthio bedeuten, oder eine unsubstituierte oder bis zu 2 Substituenten Tragende Naphtyl-(C₁-C₄)-Alkyl- Gruppe, wobei die Substituenten gleich oder verschieden sind und F, Cl, Br, OCH₃, OC₂H₅ oder (C₁-C₄)-Alkyl bedeuten,
oder
R³ und R⁴ gemeinsam
eine unsubstituierte oder mit (C₁-C₄)-Alkyl, OCH₃ oder Phenyl substituierte -(CH₂)ₙ-Kette mit n = 2 - 11 bedeuten, oder eine ebensolche Kohlenwasserstoffkette mit einer Doppelbindung,
oder
eine unsubstituierte oder mit (C₁-C₄)-Alkyl, OCH₃, CH₂OCH₃ oder Phenyl substituierte, einfach oder mehrfach überbrückte -(CH₂)ₘ-Kette mit m = 4 oder 5 und 1 bis 5 Brücken-Kohlenstoffatomen, wobei die Brücken-Kohlenstoffatome ihrerseits wiederum überbrückt sein können und eine Überbrückung keine oder eine C,C-Doppelbindung enthält,
sowie deren physiologisch verträgliche Säureadditionssalze und deren Stereoisomere und optisch aktiven Enantiomere.

2. Verbindung I nach Anspruch 1, dadurch gekennzeichnet, daß mindestens eines der folgenden Merkmale erfüllt ist:
Aryl einen Rest 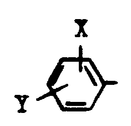 oder einen unsubstituierten oder durch einen Substituent V substituierten 2-Naphthyl- rest bedeutet, in denen
X H, (C₁-C₄)-Alkyl, Phenyl, F, CI, Br, (C₁-C₄)-Alkoxy, 3-CF₃ oder eine Benzyloxy-Gruppe,
Y H, CH₃, CI oder OCH₃ und
V (C₁-C₄)-Alkyl, Cl, Br, OH oder OCH₃ bedeuten,
Q H, CH₃ oder C₂H₅
R¹ H,
R² H,
R³ (C₁-C₈)-Alkyl, (C₃-C₁₀)-Alkenyl, (C₃-C₁₂)-Cycloalkyl, (C₅-C₁₂)-Cycloalkenyl, (C₇-C₁₂)-Polycycloalkyl, (C₇-C₁₂)-Polycycloalkenyl, (C₅-C₁₂)-Cycloalkyl-(C₁-C₄)-Alkyl, (C₅-C₁₂)-Cycloalkyliden-(C₂-C₄)-Alkyl, (C₅-C₁₂)-Cycloalkenyl-(C₁-C₄)-Alkyl, (C₇-C₁₂)-Polycycloalkyl-(C₁-C₄-)-Alkyl, (C₇-C₁₂)-Polycycloalkyllden-(C₂-C₄)-Alkyl, (C₇-C₁₂)-Polycycloalkenyl-(C₁-C₄)-Alkyl oder ebendiese cyclischen Kohlenwasserstoffreste mit bis zu 3 gleichen oder verschiedenen Substituenten, wobei diese F, Cl, Br oder CH₃ bedeuten
oder
eine unsubstituierte oder eine im Phenylrest bis zu 3 Substituenten tragende Phenyl-(C₂-C₄)-Alkyl-Gruppe, wobei die Substitutenten gleich oder verschieden sind und F, CI, Br, (C₁-C₄)-Alkoxy, (C₁-C₄)-Alkyl oder (C₁-C₄)-Alkylthio bedeuten,
R⁴ H, (C₁-C₁₂)-Alkyl, (C₃-C₅)-Alkenyl, Cyclopropyl,(C₅-C₈)-Cycloalkyl, eine unsubstituierte oder eine im Phenylrest bzw. Naphthylrest bis zu 2 Substituenten tragende Phenyl-(C₂-C₄)-Alkyl- oder Naphthyl-(C₁-C₄)-Alkyl-Gruppe, wobei die Substituenten gleich oder verschieden sind und F, Cl, Br, OCH₃, OC₂Hs oder (C₁-C₄)-Alkyl bedeuten, bedeuten, oder
R³ und R⁴ gemeinsam eine unsubstituierte oder mit (C₁-C₄)-Alkyl oder Phenyl substituierte -(CH₂)n-Kette mit n = 4 - 11,
oder eine ebensolche Kohlenwasserstoffkette mit einer Doppelbindung,
oder eine unsubstituierte oder mit CH₃, C₂Hₛ, OCH₃ oder CH₃OCH₂ substituierte,
einfach oder mehrfach überbrückte -(CH₂)m-Kette mit m = 4 oder 5 und 1 bis 5 Brücken-Kohlenstoffatomen, wobei die Brücken-Kohlenstoffatome ihrerseits wiederum überbrückt sein können und eine Überbrückung keine oder eine C,C-Doppelbindung enthält,
bedeuten.

3. Verbindung I nach einem der Ansprüche 1 oder 2, dadurch gekennzeichnet, daß mindestens eines der folgenden Merkmale erfüllt ist:
Aryl ein Rest 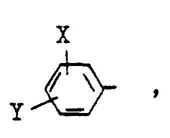 in dem
X (C₁-C₄)-Alkyl, Phenyl, F, CI oder (C₁-C₄)-Alkoxy,
Y H, CH₃, Cl oder OCH₃ bedeuten und die 6-Stellung stets unsubstituiert ist, oder ein unsubstituierter oder einfach mit Br oder Cl substituierter 2-Naphthyl-Rest ist,
Q, R¹ und R² H,
R³ (C₁-C₈)-Alkyl, (C₅-C₇)-Cycloalkyl, (C₇-C₁₂)-Polycyloalkyl, (C₇-C₁₂)-Polycycloalkenyl, (C₇-C₁₂)-Polycycloalkyl-(C₁-C₄)-Alkyl oder ebendiese cyclischen Kohlenwasserstoffreste mit 1 oder 2 gleichen oder verschiedenen Substituenten, wobei diese Cl, Br oder CH₃ bedeuten
oder
eine unsubstituierte oder eine im Phenylrest bis zu 3 Substituenten tragende Phenyl-(C₂-C₄)-Alkyl-Gruppe, wobei die Substitutenten gleich oder verschieden sind und F, Cl, Br, (C₁-C₄)-Alkoxy oder (C₁-C₄)-Alkylthio bedeuten,
R⁴ H, (C₁-C₄)-Alkyl, Cyclopentyl, Cyclohexyl, oder Cyclopropyl oder
R³ und R⁴ gemeinsam eine unsubstituierte oder mit CH₃ oder C₆H₅ substituierte -(CH₂)ₙ-Kette mit n = 4-7, oder
eine unsubstituierte oder mit CH₃ oder CH₃OCH₂ substituierte, einfach oder mehrfach überbrückte -(CH₂)ₘ-Kette mit m = 4 oder 5 und 1 bis 5 Brücken- Kohlenstoffatomen, wobei die Brücken-Kohlenstoffatome ihrerseits wiederum überbrückt sein können und eine Überbrückung keine oder eine C,C-Doppelbindung enthält, bedeuten.

4. Verbindung I nach Anspruch 1, dadurch gekennzeichnet, daß mindestens eines der folgenden Merkmale erfüllt ist:
Aryl 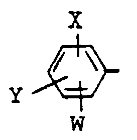 mit W = Wasserstoff X und Y Wasserstoff, F, CI oder Br
Q und R¹ Wasserstoff
_{R}2 Wasserstoff
R³ und R⁴ gemeinsam eine (CH₂)ₙ-Kette mit n= 4 bis 6 oder R³ und R⁴ eine überbrückte (CH₂)ₘ Kette mit m = 4 oder 5 und 1 bis 5 Brücken-Kohlenstoffatomen.

5. Verfahren zum Herstellen einer Verbindung I nach Anspruch 1, dadurch gekennzeichnet, daß man
a) ein Arylmethylazol der Formel II 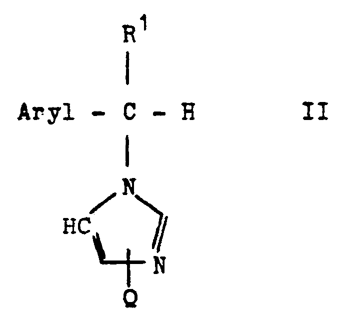 worin Aryl, Q und R¹ die oben angegebenen Bedeutungen haben, mit einem oder zwei Äquivalenten einer starken Base versetzt und anschließend mit einer Carbonylverbindung der Formel III, 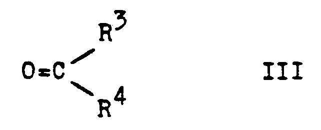 in welcher R³ und R die obengenannten Bedeutungen haben, zu einer Verbindung der Formel I umsetzt; oder
b) Oxirane der Formel V, 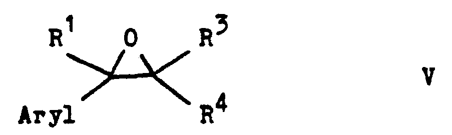 worin Aryl, R¹, R³ und R⁴ die obengenannte Bedeutung besitzen, mit einer Verbindung der Formel VI 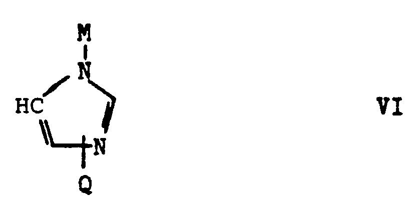 in der M Wasserstoff oder ein Alkali- oder Erdalkalimetall bedeutet, und Q die oben angegebene Bedeutung hat , umsetzt, oder
c) bei Verbindungen der Formel I, die nach Verfahren a) oder b) hergestellt worden sind, nach bekannten Methoden einen Substituenten X oder V in einem Aryl-Rest der Formel I in einen anderen Substituenten X oder V umwandelt, und gegebenenfalls die nach a), b) oder c) erhaltenen Verbindungen I mit anorganischen oder organischen Säuren in ihre Säureadditionssalze überführt und gegebenenfalls eine nach a), b) oder c) erhaltene Verbindung in ihre Stereoisomeren und/oder ihre optisch aktiven Enantiomeren auftrennt.

6. Verfahren zur Herstellung eines antidepressiv wirkenden Arzneimittels, dadurch gekennzeichnet, daß man eine wirksame Menge einer Verbindung I nach Anspruch 1 mit pharmazeutisch verträglichen Zusatzstoffen mischt.

7. Verwendung einer Verbindung der Formel I nach Anspruch 1 zur Herstellung eines antidepressiv wirkenden Arzneimittels.

8. Arzneimittel mit antidepressiver Wirkung, gekennzeichnet durch einen wirksamen Gehalt einer Verbindung I nach Anspruch 1.

## Patentansprüche (Patentansprüche für folgende(n) Vertragsstaat(en) : AT)

1. Verfahren zum Herstellen einer Verbindung der Formel I 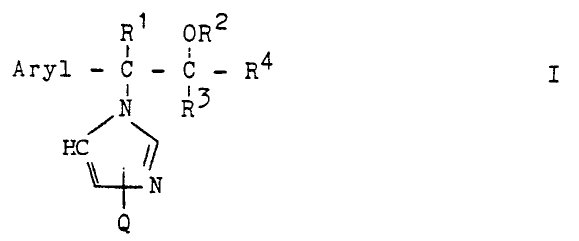 in der bedeuten
Aryl einen Rest 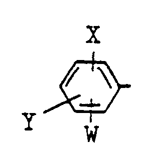 oder einen unsubstituierten oder durch einen Substituenten U und/oder einen Substituenten V substituierten 1- oder 2-Naphthylrest, wobei
X H, (C₁-C₄)-Alkyl, Phenyl, Fluor, Chlor, Brom, (C₁-C₄)-Alkoxy, 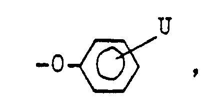 (C₁-C₄)-Alkylthio, -N(R⁵)₂,in dem R^{S -} gleich oder verschieden - (C₁-C₄)-Alkyl oder zusammen mit dem Stickstoffatom einen Pyrrolidin-, Piperidin- oder Morpholin-Rest bedeutet, CF₃ oder eine unsubstituierte oder eine im Phenylrest 1 oder 2 Substituenten tragende Benzyloxy-Gruppe, wobei die Substituenten gleich oder verschieden sind und Fluor, Chlor, OCH₃, OC₂Hₛ oder (C₁-C₃)-Alkyl bedeuten,
Y H, (C₁-C₄)-Alkyl, Fluor, Chlor, Brom, (C₁-C₄)-Alkoxy oder (C₁-C₄)-Alkylthio, oder
X und Y zusammen in 2,3- oder 3,4-Stellung eine -(CH2)L-Kette mit L= 3 oder 4, -OCH₂CH₂-oder -O-CH₂-O-,
W H, CH₃ oder OCH₃,
V (C₁-C₄)-Alkyl, Phenyl, Fluor, Chlor, Brom, Hydroxy, (C₁-C₄)-Alkoxy, (C₁-C₄)-Alkylthio, -N(R⁵)₂, in dem R⁵ (C₁-C₄)-Alkyl oder zusammen mit dem Stickstoffatom einen Pyrrolidin-, Piperidin- oder Morpholin-Rest bedeutet, Benzyloxy oder CF₃ und
U CH₃, F, CI oder OCH₃ bedeuten,
R¹ H,
Q H oder (C₁-C₄)-Alkyl,
R² H,
R³ (C1-C_{1z})-Alkyl, (C₃-C₁₀)-Alkenyl, (C3-C12)-Cycloalkyl, (C₅-C₁₂)-Cycloalkenyl, (C₇-C₁₂)-Polycycloalkyl, (C₇-C₁₂)-Polycycloalkenyl, (C₅-C₁₂)-Cycloalkyl-(C₁-C₄)-Alkyl, (C₅-C₁₂)-Cycloalkyliden-(C₂-C₄)-Alkyl, (C₅-C₁₂)-Cycloalkenyl-(C₁-C₄)Alkyl, (C₇-C₁₂)-Polycycloalkyl-(C₁-C₄)-Alkyl, (C₇-C₁₂)-Polycycloalkyliden-(C₂-C₄)-Alkyl, (C₇-C₁₂)-Polycycloalkenyl-(C₁-C₄)-Alkyl oder ebendiese Kohlenwasserstoffreste mit bis zu 3 gleichen oder verschiedenen Substituenten, wobei diese F, CI Br, oder CH₃, bedeuten,
oder
eine unsubstituierte oder eine im Phenylrest bis zu 3 Substituenten tragende Phenyl-(C₂-C₄)-Alkyl-Gruppe, wobei die Substitutenten gleich oder verschieden sind und F, CI, Br, (C₁-C₄)-Alkoxy, (C₁-C₄)-Alkyl oder (C₁-C₄)-Alkylthio bedeuten,
oder eine unsubstituierte oder bis zu 2 Substituenten tragende Naphthyl-(C₁-C₄)-Alkyl- Gruppe, wobei die Substituenten gleich oder verschieden sind und F, Cl, Br, OCH₃, OC₂Hs oder (C₁-C₄)-Alkyl bedeuten,
R⁴⁻ H,(C₁-_{1z})-Alkyl, (C₃-C₁₀)-Alkenyl, (C₃-C₁₂)-Cycloalkyl, (C₅-C₁₂)-Cycloalkenyl, (C₇-C ₁₂)-Polycycloalkyl, (C₇-C₁₂)-Polycycloalkenyl, (C₅-C₁₂)-Cycloalkyl-(C₁-C₄)-Alkyl, (Cₛ-C₁₂)-Cycloalkyliden-(C₂-C₄)-Alkyl, (C₅-C₁₂)-Cycloalkenyl-(C₁-C₄)-Alkyl, (C₇-C₁₂)-Polycycloalkyl-(C₁-C₄)-Alkyl, (C₇-C₁₂)-Polycycloalkyliden-(C₂-C₄)-Alkyl, (C₇-C₁₂)-Polycycloalkenyl-(C₁-C₄)-Alkyl oder ebendiese Kohlenwassserstoffreste mit bis zu 3 gleichen oder verschiedenen Substituenten, wobei diese F, CI oder Br bedeuten, und wobei die cyclischen Kohlenwasserstoffreste zusätzlich mit (C₁-C₄)-Alkyl substituiert sein können, oder
eine unsubstituierte oder eine im Phenylrest bis zu 3 Substituenten tragende Phenyl-(C₂-C₄)-Alkyl-Gruppe, wobei die Substitutenten gleich oder verschieden sind und F, Cl, Br, (C₁-C₄)-Alkoxy, (C₁-C₄)-Alkyl oder (C₁-C₄)-Alkylthio bedeuten,
oder eine unsubstituierte oder bis zu 2 Substituenten tragende Naphthyl-(C₁-C₄)-Alkyl- Gruppe, wobei die Substituenten gleich oder verschieden sind und F, Cl, Br, OCH₃, OC₂H₅ oder (C₁-C₄)-Alkyl bedeuten, oder
R³ und R⁴ gemeinsam eine unsubstituierte oder mit (C₁-C₄)-Alkyl, OCH₃ oder Phenyl substituierte -(CH2)n-Kette mit n = 2 - 11 bedeuten, oder eine ebensolche Kohlenwasserstoffkette mit einer Doppelbindung, oder
eine unsubstituierte oder mit (C₁-C₄)-Alkyl, OCH₃, CH₂OCH₃ oder Phenyl substituierte, einfach oder mehrfach überbrückte -(CH₂)ₘ-Kette mit m= 4 oder 5 und 1 bis 5 Brücken-Kohlenstoffatomen, wobei die Brücken-Kohlenstoffatome ihrerseits wiederum überbrückt sein können und eine Überbrückung keine oder eine C,C-Doppelbindung enthält,
sowie deren physiologisch verträgliche Säureadditionssalze und deren Stereoisomere und optisch aktiven Enantiomere, dadurch gekennzeichnet, daß man
a) ein Arylmethylazol der Formel II, 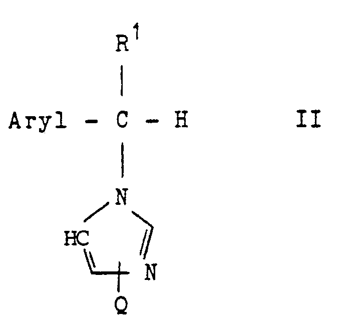 worin Aryl, Q und R¹ die oben angegebenen Bedeutungen haben, mit einem oder zwei Äquivalenten einer starken Base versetzt und anschließend mit einer Carbonylverbindung der Formel III, 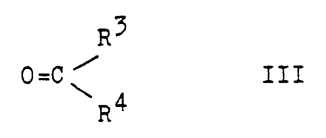 in welcher R³ und R⁴ die obengenannten Bedeutungen haben, zu einer Verbindung der Formel I umsetzt; oder
b) Oxirane der Formel V, 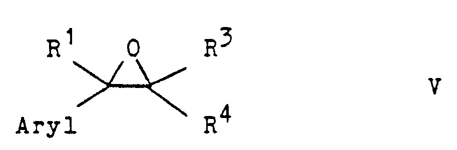 worin Aryl, R¹, R³ und R⁴ die obengenannte Bedeutung besitzen, mit einer Verbindung der Formel VI, 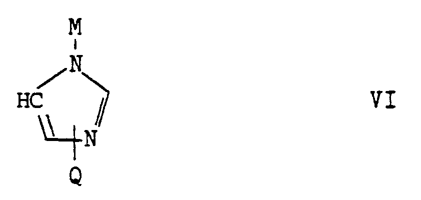 in der M Wasserstoff oder ein Alkali- oder Erdalkalimetall bedeutet, und Q die oben angegebene Bedeutung hat, umsetzt, oder
c) bei Verbindungen der Formel I, die nach Verfahren a) oder b) hergestellt worden sind, nach bekannten Methoden einen Substituenten X oder V in einem Aryl-Rest der Formel I in einen anderen Substituenten X oder V umwandelt, und gegebenenfalls die nach a), b) oder c) erhaltenen Verbindungen I mit anorganischen oder organischen Säuren in ihre Säureadditionssalze überführt und gegebenenfalls eine nach a), b) oder c) erhaltene Verbindung in ihre Stereoisomeren und/oder ihre optisch aktiven Enantiomeren auftrennt.

2. Verfahren nach Anspruch 1, dadurch gekennzeichnet, daß mindestens eines der folgenden Merkmale erfüllt ist:
Aryl einen Rest 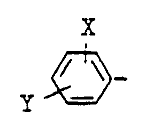 oder einen unsubstituierten oder durch einen Substituent V substituierten 2-Naphthyl- rest bedeutet, in denen
X H, (C₁-C₄)-Alkyl, Phenyl, F, Cl, Br, OH, (C₁-C₄)-Alkoxy, 3-CF₃ oder eine Benzyloxy-Gruppe,
Y H, CH₃, CI oder OCH₃ und
V (C₁-C₄)-Alkyl, CI, Br, OH oder OCH₃ bedeuten,
Q₁ H, CH₃ oder C₂H₅
R¹ H,
R² H,
R³ (C₁-C₈)-Alkyl, (C₃-C₁₀)-Alkenyl, (C₃-C₁₂)-Cycloalkyl, (C₅-C₁₂)-Cycloalkenyl, (C₇-C₁₂)-Polycycloalkyl, (C₇-C₁₂)-Polycycloalkenyl, (C₅-C₁₂)-Cycloalkyl-(C₁-C₄)-Alkyl, (C₅-C₁₂)-Cycloalkyliden-(C₂-C₄)-Alkyl, (C₅-C₁₂)-Cycloalkenyl-(C₁-C₄)-Alkyl, (C₇-C₁₂)-Polycycloalkyl-(C₁-C₄)-Alkyl, (C₇-C₁₂-Polycycloalkyliden-(C₂-C₄)-Alkyl, (C₇-C₁₂)-Polycycloalkenyl-(C₁-C₄-)-Alkyl oder ebendiese cyclischen Kohlenwasserstoffreste mit bis Zu 3 gleichen oder verschiedenen Substituenten, wobei diese F, Cl, Br oder CH₃ bedeuten oder
eine unsubstituierte oder eine im Phenylrest bis Zu 3 Substituenten tragende Phenyl-(C₂-C₄)-Alkyl-Gruppe, wobei die Substitutenten gleich oder verschieden sind und F, CI, Br, (C₁-C₄)-Alkoxy, (C₁-C₄)-Alkyl oder (C₁-C₄)-Alkythio bedeuten,
R⁴ H, (C₁-C₁₂)-Alkyl, (C₃-Cₛ)-Alkenyl, Cyclopropyl,(C₅-C₈)-Cycloalkyl, eine unsubstituierte oder eine im Phenylrest bzw. Naphthylrest bis zu 2 Substituenten tragende Phenyl-(C₂-C₄)-Alkyl-oder Naphthyl-(C₁-C₄)-Alkyl-Gruppe, wobei die Substituenten gleich oder verschieden sind und F, Cl, Br, OCH₃, OC₂Hs oder (C₁-C₄)-Alkyl bedeuten, bedeuten, oder
R³ und R⁴ gemeinsam eine unsubstituierte oder mit (C₁-C₄)-Alkyl oder Phenyl substituierte -(CH2)n-Kette mit n = 4 - 11, oder eine ebensolche Kohlenwasserstoffkette mit einer Doppelbindung,
oder eine unsubstituierte oder mit CH₃, C₂H₅, OCH₃ oder CH₃OCH₂ substituierte, einfach oder mehrfach überbrückte -(CH2)m-Kette mit m = 4 oder 5 und 1 bis 5 Brücken-Kohlenstoffatomen, wobei die Brücken-Kohlenstoffatome ihrerseits wiederum überbrückt sein können und eine Überbrückung keine oder eine C,C-Doppelbindung enthält, bedeuten.

3. Verfahren nach einem der Ansprüche 1 oder 2, dadurch gekennzeichnet, daß mindestens eines der folgenden Merkmale erfüllt ist: -
Aryl ein Rest 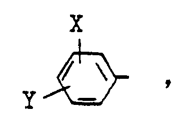 in dem
X (C₁-C₄)-Alkyl, Phenyl, F, CI oder (C₁-C₄)-Alkoxy,
Y H, CH₃, CI oder OCH₃ bedeuten und die 6-Stellung stets unsubstituiert ist, oder ein unsubstituierter oder einfach mit Br oder CI substituierter 2-Naphthyl-Rest ist,
Q, R¹ und R² _{H},
R³ (C₁-C₈)-Alkyl, (C₅-C₇)-Cycloalkyl, (C₇-C₁₂)-Polycyloalkyl, (C₇-C₁₂)-Polycycloalkenyl, (C₇-C₁₂)-Polycycloalkyl-(C₁-C₄)-Alkyl oder ebendiese cyclischen Kohlenwasserstoffreste mit 1 oder 2 gleichen oder verschiedenen Substituenten, wobei diese Cl, Br oder CH₃ bedeuten
oder
eine unsubstituierte oder eine im Phenylrest bis zu 3 Substituenten tragende Phenyl-(C₂-C₄)-Alkyl-Gruppe, wobei die Substitutenten gleich oder verschieden sind und F, Cl, Br, (C₁-C₄)-Alkoxy oder (C₁-C₄)-Alkylthio bedeuten,
R⁴ H, (C₁-C₄)-Alkyl, Cyclopentyl, Cyclohexyl, oder Cyclopropyl oder
R³ und R⁴ gemeinsam eine unsubstituierte oder mit CH₃ oder C₆H₅ substituierte -(CH₂)ₙ-Kette mit n = 4 - 7, oder
eine unsubstituierte oder mit CH₃ oder CH₃OCH₂ substituierte, einfach oder mehrfach überbrückte -(CH₂)ₘ-Kette mit m= 4 oder 5 und 1 bis 5 Brücken- Kohlenstoffatomen, wobei die Brücken-Kohlenstoffatome ihrerseits wiederum überbrückt sein können und eine Überbrückung keine oder eine C,C-Doppelbindung enthält, bedeuten.

4. Verfahren nach Anspruch 1, dadurch gekennzeichnet, daß mindestens eines der folgenden Merkmale erfüllt ist:
Aryl 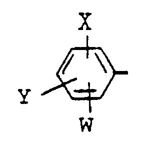 mit W = Wasserstoff X und Y Wasserstoff, F, CI oder Br
Q und R¹ Wasserstoff
_{R}2 Wasserstoff
R3 und R⁴ gemeinsam eine (CH₂)ₙ-Kette mit n= 4 bis 6 oder R³ und R⁴ eine überbrückte (CH₂)ₘ₋Kette mit m = 4 oder 5 und 1 bis 5 Brücken-Kohlenstoffatomen.

5. Verfahren zur Herstellung eines antidepressiv wirkenden Arzneimittels, dadurch gekennzeichnet, daß man eine wirksame Menge einer Verbindung I nach Anspruch 1 mit pharmazeutisch verträglichen Zusatzstoffen mischt.

6. Verwendung einer Verbindung der Formel I nach Anspruch 1 zur Herstellung eines antidepressiv wirkenden Arzneimittels.

## Claims (Claims for the following Contracting State(s) : BE, CH, DE, FR, GB, IT, LI, LU, NL, SE)

1. A compound of the formula I 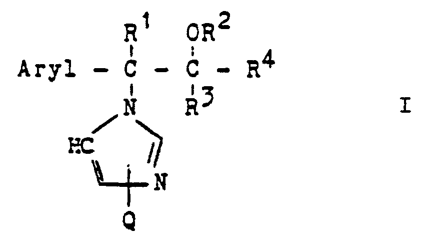 in which
Aryl denotes a radical 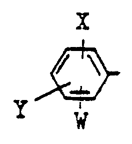 or a one- or two-naphthyl radical which is unsubstituted or substituted by U and/or a substituent V, where
X denotes H, (C₁-C₄)-alkyl, phenyl, fluorine, chlorine, bromine, (C₁-C₄)-alkoxy, 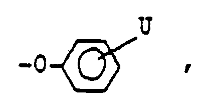 (C₁-C₄)-alkylthio, -N(R⁵)₂, in which the radicals R^{5 -} identical or different - denote (C₁-C₄)-alkyl or together with the nitrogen atom denote a pyrrolidine, piperidine or morpholine radical, or X denotes CF₃ or a benzyloxy group which is unsubstituted or carries one or two substituents in the phenyl radical, the substituents being identical or different and denoting fluorine, chlorine, OCH₃, OC₂H₅ or (C₁-C₃)-alkyl,
Y denotes H, (C₁-C₄)-alkyl, fluorine, chlorine, bromine, (C₁-C₄)-alkoxy or (C₁-C₄)-alkylthio, or
X and Y together in the 2,3- or 3,4-position denote a -(CH₂)_{L}- chain, in which L = 3 or 4, -OCH₂CH₂- or -O-CH₂-O-,
W denotes H, CH₃ or OCH₃,
V denotes (C₁-C₄)-alkyl, phenyl, fluorine, chlorine, bromine, hydroxyl, (C₁-C₄)-alkoxy, (C₁-C₄)-alkylthio, -N(R⁵)₂, in which R⁵ denotes (Cₗ-C₄)-alkyl or together with the nitrogen atom denotes a pyrrolidine, piperidine or morpholine radical, benzyloxy or CF₃, and
U denotes CH₃, F, CI or OCH₃,
R¹ denotes H,
Q denotes H or (C₁-C₄)-alkyl,
_{R}2 denotes H,
R³ denotes (C₁-C₁₂)-alkyl, (C₃-C₁₀)-alkenyl, (C3-C1z)-cycloalkyl, (C₅-C₁₂)-cycloalkenyl, (C₇-C₁₂)-polycycloalkyl,(C₇-C₁₂)-polycycloalkenyl, (C₅-C₁₂)-cycloalkyl-(C₁-C₄)-alkyl, (C₅-C₁₂)-cycloalkylidene-(C₂-C₄)-alkyl, (C₁-C₄)-cycloalkenyl-(C₁-C₄)-alkyl, (C₇-C₁₂)-polycycloalkyl-(C₁-C₄)-alkyl, (C₇-C₁₂)-polycycloalkylidene-(C₂-C₄)-alkyl, (C₇-C₁₂)-polycycloalkenyl-(C₁-C₄)-alkyl or these hydrocarbon radicals possessing up to 3 identical or different substituents, the latter denoting F, Cl, Br or CH₃, or a phenyl-(C₂-C₄)-alkyl group which is unsubstituted or carries up to 3 substituents in the phenyl radical, the substituents being identical or different and denoting F, Cl, Br,(C₁-C₄)-alkoxy, (C₁-C₄)-alkyl or (C₁-C₄)-alkythio, or a naphthyl-(C₁-C₄)-alkyl group which is unsubstituted or carries up to 2 substituents, the substituents being identical or different and denoting F, Cl, Br, OCH₃, OC₂H₅ or (C₁-C₄)-alkyl,
R⁴ denotes H, (C₁-C₁₂)-alkyl, (C₃-C₁₀)-alkenyl, (C₃-C₁₂)-cycloalkyl, (C₅-C₁₂)-cycloalkenyl, (C₇-C₁₂)-polycycloalkyl, (C₇-C₁₂)-polycycloalkenyl, (C₅-C₁₂)-cycloalkyl-(C₁-C₄)-alkyl, (C₅-C₁₂)-cycloalkylidene-(C₂-C₄)-alkyl, (C₅-C₁₂)-cycloalkenyl-(C₁-C₄)-alkyl, (C₇-C₁₂)-polycycloalkyl-(C₁-C₄)-alkyl, (C₇-C₁₂)-polycycloalkylidene-(C₂-C₄)-alkyl, (C₇-C₁₂)-polycycloalkenyl-(C₁-C₄)-alkyl or these hydrocarbon radicals possessing up to 3 identical or different substituents, the latter denoting F, CI or Br and it being possible for the cyclic hydrocarbon radicals additionally to be substituted by (C₁-C₄)-alkyl, or a phenyl-(C₂-C₄)-alkyl group which is unsubstituted or carries up to 3 substituents in the phenyl radical, the substituents being identical or different and denoting F, Cl, Br, (C₁-C₄)-alkoxy, (C₁-C₄)-alkyl or (C₁-C₄)-alkylthio, or a naphthyl-(C₁-C₄)-alkyl group which is unsubstituted or carries up to 2 substituents, the substituents being identical or different and denoting F, Cl, Br, OCH₃, OC₂H₅ or (C₁-C₄)-alkyl, or
R³ and R⁴ together denote a-(CH₂)ₙ-chain, in which n = 2-11, which is unsubstituted or substituted by (Cₗ-C₄)-alkyl,or OCH₃ or phenyl, or such a hydrocarbon chain possessing a double bond, or a -(CH₂)ₘ-chain which is unsubstituted or substituted by (Cₗ-C₄)-alkyl, OCH₃, CH₂OCH₃ or phenyl and is singly or multiply bridged, in which m = 4 or 5 and which contains 1 to 5 bridge carbon atoms, where the bridge carbon atoms in turn may be bridged and a bridge contains no C-C double bonds or one C-C double bond,
and its physiologically tolerated acid addition salts and its stereoisomers and optically active enantiomers.

2. A compound I as claimed in claim 1, wherein at least one of the following characteristics is fulfilled:
Aryl denotes a radical 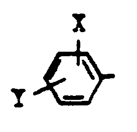 or a 2-naphthyl radical which is unsubstituted or substituted by a substituent V, in which
X denotes H, (Cₗ-C₄)-alkyl, phenyl, F, Cl, Br, (C₁-C₄)-alkoxy, 3-CF₃ or a benzyloxy group,
Y denotes H, CH₃, CI or OCH₃ and
V denotes (C₁-C₄)-alkyl, Cl, Br, OH or OCH₃,
Q denotes H, CH₃ or C₂H₅,
_{R1} denotes H,
R² denotes H,
R³ denotes (C₁-C₈)-alkyl, (C₃-C₁₀)-alkenyl, (C₃-C₁₂)-cycloalkyl, (C₅-C₁₂)-cycloalkenyl, ( C₇-C₁₂)-polycycloalkyl, (C₇-C₁₂)-polycycloalkenyl, (C₅-C₁₂)-cycloalkyl-(C₁-C₄)-alkyl, (C₅-C₁₂)-cycloalkylidene-(C₂-C₄)-alkyl, (C₅-C₁₂)-cycloalkenyl-(C₁-C₄)-alkyl, (C₇-C₁₂)-polycycloalkyl-(C₁ -C₄)-alkyl, (C₇-C₁₂)-polycycloalkylidene-(C₂-C₄)-alkyl, (C₇-C₁₂)-polycycloalkenyl-(C₁-C₄)-alkyl or these cyclic hydrocarbon radicals possessing up to 3 identical or different substituents, the latter denoting F, Cl, Br or CH₃, or a phenyl-(C₂-C₄)-alkyl group which is unsubstituted or carries up to 3 substituents in the phenyl radical, the substituents being identical or different and denoting F, Cl, Br, (C₁-C₄)-alkoxy,(C₁-C₄)-alkyl or (C₁-C₄)-alkylthio,
R4 denotes H, (C₁-C₁₂)-alkyl, (C₃-Cₛ)-alkenyl, cyclopropyl, (C₅-C₈)-cycloalkyl, or a phenyl-(C₂-C₄)-alkyl or naphthyl-(C₁-C₄)-alkyl group which is unsubstituted or carries up to 2 substituents in the phenyl radical or naphthyl radical, the substituents being identical or different and denoting F, Cl, Br, OCH₃, OC₂H₅ or (C₁-C₄)-alkyl, or
R³ and R4 together denote a -(CH₂)ₙ-chain which is unsubstituted or substituted by (C₁-C₄)-alkyl or phenyl, in which n = 4-11, or a hydrocarbon chain of this type which contains a double bond, or a -(CH₂)ₘ- chain which is unsubstituted or substituted by CH₃, C₂H₅, OCH₃ or CH₃0CH₂ and is singly or multiply bridged, in which m = 4 or 5 and which contains 1 to 5 bridge carbon atoms, where the bridge carbon atoms in turn may be bridged and a bridge contains no C-C double bonds or one C-C double bond.

3. A compound I as claimed in one of claims 1 or 2, wherein at least one of the following characteristics is fulfilled:
Aryl denotes a radical 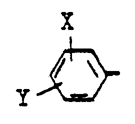 in which
X denotes (C₁-C₄)-alkyl, phenyl, F, CI or (C₁-C₄)-alkoxy,
Y denotes H, CH₃, CI or OCH₃ and the 6-position is always unsubstituted, or a 2-naphthyl radical which is unsubstituted or monosubstituted by Br or Cl,
Q, R¹ and R² denote H,
R³ denotes (C₁-C₈)-alkyl, (C₅-C₇)-cycloalkyl, (C₇-C₁₂)-polycycloalkyl, (C₇-C₁₂)-polycycloalkenyl, (C₇-C₁₂)-polycycloalkyl-(C₁-C₄)-alkyl or the cyclic hydrocarbon radicals possessing 1 or 2 identical or different substituents, the latter denoting Cl, Br or CH₃, or a phenyl-(C₂-C₄)-alkyl group which is unsubstituted or carries up to 3 substituents in the phenyl radical, the substituents being identical or different and denoting F, Cl, Br, (C₁-C₄)-alkoxy or (C₁-C₄)-alkylthio,
R⁴ denotes H, (C₁-C₄)-alkyl, cyclopentyl, cyclohexyl or cyclopropyl, or
R³ and R⁴ together denote a - (CH₂)ₙ- chain which is unsubstituted or substituted by CH₃ or C₆H₅, in which n = 4-7 or
a -(CH_{z})ₘ- chain which is unsubstituted or substituted by CH₃ or CH₃OCH₂ and is singly or multiply bridged, in which m = 4 or 5 and which has 1 to 5 bridge carbon atoms, where the bridge carbon atoms in turn may be bridged and a bridge contains no C-C double bonds or one C-C double bond.

4. A compound I as claimed in claim 1, wherein at least one of the following characteristics is fulfilled:
Aryl denotes 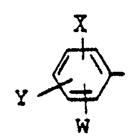 in which W denotes hydrogen and
X and Y denote hydrogen, F, CI or Br,
Q and R¹ denote hydrogen,
R² denotes hydrogen and
R³ and R4 together denote a -(CH₂)ₙ- chain in which n = 4 to 6, or R³ and R4 denote a bridged -(CH₂)-ₘ- chain in which m = 4 or 5 and which has 1 to 5 bridge carbon atoms.

5. A process for the preparation of a compound I as claimed in claim 1, wherein
a) one or two equivalents of a strong base are added to an arylmethylazole of the formula II 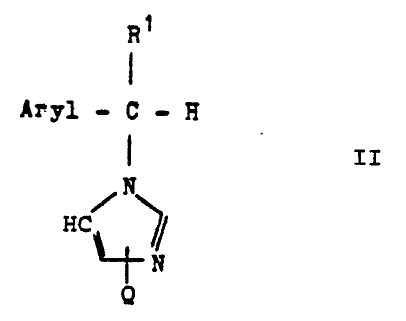 wherein aryl, Q and R' have the meanings given above, and the product is then reacted with a carbonyl compound of the formula III 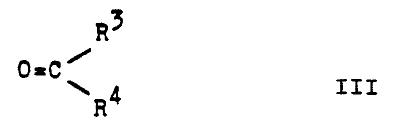 in which R³ and R4 have the abovementioned meanings, to give a compound of the formula I; or
b) oxiranes of the formula V 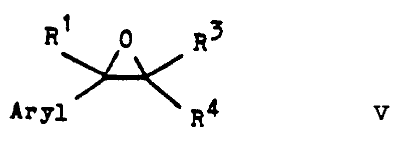 wherein aryl, R¹', R³ and R⁴ have the abovementioned meaning, are reacted with a compound of the formula VI 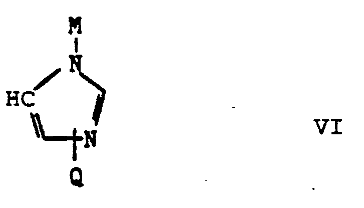 in which M denotes hydrogen or an alkali metal or alkaline earth metal and Q has the meaning given above, or
c) in the case of compounds of the formula I which have been prepared by process a) or b), a substituent X or V in an aryl radical of the formula I is converted to a different substituent X or V by known methods and, if appropriate, the compounds I obtained by a), b) or c) are converted to their acid addition salts with inorganic or organic acids and, if appropriate, a compound obtained by a), b) or c) is resolved into its stereoisomers and/or its optically active enantiomers.

6. A process for the preparation of a medicament having an antidepressant action, wherein an effective amount of a compound I as claimed in claim 1 is mixed with pharmaceutically tolerated additives.

7. Use of a compound of the formula I as claimed in claim 1 for the preparation of a medicament having an antidepressant action.

8. A medicament having an antidepressant action, which contains an effective amount of a compound I as claimed in claim 1.

## Claims (Claims for the following Contracting State(s) : AT)

1. A process for the preparation of a compound of the formula I 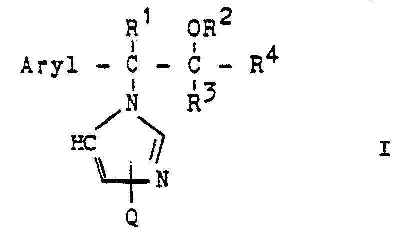 in which
Aryl denotes a radical 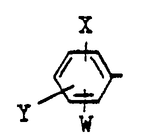 or a one- or two-naphthyl radical which is unsubstituted or substituted by U and/or a substituent V, where
X denotes H, (C₁-C₄)-alkyl, phenyl, fluorine, chlorine, bromine, (C₁-C₄)-alkoxy, 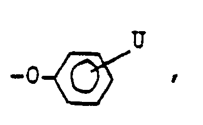 (C₁-C₄)-alkylthio, -N(R^{s})₂, in which the radicals R⁵- identical or different - denote (C₁-C₄)-alkyl or together with the nitrogen atom denote a pyrrolidine, piperidine or morpholine radical, or X denotes CF₃ or a benzyloxy group which is unsubstituted or carries one or two substituents in the phenyl radical, the substituents being identical or different and denoting fluorine, chlorine, OCH₃, OC₂Hs or (C₁-C₃)-alkyl,
Y denotes H, (C₁-C₄)-alkyl, fluorine, chlorine, bromine, (C₁-C₄)-alkoxy or (C₁-C₄)-alkylthio, or
X and Y together in the 2,3- or 3,4-position denote a -(CH₂)_{L}- chain, in which L = 3 or 4, -OCH₂CH_{z}- or -O-CH₂-O-,
W denotes H, CH₃ or OCH₃,
V denotes (C₁-C₄)-alkyl, phenyl, fluorine, chlorine, bromine, hydroxyl, (C₁-C₄)-alkoxy, (C₁-C₄)-alkylthio, -N(R⁵)₂, in which R⁵ denotes (C₁-C₄)-alkyl or together with the nitrogen atom denotes a pyrrolidine, piperidine or morpholine radical, benzyloxy or CF₃, and
U denotes CH₃, F, CI or OCH₃,
R¹ denotes H,
Q denotes H or (Cᵢ-C₄)-alkyl,
_{R}² denotes H,
R³ denotes (C₁-C₁₂)-alkyl, (C₃-Cₗₒ)-alkenyl, (C₃-C₁₂)-cycloalkyl, (C₅-C₁₂)-cycloalkenyl, (C₇-C₁₂)-polycycloalkyl, (C₇-C₁₂)-polycycloalkenyl, (C₅-C₁₂)-cycloalkyl-(C₁-C₄)-alkyl, (C₅-C₁₂)-cycloalkylidene-(C₂-C₄)-alkyl, (C₅-C₁₂)-cycloalkenyl-(C₁-C₄)-alkyl, (C₇-C₁₂)-polycycloalkyl-(C₁-C₄)-alkyl, (C₇-C₁₂)-polycycloalkylidene-(C₂-C₄)-alkyl, (C₇-C₁₂)-polycycloalkenyl-(C₁-C₄)-alkyl or these hydrocarbon radicals possessing up to 3 identical or different substituents, the latter denoting F, Cl, Br or CH₃,
or a phenyl- (C₂-C₄)-alkyl group which is unsubstituted or carries up to 3 substituents in the phenyl radical, the substituents being identical or different and denoting F, Cl, Br, (Cₗ-C₄)-alkoxy, (C₁-C₄)-alkyl or (C₁-C₄)-alkylthio,
or a naphthyl-(C₁-C₄)-alkyl group which is unsubstituted or carries up to 2 substituents, the substituents being identical or different and denoting F, Cl, Br, OCH₃, OC₂H₅ or (C₁-C₄)-alkyl,
R4 denotes H, (C₁-C₁₂)-alkyl, (C₃-C₁₀)-alkenyl, R⁴⁻ denotes H, (C₁-C₁₂)-alkyl, (C₃-C₁₀)-alkenyl, (C₃-C₁₂)-cycloalkyl, (C₅-C₁₂)-cycloalkenyl, (C₇-C₁₂)-polycycloalkyl, (C₇-C₁₂)-polycycloalkenyl, (C₅-C₁₂)-cycloalkyl-(C₁-C₄)-alkyl, (C₅-C₁₂)-cycloalkylidene-(C₂-C₄)-alkyl, (C₅-C₁₂)-cycloalkenyl-(C₁-C₄)-alkyl, (C₇-C₁₂)-polycycloalkyl-(C₁-C₄)-alkyl, (C₇-C, ₁₂)-polycycloalkylidene-(C₂-C₄)-alkyl, (C₇-C₁₂)-polycycloalkenyl-(C₁-C₄)-alkyl or these hydrocarbon radicals possessing up to 3 identical or different substituents, the latter denoting F, CI or Br and it being possible for the cyclic hydrocarbon radicals additionally to be substituted by (C₁-C₄)-alkyl,
or a phenyl- (C₂-C₄)-alkyl group which is unsubstituted or carries up to 3 substituents in the phenyl radical, the substituents being identical or different and denoting F, Cl, Br, (C₁-C₄)-alkoxy, (C₁-C₄)-alkyl or (C₁-C₄)-alkylthio,
or a naphthyl-(C₁-C₄)-alkyl group which is unsubstituted or carries up to 2 substituents, the substituents being identical or different and denoting F, Cl, Br, OCH₃, OC₂Hs or (C₁-C₄)-alkyl, or
R³ and R⁴ together denote a -(CH₂)ₙ-chain, in which n = 2-11, which is unsubstituted or substituted by (C₁-C₄)-alkyl,or OCH₃ or phenyl, or such a hydrocarbon chain possessing a double bond,
or a -(CH₂)ₘ-chain which is unsubstituted or substituted by (C₁-C₄)-alkyl, OCH₃, CH₂0CH₃ or phenyl and is singly or multiply bridged, in which m = 4 or 5 and which contains 1 to 5 bridge carbon atoms, where the bridge carbon atoms in turn may be bridged and a bridge contains no C-C double bonds or one C-C double bond,
and its physiologically tolerated acid addition salts and its stereoisomers and optically active enantiomers, wherein
a) one or two equivalents of a strong base are added to an arylmethylazole of the formula II 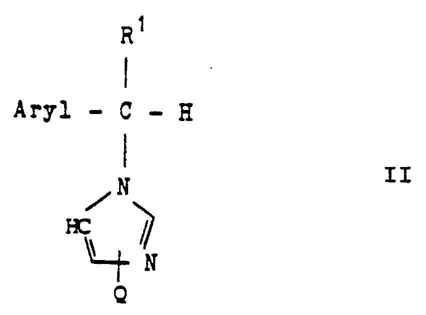 wherein aryl, Q and R' have the meanings given above, and the product is then reacted with a carbonyl compound of the formula III 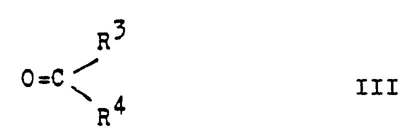 in which R³ and R4 have the abovementioned meanings, to give a compound of the formula I; or
b) oxiranes of the formula V 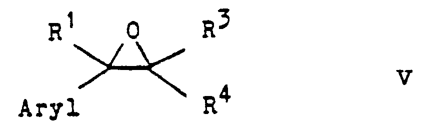 wherein aryl, R¹, R³ and R⁴ have the abovementioned meaning, are reacted with a compound of the formula VI, 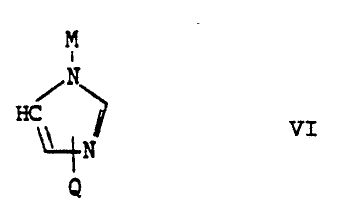 in which M denotes hydrogen or an alkali metal or alkaline earth metal and Q has the meaning given above, or
c) in the case of compounds of the formula I which have been prepared by process a) or b), a substituent X or V in an aryl radical of the formula I is converted to a different substituent X or V by known methods and, if appropriate, the compounds I obtained by a), b) or c) are converted to their acid addition salts with inorganic or organic acids and, if appropriate, a compound obtained by a), b) or c) is resolved into its stereoisomers and/or its optically active enantiomers.

2. The process as claimed in claim 1, wherein at least one of the following characteristics is fulfilled:
Aryl denotes a radical 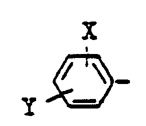 or a 2-naphthyl radical which is unsubstituted or substituted by a substituent V, in which
X denotes H, (C₁-C₄)-alkyl, phenyl, F, Cl, Br, OH, (C₁-C₄)-alkoxy, 3-CF₃ or a benzyloxy group,
Y denotes H, CH₃, CI or OCH₃ and
V denotes (C₁-C₄)-alkyl, Cl, Br, OH or OCH₃,
Q denotes H, CH₃ or C₂H₅,
R¹ denotes H,
R² denotes H,
R³ denotes (C₁-C₈)-alkyl, (C₃-C₁o)-alkenyl, (C₃-C₁₂)-cycloalkyl, (C₅-C₁₂)-cycloalkenyl, ( C₇-C₁₂)-polycycloalkyl, (C₇-C₁₂)-polycycloalkenyl, (C₅-C₁₂)-cycloalkyl-(C₁-C₄)-alkyl, (C₅-C₁₂)-cycloalkylidene-(C₂-C₄)-alkyl, (C₅-C₁₂)-cycloalkenyl-(C₁-C₄)-alkyl, (C₇-C₁₂)-polycycloalkyl-(C₁-C₄)-alkyl, (C₇-C₁₂)-polycycloalkylidene-(C₂-C₄)-alkyl, (C₇-C₁₂)-polycycloalkenyl-(C₁-C₄)-alkyl or these cyclic hydrocarbon radicals possessing up to 3 identical or different substituents, the latter denoting F, Cl, Br or CH₃,
or a phenyl-(C₂-C₄)-alkyl group which is unsubstituted or carries up to 3 substituents in the phenyl radical, the substituents being identical or different and denoting F, Cl, Br, (C₁-C₄)-alkoxy, (C₁-C₄)-alkyl or (C₁-C₄)-alkylthio,
R⁴ denotes H, (C₁-C₁₂)-alkyl, (C₃-C₅)-alkenyl, cyclopropyl, (C₅-C₈)-cycloalkyl, or a phenyl-(C₂-C₄)-alkyl or naphthyl-(C₁-C₄)-alkyl group which is unsubstituted or carries up to 2 substituents in the phenyl radical or naphthyl radical, the substituents being identical or different and denoting F, Cl, Br, OCH₃, OC₂H₅ or (C₁-C₄)-alkyl, or
R³ and R⁴ together denote a - (CH₂)ₙ-chain which is unsubstituted or substituted by (C₁-C₄)-alkyl or phenyl, in which n = 4-11,
or a hydrocarbon chain of this type which contains a double bond,
or a -(CH₂)ₘ- chain which is unsubstituted or substituted by CH₃, C₂H₅, OCH₃ or CH₃OCH₂ and is singly or multiply bridged, in which m = 4 or 5 and which contains 1 to 5 bridge carbon atoms, where the bridge carbon atoms in turn may be bridged and a bridge contains no C-C double bonds or one C-C double bond.

3. The process as claimed in one of claims 1 or 2, wherein at least one of the following characteristics is fulfilled:
Aryl denotes a radical 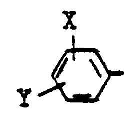 in which
X denotes (Cₗ-C₄)-alkyl, phenyl, F, Cl or (C₁-C₄)-alkoxy,
Y denotes H, CH₃, CI or OCH₃ and the 6-position is always unsubstituted, or a 2-naphthyl radical which is unsubstituted or monosubstituted by Br or Cl,
Q, R¹ and R² denote H,
R³ denotes (C₁-C₈)-alkyl, (C₅-C₇)-cycloalkyl, (C₇-C₁₂)-Polycycloalkyl, (C₇-C₁₂)-polycycloalkenyl, (C₇-C₁₂)-polycycloalkyl-(C₁-C₄)-alkyl or the cyclic hydrocarbon radicals possessing 1 or 2 identical or different substituents, the latter denoting Cl, Br or CH₃,
or a phenyl-(C₂-C₄)-alkyl group which is unsubstituted or carries up to 3 substituents in the phenyl radical, the substituents being identical or different and denoting F, Cl, Br, (C₁-C₄)-alkoxy or (C₁-C₄)-alkylthio,
R4 denotes H, (C₁-C₄)-alkyl, cyclopentyl, cyclohexyl or cyclopropyl, or
R³ and R4 together denote a -(CH₂)ₙ- chain which is unsubstituted or substituted by CH₃ or CsHs, in which n = 4-7 or
a -(CHz)ₘ- chain which is unsubstituted or substituted by CH₃ or CH₃OCH₂ and is singly or multiply bridged, in which m = 4 or 5 and which has 1 to 5 bridge carbon atoms, where the bridge carbon atoms in turn may be bridged and a bridge contains no C-C double bonds or one C-C double bond.

4. The process as claimed in claim 1, wherein at least one of the following characteristics is fulfilled:
Aryl denotes 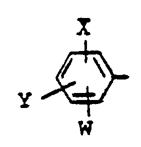 in which W denotes hydrogen and
X and Y denote hydrogen, F, CI or Br,
Q and R¹ denote hydrogen,
R² denotes hydrogen and
R³ and R⁴ together denote a (CH₂)ₙ- chain in which n = 4 to 6, or R³ and R⁴ denote a bridged (CH₂)ₘ- chain in which m = 4 or 5 and which has 1 to 5 bridge carbon atoms.

5. A process for the preparation of a medicament having an antidepressant action, wherein an effective amount of a compound I as claimed in claim 1 is mixed with pharmaceutically tolerated additives.

6. Use of a compound of the formula I as claimed in claim 1 for the preparation of a medicament having an antidepressant action.

## Revendications (Revendications pour l'(les) Etat(s) contractant(s) suivant(s) : BE, CH, DE, FR, GB, IT, LI, LU, NL, SE)

1. Composé de formule I 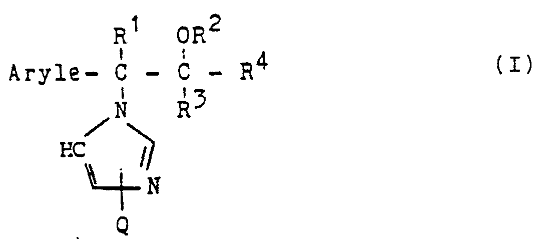 dans laquelle
"aryle" signifie un radical 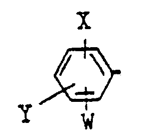 ou un radical 1- ou 2-naphtyle non substitué ou substitué par un substituant U et/ou un substituant V,
X représentant H, un groupe alkyle en C₁-C₄ ou phényle, un atome de fluor, chlore, brome, un groupe alcoxy en C₁-C₄, 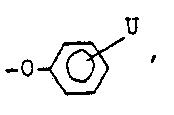
alkylthio en C₁-C₄, un groupe -N(R⁵)₂, dans lequel les restes R^{S -} identiques ou différents - représentent des groupes alkyle en Ci-C₄ ou forment ensemble, avec l'atome d'azote, un reste pyrrolidino, pipéridino ou morpholino,
CF³ ou un groupe benzyloxy non substitué ou portant sur le fragment phényle un ou deux substituants, les substituants étant identiques ou différents et représentant des atomes de fluor ou de chlore ou des groupes OCH₃, OC₂H₅ ou alkyle en C₁-C₃,
Y représentant H, un groupe alkyle en Ci-C₄, un atome de fluor, chlore, brome, un groupe alcoxy en C₁-C₄ ou alkylthio en C₁-C₄, ou
X et Y formant ensemble en position 2,3 ou 3,4 une chaîne -(CH₂)_{L} avec L= 3 ou 4, -OCH₂CH₂- ou -O-CH₂-O-,
W représentant H, CH₃ ou OCH₃,
V représentant un atome de fluor, chlore ou brome ou un groupe alkyle en C₁-C₄, phényle, hydroxy, alcoxy en C₁-C₄, alkylthio en C₁-C₄, un groupe -N(R^{s})2 dans lequel les restes R⁵ représentent des groupes alkyle en C₁-C₄ ou forment ensemble avec l'atome d'azote un radical pyrrolidino, pipéridino ou morpholino, le groupe benzyloxy ou CF₃, et
U représentant CH₃, F, Ci ou OCH₃,
R¹ est H,
Q est H ou un groupe alkyle en C₁-C₄,
R² est H,
R³ représente un groupe alkyle en C₁-C₁₂, alcényle en C3-Clo, cycloalkyle en C₃-C₁₂, cycloalcényle en C₅-C₁₂, polycycloalkyle en C₁-C₁₂, polycycloalcényle en C₁-C₁₂, cycloalkyl-(C₅-C₁₂)-alkyle(C₁-C₄), cycloalkylidène(C₅-C₄)-alkyle-(C₂-C₄), cycloalcényl-(C₅-C₁₂)-alkyle(C₁-C₄), polycycloalkyl(C₇-C₁₂)-alkyle(C₁-C₄), polycycloalkylidène(C₁-C₁₂)-alkyle(C₂-C₄), polycycloalcényl(C₇-C₁₂)-alkyle(C₁-C₄) ou ces mêmes radicaux hydrocarbonés comportant jusqu'à trois substituants identiques ou différents, ceux-ci représentant F, CI, Br ou CH₃,
ou un groupe phényl-alkyle(C2-C4) non substitué ou portant jusqu'à trois substituants sur le fragment phényle, les substituants étant identiques ou différents et représentant F, CI, Br, des groupes alcoxy en C₁-C₄, alkyle en C₁-C₄ ou alkylthio en C₁-C₄,
ou un groupe naphtyl-alkyle(C₁-C₄) non substitué ou portant jusqu'à deux substituants, les substituants étant identiques ou différents et représentant F, CI, Br ou des groupes OCH₃, OC₂H₅ ou alkyle en C,-C₄,
R⁴ représente H, un groupe alkyle en C₁-C₁₂, alcényle en C₃-C₁₀, cycloalkyle en C₃-C₁₂, cycloalcényle en C₅-C₁₂, polycycloalkyle en C₇-C₁₂, polycycloalcényle en C₇-C₁₂, cycloalkyl(C₅-C₁₂)-alkyle(C,-C₄), cycloalkylidène(C₅-C₁₂)-alkyle(C₂-C₄), cycloalcényl-(C₅-C₁₂)alkyle(C₁-C₄), polycycloalkyl(C₇-C₁₂)-alkyle(C₁-C₄), polycycloalkylidène(C₇-C₁₂)-alkyle(C₂-C₄), polycycloalcényl(C₇-C₁₂)-alkyle(C₁-C₄) ou ces mêmes radicaux hydrocarbonés comportant jusqu'à trois substituants indentiques ou différents, ceux-ci représentant F, CI ou Br, et les radicaux hydrocarbonés cycliques pouvant en outre être substitués par des groupes alkyle en C₁-C₄, ou
un groupe phényl-alkyle(C₂-C₄) non substitué ou portant sur le fragment phényle jusqu'à trois substituants, les substituants étant identiques ou différents et représentant F, CI, Br, des groupes alcoxy en C₁-C₄, alkyle en C₁-C₄ ou alkylthio en C₁-C₄,
ou un groupe naphtyl-alkyle(C₁-C₄) non substitué ou portant jusqu'à deux substituants, les substituants étant identiques ou différents et représentant F, CI, Br ou des groupes OCH₃, OC₂H₅ ou alkyle en C₁-C₄, ou
R³ et R⁴ forment ensemble une chaîne -(CH₂)n avec n = 2-11, non substituée ou substituée par des groupes alkyle en C₁-C₄, OCH₃ ou phényle, ou une même chaîne hydrocarbonée de ce type comportant une double liaison, ou
une chaîne -(CH₂)m avec m = 4 ou 5, et comportant de 1 à 5 atomes de carbone pontaux, non substituée ou substituée par des groupes alkyle en Ci-C₄, OCH₃, CH₂OCH₃ ou phényle, pontée une ou plusieurs fois, les atomes de carbone pontaux pouvant de leur côté être à leur tour pontés, et une liaison pontale contenant une double liaison C-C ou n'en contenant pas,
ainsi que ses sels d'addition avec des acides physiologiquement acceptables et ses stéréoisomères et énantiomères optiquement actifs.

2. Composé selon la revendication 1, caractérisé en ce qu'est satisfaite au moins l'une des caractéristiques suivantes:
"aryle" représente un radical 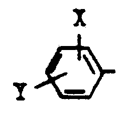 ou un radical 2-naphtyle non substitué ou substitué par un substituant V,
X représentant H ou un groupe alkyle en C₁-C₄, phényle, un atome de F, CI, Br, ou un groupe alcoxy en C₁-C₄, 3-CF₃ ou benzyloxy,
Y représentant H, CH₃, Clou OCH₃ et
V représentant un groupe alkyle en C₁-C₄, CI, Br, OH ou OCH₃,
Q représente H, CH₃ ou C₂H₅,
R' est H,
R² est H,
R³ représente un groupe alkyle en Ci-C₈, alcényle en C₃-C₁₀, cycloalkyle en C₃-C₁₂, cycloalcényle en C₅-C₁₂, polycycloalkyle en C₇-C₁₂, polycycloalcényle en C₇-C₁₂, cycloalkyl(C₅-C₁₂)-alkyle(C₁-C₄), cycloalkylidène(C₅-C₁₂)-alkyle(C₂-C₄), cycloalcényl-(C₅-C₁₂)-alkyle(C₁-C₄), polycycloalkyl(C₇-C₁₂)-alkyle(C₁-C₄), polycycloalkylidène(C₇-C₁₂)-alkyle(C₂-C₄), polycycloalcényl(C₇-C₁₂)-alkyle(C₁-C₄), ou ces mêmes radicaux hydrocarbonés cycliques comportant jusqu'à trois substituants identiques ou différents, ceux-ci représentant F, CI, Br ou CH₃, ou
un groupe phényl-alkyle(C₂-C₄) non substitué ou portant jusqu'à trois substituants sur le fragment phényle, les substituants étant identiques ou différents et représentant F, CI, Br, des groupes alcoxy en C₁-C₄, alkyle en
R4- représente H, un groupe alkyle en C₁-C₁₂, alcényle en C₃-C₅, cyclopropyle, cycloalkyle en Cs-Cₐ, un groupe phénylalkyle(C₂-C₄) ou naphtyl-alkyle(C₁-C₄) non substitués ou portant jusqu'à deux substituants sur le fragment phényle ou naphtyle, les substituants étant identiques ou différents et représentant F, CI, Br, OCH₃, OC₂H₅ ou des groupes alkyle en Ci-C₄, ou
R³ et R⁴ forment ensemble une chaîne -(CH₂)ₙ avec n= 4-11, non substituée ou substituée par un groupe alkyle en C₁-C₄ ou phényle, ou une même chaîne hydrocarbonée de ce type comportant une double liaison, ou une chaîne (CHz)m avec m = 4 ou 5, non substituée ou substituée par CH₃, C₂H₅, OCH₃ ou CH₃0CH_{z}, pontée une ou plusieurs fois, les atomes de carbone pontaux pouvant de leur côté être à leur tour reliés par des ponts et une liaison pontale contenant une double liaison C-C ou n'en contenant pas.

3. Composé I selon la revendication 1 ou 2, caractérisé en ce qu'est satisfaite au moins l'une des caractéristiques suivantes:
"aryle" représente un radical 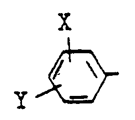 dans lequel
X représente F, CI ou un groupe alkyle en C₁-C₄, phényle ou alcoxy en C₁-C₄,
Y représente H, CH₃, Cl ou OCH₃ et la position 6 est toujours non substituée, ou
"aryle" est un radical 2-naphtyle non substitué ou substitué une fois par Br ou CI,
Q, R¹ et R² sont H,
R³ représente un groupe alkyle en C₁-C₈, cycloalkyle en C₅-C₇, polycycloalkyle en C₇-C₁₂, polycycloalcényle en C₇-C₁₂, polycycloalkyl(C₇-C₁₂)-alkyle(C₁-C₄) ou ces mêmes radicaux hydrocarbonés cycliques comportant un ou deux substituants identiques ou différents, ceux-ci représentant CI, Br ou CH₃, ou
un groupe phényl-alkyle(C₂-C₄) non substitué ou portant jusqu'à trois substituants sur le fragment phényle, les substituants étant identiques ou différents et représentant F, CI, Br, des groupes alcoxy en Ci-C₄ ou alkylthio en C₁-C₄,
R⁴ représente H ou un groupe alkyle en C₁-C₄, cyclopentyle, cyclohexyle ou cyclopropyle, ou
R³ et R4 forment ensemble une chaîne -(CH₂)ₙ- avec n = 4-7, non substituée ou substituée par CH₃ ou C₆H₅, ou
une chaîne (CH₂)ₘ- avec m = 4 ou 5, non substituée ou substituée par CH₃ ou CH₃OCH₂, pontée une ou plusieurs fois, et comportant jusqu'à 5 atomes de carbone pontaux, les atomes de carbone pontaux pouvant de leur côté être à leur tour pontés, et une liaison pontale comportant une double liaison C-C ou n'en comportant pas.

4. Composé selon la revendication 1, caractérisé en ce qu'est satisfaite au moins l'une des caractéristiques suivantes
"aryle" signifie 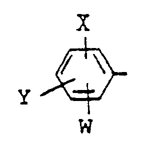 avec w = un atome d'hydrogène, X et Y représentent un atome d'hydrogène ou de F, CI ou Br,
Q et R¹ représentent un atome d'hydrogène,
_{R}² représente un atome d'hydrogène,
R³ et R⁴ forment ensemble une chaîne (CH₂)ₙ- avec n= 4 à 6, ou R³ et R⁴ forment ensemble une chaîne -(CH₂)ₘ pontée avec m = 4 ou 5 et comportant de 1 à 5 atomes de carbone pontaux.

5. Procédé pour la préparation d'un composé 1 selon la revendication 1, caractérisé en ce que l'on fait réagir
a) un arylméthylazole de formule II 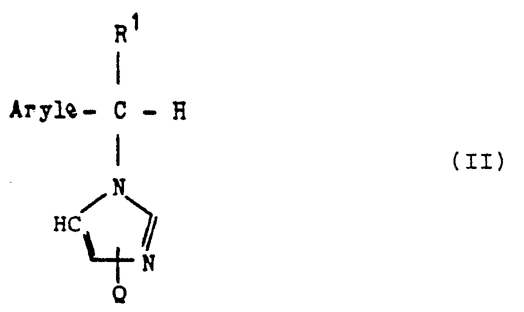 dans laquelle "aryle", Q et R¹ ont les significations données plus haut, avec un ou deux équivalents d'une base forte et ensuite avec un composé carbonyle de formule III 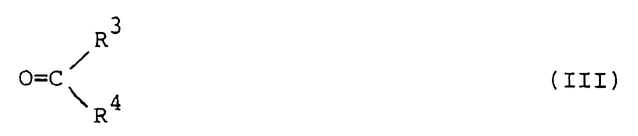 dans laquelle R³ et R⁴ ont les significations données plus haut, pour aboutir à un composé de formule I; ou
b) on fait réagir des oxirannes de Formule V 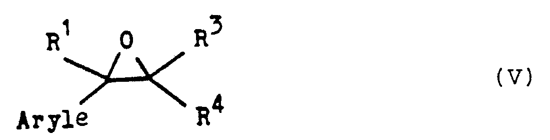 dans laquelle "aryle", R¹, R³ et R4 ont les significations données plus haut, avec un composé de formule VI 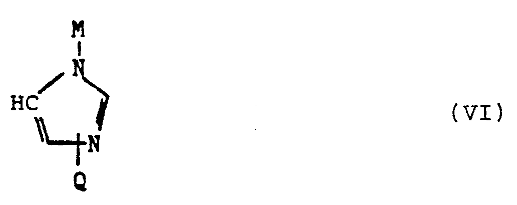 dans laquelle M représente un atome d'hydrogène ou un métal alcalin ou alcalino-terreux et Q a la significations donnée plus haut; ou
c) dans le cas de composés de formule I qui ont été préparés selon le procédé a) ou b), on convertit selon des méthodes connues un substituant X ou V, dans un radical aryle de la formule I, en un autre substituant X ou V,
et éventuellement on convertit avec des acides organiques ou minéraux les composés 1 obtenus selon a), b) ou c) en leurs sels d'addition avec des acides, et éventuellement on dédouble un composé obtenu selon a), b) ou c) en ses stéréoisomères et/ou ses énantiomères optiquement actifs.

6. Procédé pour la fabrication d'un médicament à action antidépressive, caractérisé en ce que l'on mélange une quantité efficace d'un composé I selon la revendication I avec des additifs pharmaceuti- quement acceptables.

7. Utilisation d'un composé de formule I selon la revendication 1, pour la fabrication d'un médicament à action antidépressive.

8. Médicament à action antidépressive, caractérisé par une teneur efficace en un composé 1 selon la revendication 1.

## Revendications (Revendications pour l'(les) Etat(s) contractant(s) suivant(s) : AT)

1. Procédé pour la préparation d'un composé de formule I 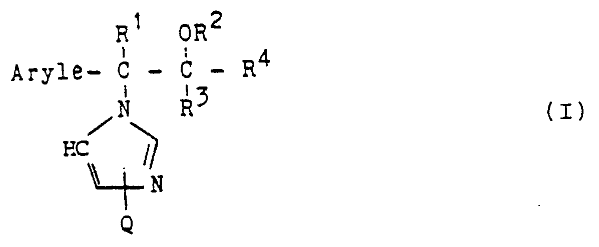 dans laquelle
"aryle" signifie un radical 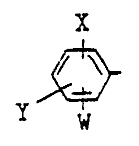 ou un radical 1- ou 2-naphtyle non substitué ou substitué par un substituant U et/ou un substituant V,
X représentant H, un groupe alkyle en C₁-C₄ ou phényle, un atome de fluor, chlore, brome, un groupe alcoxy en C₁-C₄, 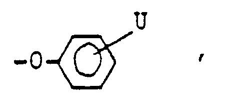 alkylthio en C₁-C₄, un groupe -N(R⁵)₂, dans lequel les restes R⁵ - identiques ou différents - représentent des groupes alkyle en C₁-C₄ ou forment ensemble, avec l'atome d'azote, un reste pyrrolidino, pipéridino ou morpholino,
CF³ ou un groupe benzyloxy non substitué ou portant sur le fragment phényle un ou deux substituants, les substituants étant identiques ou différents et représentant des atomes de fluor ou de chlore ou des groupes OCH₃, OC₂Hs ou alkyle en C₁-C₃,
Y représentant H, un groupe alkyle en Ci-C₄, un atome de fluor, chlore, brome, un groupe alcoxy en C₁-C₄ ou alkylthio en C₁-C₄ ou
X et Y formant ensemble en position 2,3 ou 3,4 une chaîne -(CH₂)L avec L= 3 ou 4, -OCH₂CH₂- ou -O-CH₂-O-,
W représentant H, CH₃ ou OCH₃,
V représentant un atome de fluor, chlore ou brome ou un groupe alkyle en C₁-C₄, phényle, hydroxy, alcoxy en C₁-C₄, alkylthio en C₁-C₄, un groupe -N(R⁵)₂ dans lequel les restes R⁵ représentent des groupes alkyle en C₁-C₄ ou forment ensemble avec l'atome d'azote un radical pyrrolidino, pipéridino ou morpholino, le groupe benzyloxy ou CF₃, et
U représentant CH₃, F, Ci ou OCH₃,
R¹ est H,
Q est H ou un groupe alkyle en C₁-C₄,
R² est H,
R³ représente un groupe alkyle en C₁-C₁₂, alcényle en C₃-C₁₀, cycloalkyle en C₃-C₁₂, cycloalcényle en Cs-Ci2, polycycloalkyle en C₇-C₁₂, polycycloalcényle en C₇-C₁₂, cycloalkyl(C₅-C₁₂)-alkyle(C₁-C₄), cycloalkylidène(C₅-C₁₂)-alkyle(C₂-C₄), cycloalcényl-(C₅-C₁₂)-alkyle(C₁-C₄), polycycloalkyl(C₇-C₁₂)-alkyle(C₁-C₄), polycycloalkylidène(C₇-C₁₂)-alkyle(C₂-C₄), polycycloalcényl(C₇-C₁₂)-alkyle(C₁-C₄) ou ces mêmes radicaux hydrocarbonés comportant jusqu'à trois substituants identiques ou différents, ceux-ci représentant F, CI, Br ou CH₃,
ou un groupe phényl-alkyle(C₂-C₄) non substitué ou portant jusqu'à trois substituants sur le fragment phényle, les substituants étant identiques ou différents et représentant F, CI, Br, des groupes alcoxy en C₁-C₄, alkyle en C₁-C₄ ou alkylthio en C₁-C₄,
ou un groupe naphtyl-alkyle(C₁-C₄) non substitué ou portant jusqu'à deux substituants, les substituants étant identiques ou différents et représentant F, CI, Br ou des groupes OCH₃, OC₂H₅ ou alkyle en C₁-C₄,
R⁴ représente H, un groupe alkyle en C₁-C₁₂, alcényle en C₃-C₁₀, cycloalkyle en C₃-C₁₂, cycloalcényle en C₅-C₁₂, polycycloalkyle en C₇-C₁₂, polycycloalcényle en C₇-C₁₂, cycloalkyl(C₅-C₁₂)-alkyle(C₁-C₄), cycloalkylidène(C₅-C₁₂)-alkyle(C₂-C₄), cycloalcényl-(C₅-C₁₂)-alkyle(C₁-C₄), polycycloalkyl(C₇-C₁₂)-alkyle(C₁-C₄), polycycloalkylidène(C₇-C₁₂)-alkyle(C₂-C₄), polycycloalcényl(C₇-C₁₂)-alkyle(C₁-C₄) ou ces mêmes radicaux hydrocarbonés comportant jusqu'à trois substituants indentiques ou différents, ceux-ci représentant F, CI ou Br, et les radicaux hydrocarbonés cycliques pouvant en outre être substitués par des groupes alkyle en C₁-C₄, ou
un groupe phényl-alkyle(C₂-C₄) non substitué ou portant sur le fragment phényle jusqu'à trois substituants, les substituants étant identiques ou différents et représentant F, CI, Br, des groupes alcoxy en C₁-C₄, alkyle en C₁-C₄ ou alkylthio en C₁-C₄,
ou un groupe naphtyl-alkyle(C₁-C₄) non substitué ou portant jusqu'à deux substituants, les substituants étant identiques ou différents et représentant F, CI, Br ou des groupes OCH₃, OC2H5 ou alkyle en C,-C₄, ou
R³ et R⁴ forment ensemble une chaîne -(CH₂)n avec n = 2-11, non substituée ou substituée par des groupes alkyle en C₁-C₄, OCH₃ ou phényle, ou une même chaîne hydrocarbonée de ce type comportant une double liaison, ou
une chaîne -(CH₂)m avec m = 4 ou 5, et comportant de 1 à 5 atomes de carbone pontaux, non substituée ou substituée par des groupes alkyle en C₁-C₄, OCH₃, CH₂OCH₃ ou phényle, pontée une ou plusieurs fois, les atomes de carbone pontaux pouvant de leur côté être à leur tour pontés, et une liaison pontale contenant une double liaison C-C ou n'en contenant pas,
ainsi que de ses sels d'addition avec des acides physiologiquement acceptables et ses stéréoisomères et énantiomères optiquement actifs, caractérisé en ce que l'on fait réagir
a) un arylméthylazole de formule Il 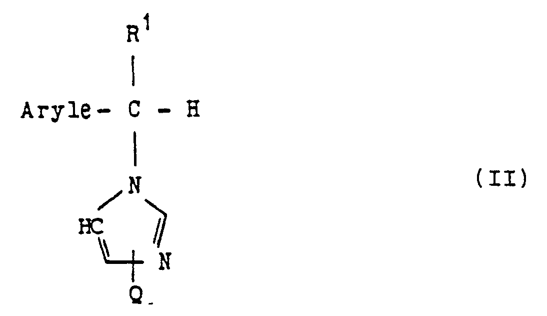 dans laquelle "aryle", Q et R¹ ont les significations données plus haut, avec un ou deux équivalents d'une base forte et ensuite avec un composé carbonyle de formule III 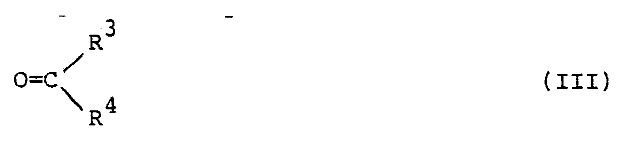 dans laquelle R³ et R⁴ ont les significations données plus haut, pour aboutir à un composé de formule I; ou
b) on fait réagir des oxirannes de formule V 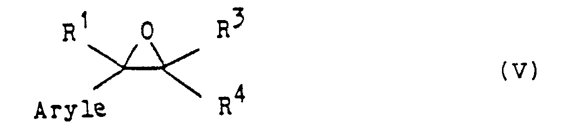 dans laquelle "aryle", R¹, R³ et R⁴⁻ ont les significations données plus haut, avec un composé de formule VI 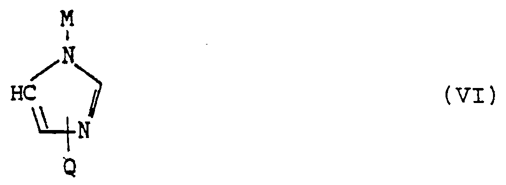 dans laquelle M représente un atome d'hydrogène ou un métal alcalin ou alcalino-terreux et Q a la significations donnée plus haut; ou
c) dans le cas de composés de formule I qui ont été préparés selon le procédé a) ou b), on convertit selon des méthodes connues un substituant X ou V, dans un radical aryle de la formule I, en un autre substituant X ou V, et éventuellement on convertit avec des acides organiques ou minéraux les composés 1 obtenus selon a), b) ou c) en leurs sels d'addition avec des acides, et éventuellement on dédouble un composé obtenu selon a), b) ou c) en ses stéréoisomères et/ou ses énantiomères optiquement actifs.

2. Procédé selon la revendication 1, caractérisé en ce qu'est satisfaite au moins l'une des caractéristiques suivantes:
"aryle" représente un radical 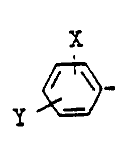 ou un radical 2-naphtyle non substitué ou substitué par un substituant V, dans lequel
X représente H ou un groupe alkyle en Ci-C₄, phényle, un atome de F, CI, Br, ou un groupe alcoxy en C₁-C₄, 3-CF₃ ou benzyloxy,
Y représente H, CH₃, CI ou OCH₃ et
V représente un groupe alkyle en C₁-C₄, CI, Br, OH ou OCH₃,
Q représente H, CH₃ ou C₂H₅,
R¹ est H,
R² est H,
R³ représente un groupe alkyle en C₁-C₈, alcényle en C₃-C₁₀, cycloalkyle en C₃-C₁₂, cycloalcényle en C₅-C₁₂, polycycloalkyle en C₇-C₁₂, polycycloalcényle en C₇-C₁₂, cycloalkyl(C₅-C₁₂)-alkyle(C₁-C₄), cycloalkylidène(C₅-C₁₂)-alkyle(C₂-C₄), cycloalcényl-(C₅-C₁₂)-alkyle(C₁-C₄), polycycloalkyl(C₇-C₁₂)-alkyle(C₁-C₄), polycycloalkylidène(C₇-C₁₂)-alkyle(C₂-C₄), polycycloalcényl(C₇-C₁₂)-alkyle(C₁-C₄), ou ces mêmes radicaux hydrocarbonés cycliques comportant jusqu'à trois substituants identiques ou différents, ceux-ci représentant F, CI, Br ou CH₃, ou
un groupe phényl-alkyle(C₂-C₄) non substitué ou portant jusqu'à trois substituants sur le fragment phényle, les substituants étant identiques ou différents et représentant F, CI, Br, des groupes alcoxy en C₁-C₄, alkyle en C₁-C₄ ou alkylthio en C₁-C₄,
R⁴ représente H, un groupe alkyle en C₁-C₁₂, alcényle en C₃-C₅, cyclopropyle, cycloalkyle en Cs-Cs, un groupe phénylalkyle(C₂-C₄) ou naphtyl-alkyle(C₁-C₄) non substitués ou portant jusqu'à deux substituants sur le fragment phényle ou naphtyle, les substituants étant identiques ou différents et représentant F, CI, Br, OCH₃, OC₂H₅ ou des groupes alkyle en C₁-C₄, ou
R³ et R4 forment ensemble une chaîne -(CH₂)ₙ avec n = 4-11, non substituée ou substituée par un groupe alkyle en C₁-C₄ ou phényle, ou une même chaîne hydrocarbonée de ce type comportant une double liaison, ou une chaîne -(CH₂)ₘ avec m = 4 ou 5, non substituée ou substituée par CH₃, C₂H₅, OCH₃ ou CH₃0CH₂, pontée une ou plusieurs fois, les atomes de carbone pontaux pouvant de leur côté être à leur tour reliés par des ponts et une liaison pontale contenant une double liaison C-C ou n'en contenant pas.

3. Procédé selon la revendication 1 ou 2, caractérisé en ce qu'est satisfaite au moins l'une des caractéristiques suivantes:
"aryle" représente un radical 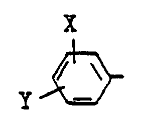 dans lequel
X représente F, CI ou un groupe alkyle en C₁-C₄, phényle ou alcoxy en C₁-C₄,
Y représente H, CH₃, Ci ou OCH₃ et la position 6 est toujours non substituée, ou
"aryle" est un radical 2-naphtyle non substitué ou substitué une fois par Br ou CI,
Q, R¹ et R² sont H,
R³ représente un groupe alkyle en C₁-C₈, cycloalkyle en Cₛ-C₇, polycycloalkyle en C₇-Cᵢ₂, polycycloalcényle en C₇-C₁₂, polycycloalkyl(C₇-C₁₂)-alkyle(C₁-C₄) ou ces mêmes radicaux hydrocarbonés cycliques comportant un ou deux substituants identiques ou différents, ceux-ci représentant CI, Br ou CH₃, ou
un groupe phényl-alkyle(C₂-C₄) non substitué ou portant jusqu'à trois substituants sur le fragment phényle, les substituants étant identiques ou différents et représentant F, CI, Br, des groupes alcoxy en C₁-C₄ ou alkylthio en C₁-C₄,
R⁴ représente H ou un groupe alkyle en C₁-C₄, cyclopentyle, cyclohexyle ou cyclopropyle, ou
R³ et R⁴ forment ensemble une chaîne -(CH₂)ₙ- avec n = 4-7, non substituée ou substituée par CH₃ ou C₆H₅, ou
une chaîne (CHz)m- avec m = 4 ou 5, non substituée ou substituée par CH₃ ou CH₃OCH₂, pontée une ou plusieurs fois, et comportant jusqu'à 5 atomes de carbone pontaux, les atomes de carbone pontaux pouvant de leur côté être à leur tour pontés, et une liaison pontale comportant une double liaison C-C ou n'en comportant pas.

4. Procédé selon la revendication 1, caractérisé en ce qu'est satisfaite au moins l'une des caractéristiques suivantes
"aryle" signifie 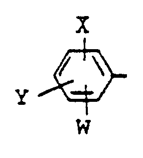 avec W = un atome d'hydrogène,
X et Y représentent un atome d'hydrogène ou de F, CI ou Br,
Q et R¹ représentent un atome d'hydrogène,
R² représente un atome d'hydrogène,
R³ et R⁴ forment ensemble une chaîne -(CH₂)ₙ- avec n = 4 à 6, ou R³ et R⁴ forment ensemble une chaîne -(CH₂)ₘ pontée avec m = 4 ou 5 et comportant de 1 à 5 atomes de carbone pontaux.

5. Procédé pour la fabrication d'un médicament à action antidépressive, caractérisé en ce que l'on mélange une quantité efficace d'un composé 1 selon la revendication I avec des additifs pharmaceuti- quement acceptables.

6. Utilisation d'un composé de formule I selon la revendication 1, pour la fabrication d'un médicament à action antidépressive.
